# EUROPEAN PATENT APPLICATION

(11) **EP 4 603 593 A1**
(43) Date of publication of application: **20.08.2025**
(21) Application number: 23877741.1
(22) Date of filing: 13.10.2023
(51) Int. Cl.: C12Q 1/6818, C12Q 1/6876

(54) **METHOD FOR DETECTING TARGET NUCLEIC ACID IN SAMPLE USING TWO PROBES WITH DIFFERENT TM VALUES**

(30) Priority: 14.10.2022 KR 20220132461
(71) Applicant: Seegene, Inc., Seoul 05548 (KR)
(72) Inventor: LEE, Han Bit, Seoul 05546 (KR); KIM, Jeong Woo, Gwangju-si, Gyeonggi-do 12772 (KR)
(74) Representative: Dehns
(86) International application number: PCT/KR2023/015809
(87) International publication number: WO 2024/080818

(57) **Abstract**

The present disclosure relates to a method for detecting n target nucleic acids in a sample using a composition for detecting a target nucleic acid, which comprises two probes having different Tm values. The method according to the present disclosure enables the real-time detection of multiple target nucleic acids using a single type of label.

## Description

### Technical Field

The present disclosure relates to a method for detecting a target nucleic acid in a sample using two probes having different Tm values.

### Background Technology

For the detection of target nucleic acids, real-time detection methods that allow for monitoring target amplification in real time are widely used. Real-time detection methods generally utilize labeled probes or primers that hybridize specifically with target nucleic acids.

Examples of methods utilizing hybridization between labeled probes and target nucleic acids include molecular beacon method using dual-labeled probes with a hairpin structure (Tyagi et al., Nature Biotechnology, v.14 MARCH 1996), HyBeacon method (French DJ et al., Mol. Cell Probes, 15(6):363-374(2001)), hybridization probe method using two probes, each labeled as donor and acceptor (Bernad et al., 147-148 Clin Chem 2000; 46), and Lux method using single-labeled oligonucleotides (U.S. Pat. No. 7,537,886). TaqMan method (U.S. Pat. Nos. 5,210,015 and 5,538,848) using cleavage of dual-labeled probe by 5'-nuclease activity of DNA polymerase is also widely used in the art.

Examples of methods using labeled primers include Sunrise primer method (Nazarenko et al., 2516-2521 Nucleic Acids Research, 1997, v.25 no.12, and U.S. Pat. No. 6,117,635), Scorpion primer method (Whitcombe et al., 804-807, Nature Biotechnology v.17 AUGUST 1999 and U.S. Pat. No. 6,326,145), and TSG primer method (WO 2011/078441).

Since the above-described conventional real-time detection technologies can only detect a single target nucleic acid per a label, the number of target nucleic acids that can be simultaneously detected in a single reaction is limited by the number of labels that can be used (e.g., 5 or less).

Therefore, there is a need to develop a novel method or approach that enables not only real-time detection of a single target nucleic acid but also real-time detection of multiple target nucleic acids using a single type of label.

Throughout this application, various patents and publications are referenced and citations are provided in parentheses. The disclosure of these patents and publications in their entities are hereby incorporated by references into this application in order to more fully describe this invention and the state of the art to which this invention pertains.

### Disclosure

### Technical Problem

The present inventors have endeavored to develop a novel method for detecting target nucleic acids in real time with improved convenience, high cost-effectiveness, and efficiency. In particular, efforts were made to develop a method that can detect multiple target nucleic acid sequences using a single type of label. As a result, the present inventors confirmed that when two probes having different Tm values capable of hybridizing adjacent to the target nucleic acid are used, the target nucleic acid can be detected. Furthermore, it was confirmed that multiple target nucleic acids can be detected in real time using a single type of label.

Therefore, it is an object of the present disclosure to provide a method for detecting *n* target nucleic acids in a sample.

It is another object of the present disclosure to provide a method for detecting a target nucleic acid in a sample.

It is another object of the present disclosure to provide a composition for detecting a target nucleic acid in a sample.

Other objects and advantages of the present invention will become apparent from the detailed description to follow taken in conjugation with the appended claims and drawings.

### Solution to Problem

According to an aspect of the present disclosure, there is provided a method for detecting *n* target nucleic acids in a sample, comprising:
(a) detecting signals at *n* detection temperatures, while incubating with *n* compositions for detecting the target nucleic acids, a sample suspected of containing at least one of the *n* target nucleic acids in a reaction vessel;
   wherein *n* is an integer of 2 or more,
   wherein the incubating comprises a plurality of cycles and the detection of signals is carried out at at least one of the plurality of cycles,
   wherein each of the *n* compositions for detecting the target nucleic acids provides a signal change at a corresponding detection temperature among the *n* detection temperatures in the presence of a corresponding target nucleic acid, the signal change indicating the presence of a corresponding target nucleic acid,
   wherein a composition for detecting an *i*^{th} target nucleic acid among the *n* compositions for detecting the target nucleic acids provides a signal change at an *i^{th}* detection temperature among the *n* detection temperatures and provides a constant signal at the other detection temperatures in the presence of the *i^{th}* target nucleic acid, the signal change indicating the presence of the *i^{th}* target nucleic acid,
   wherein *i* represents an integer from 1 to *n,* and the *i^{th}* detection temperature is lower than the (*i+*1)*^{th}* detection temperature,
   wherein in the temperature range covering all of the *n* detection temperatures, the composition for detecting the *i^{th}* target nucleic acid has a signal-changing temperature range in which the signal changes depending on the presence of the *i^{th}* target nucleic acid, and one or two signal-constant temperature ranges in which the signal is constant even in the presence of the *i^{th}* target nucleic acid,
   wherein the composition for detecting the *i^{th}* target nucleic acid is any one of:
      (i) an Under-Signal-Change (UnderSC) composition having a characteristic that the signal-changing temperature range is lower than the signal-constant temperature range,
      (ii) an Inter-Signal-Change (InterSC) composition having a characteristic that the signal-changing temperature range is higher than one of two signal-constant temperature ranges, and lower than the other of the two signal-constant temperature ranges, and
      (iii) an Over-Signal-Change (OverSC) composition having a characteristic that the signal-changing temperature range is higher than the signal-constant temperature range,
   wherein at least one of the *n* compositions for detecting target nucleic acids comprises a first probe and a second probe having different Tm values,
   wherein the first probe comprises a hybridizing nucleotide sequence to a first region of a corresponding target nucleic acid,
   wherein the first probe has a reporter molecule and a first quencher molecule,
   wherein the reporter molecule and the first quencher molecule are located at positions such that (i) the reporter molecule and the first quencher molecule are in close proximity to each other, prior to hybridization of the first probe with the corresponding target nucleic acid, thereby causing the first quencher molecule to quench a signal from the reporter molecule, and (ii) the reporter molecule and the first quencher molecule are separated upon hybridization of the first probe with the corresponding target nucleic acid, thereby causing the first quencher molecule to unquench a signal from the reporter molecule,
   wherein the second probe comprises a hybridizing nucleotide sequence to a second region of the corresponding target nucleic acid,
   wherein the second probe has a second quencher molecule,
   wherein the Tm value of the first probe is higher than the Tm value of the second probe, and
   wherein the reporter molecule of the first probe is quenched by the second quencher molecule of the second probe when the first probe and the second probe are hybridized with the corresponding target nucleic acid; and
(b) determining the presence of the *n* target nucleic acids from the signals detected in step (a), wherein the presence of the *i^{th}* target nucleic acid is determined by the signal change detected at the *i^{th}* detection temperature.

According to an embodiment of the present disclosure, the first quencher molecule is located at 12 to 60 nucleotides apart from the reporter molecule.

According to an embodiment of the present disclosure, the reporter molecule is linked to the 3'-end portion or the 5'-end portion of the first probe.

According to an embodiment of the present disclosure, the reporter molecule and the second quencher molecule are located at positions such that the second quencher molecule of the second probe is in close proximity to the reporter molecule of the first probe upon hybridization of the first probe and the second probe with the corresponding target nucleic acid, thereby causing the second quencher molecule to quench a signal from the reporter molecule.

According to an embodiment of the present disclosure, when the first probe and the second probe are hybridized with the corresponding target nucleic acid, the second quencher molecule is located immediately adjacent to or 1 to 4 nucleotides apart from the reporter molecule.

According to an embodiment of the present disclosure, the second quencher molecule is linked to the 3'-end portion or the 5'-end portion of the second probe.

According to an embodiment of the present disclosure, when the second probe is hybridized with the corresponding target nucleic acid in the 3' direction downstream of the first probe, the reporter molecule is located at the 3'-end portion of the first probe, and the second quencher molecule is located at the 5'-end portion of the second probe.

According to an embodiment of the present disclosure, when the second probe is hybridized with the corresponding target nucleic acid in the 5' direction upstream of the first probe, the reporter molecule is located at the 5'-end portion of the first probe, and the second quencher molecule is located at the 3'-end portion of the second probe.

According to an embodiment of the present disclosure, the first quencher molecule of the first probe and the second quencher molecule of the second probe are the same type of quencher molecule.

According to an embodiment of the present disclosure, the first probe and/or the second probe are blocked at its 3'-end to prevent its extension.

According to an embodiment of the present disclosure, the composition comprising the first probe and the second probe having different Tm values is an InterSC composition having a characteristic that the signal-changing temperature range is higher than a first signal-constant temperature of two signal-constant temperature ranges, and lower than a second signal-constant of the two signal-constant temperature ranges.

According to an embodiment of the present disclosure, the first probe and the second probe are hybridized with the corresponding target nucleic acid at temperatures within the first signal-constant temperature range in the presence of the corresponding target nucleic acid.

According to an embodiment of the present disclosure, the first probe and the second probe are not hybridized with the corresponding target nucleic acid at temperatures within the second signal-constant temperature range in the presence of the corresponding target nucleic acid.

According to an embodiment of the present disclosure, the first probe is hybridized with the corresponding target nucleic acid but the second probe is not hybridized with the corresponding target nucleic acid at temperatures within the signal-changing temperature range in the presence of the corresponding target nucleic acid.

According to an embodiment of the present disclosure, the signal-changing temperature range depends on the Tm values of the first probe and the second probe.

According to an embodiment of the present disclosure, the *i^{th}* detection temperature is selected within the signal-changing temperature range of the composition for detecting the *i^{th}* target nucleic acid, wherein the *i^{th}* detection temperature is not comprised in the signal-changing temperature ranges of the compositions for detecting the other target nucleic acids.

According to an embodiment of the present disclosure, the signal-changing temperature range of the composition for detecting the *i^{th}* target nucleic acid overlaps partially with the signal-changing temperature range of a composition for detecting a target nucleic acid having an adjacent detection temperature, and does not overlap with the signal-changing temperature range of a composition for detecting a target nucleic acid having a detection temperature that is not adjacent thereto.

According to an embodiment of the present disclosure, when *n* is 2, the composition for detecting the first target nucleic acid is an UnderSC or InterSC composition, and the composition for detecting the second target nucleic acid is an InterSC or OverSC composition.

According to an embodiment of the present disclosure, when *n is* 3 or more, the composition for detecting the first target nucleic acid is an UnderSC or InterSC composition, the composition for detecting the *n*^{th} target nucleic acid is an InterSC or OverSC composition, and each of compositions for detecting target nucleic acids other than the first target nucleic acid and the *n*^{th} target nucleic acid is an InterSC composition.

According to an embodiment of the present disclosure, the composition for detecting the *i^{th}* target nucleic acid comprises a label that provides a signal dependent on the presence of the *i^{th}* target nucleic acid.

According to an embodiment of the present disclosure, the label is linked to an oligonucleotide or is incorporated into an oligonucleotide during the incubating.

According to an embodiment of the present disclosure, the composition for detecting the *i^{th}* target nucleic acid provides a duplex providing a signal change.

According to an embodiment of the present disclosure, the duplex providing the signal change has initially been included in the composition for detecting the *i^{th}* target nucleic acid.

According to an embodiment of the present disclosure, the duplex providing the signal change is generated by hybridization between a label-linked oligonucleotide and an oligonucleotide hybridizable with the label-linked oligonucleotide.

According to an embodiment of the present disclosure, the duplex providing the signal change is generated in the incubating.

According to an embodiment of the present disclosure, the duplex providing the signal change is generated by hybridization between a label-linked oligonucleotide and the corresponding target nucleic acid.

According to an embodiment of the present disclosure, the duplex providing the signal change is generated by a cleavage reaction dependent on the presence of the corresponding target nucleic acid.

According to an embodiment of the present disclosure, the duplex providing the signal change comprises a label.

According to an embodiment of the present disclosure, the composition for detecting the *i^{th}* target nucleic acid provides a duplex providing a signal change, and the signal-changing temperature range of the composition for detecting the *i^{th}* target nucleic acid varies depending on the length and/or sequence of the duplex.

According to an embodiment of the present disclosure, the detection of signals is carried out at at least two of the plurality of cycles.

According to an embodiment of the present disclosure, the signal change is measured using the signals detected at the at least two of the plurality of cycles.

According to an embodiment of the present disclosure, the signal change at the *i^{th}* detection temperature is measured using a signal detected at the at least one of the plurality of cycles and a reference signal value.

According to an embodiment of the present disclosure, the reference signal value is obtained from a reaction in the absence of the *i^{th}* target nucleic acid.

According to an embodiment of the present disclosure, the detection of a signal at each of the *n* detection temperatures is carried out using a single type of detector.

According to an embodiment of the present disclosure, the signals detected at the *n* detection temperatures are not differentiated from each other by the single type of detector.

According to an embodiment of the present disclosure, the incubating comprises a nucleic acid amplification reaction.

According to another aspect of the present disclosure, there is provided a method for detecting a target nucleic acid in a sample, comprising:
(a) incubating the sample with a composition for detecting the target nucleic acid comprising a first probe and a second probe having different Tm values;
   wherein the first probe comprises a hybridizing nucleotide sequence to a first region of the target nucleic acid,
   wherein the first probe has a reporter molecule and a first quencher molecule,
   wherein the reporter molecule and the first quencher molecule are located at positions such that (i) the reporter molecule and the first quencher molecule are in close proximity to each other, prior to hybridization of the first probe with the target nucleic acid, thereby causing the first quencher molecule to quench a signal from the reporter molecule, and (ii) the reporter molecule and the first quencher molecule are separated upon hybridization of the first probe with the target nucleic acid, thereby causing the first quencher molecule to unquench a signal from the reporter molecule,
   wherein the second probe comprising a hybridizing nucleotide sequence to a second region of the target nucleic acid,
   wherein the second probe has a second quencher molecule,
   wherein the Tm value of the first probe is higher than the Tm value of the second probe, and
   wherein the reporter molecule of the first probe is quenched by the second quencher molecule of the second probe when the first probe and the second probe are hybridized with the target nucleic acid; and
(b) detecting a signal; and
(c) determining the presence of the target nucleic acid from the signal detected in the step (b).

According to an embodiment of the present disclosure, the reporter molecule and the second quencher molecule are located at positions such that the second quencher molecule of the second probe is in close proximity to the reporter molecule of the first probe upon hybridization of the first probe and the second probe with the target nucleic acid, thereby causing the second quencher molecule to quench a signal from the reporter molecule.

According to an embodiment of the present disclosure, the second quencher molecule is linked to the 3'-end portion or the 5'-end portion of the second probe.

According to an embodiment of the present disclosure, when the second probe is hybridized with the corresponding target nucleic acid in the 3' direction downstream of the first probe, the reporter molecule is located at the 3'-end portion of the first probe, and the second quencher molecule is located at the 5'-end portion of the second probe.

According to an embodiment of the present disclosure, when the second probe is hybridized with the corresponding target nucleic acid in the 5' direction upstream of the first probe, the reporter molecule is located at the 5'-end portion of the first probe, and the second quencher molecule is located at the 3'-end portion of the second probe.

According to an embodiment of the present disclosure, the first quencher molecule of the first probe and the second quencher molecule of the second probe are the same type of quencher molecule.

According to an embodiment of the present disclosure, the composition comprising the first probe and the second probe having different Tm values has a signal-changing temperature range (SChTR) in which the signal changes depending on the presence of the target nucleic acid, and two signal-constant temperature ranges (SCoTRs) in which the signal is constant even in the presence of the target nucleic acid,

According to an embodiment of the present disclosure, the signal-changing temperature range is higher than a first signal-constant temperature range of the two signal-constant temperature ranges, and lower than a second signal-constant temperature range of the two signal-constant temperature ranges.

According to an embodiment of the present disclosure, the first probe and the second probe are hybridized with the target nucleic acid at temperatures within the first signal-constant temperature range in the presence of the target nucleic acid.

According to an embodiment of the present disclosure, the first probe and the second probe are not hybridized with the target nucleic acid at temperatures within the second signal-constant temperature range in the presence of the target nucleic acid.

According to an embodiment of the present disclosure, the first probe is hybridized with the target nucleic acid but the second probe is not hybridized with the target nucleic acid at temperatures within the signal-changing temperature range in the presence of the target nucleic acid.

According to an embodiment of the present disclosure, the signal-changing temperature range depends on the Tm values of the first probe and the second probe.

According to an embodiment of the present disclosure, the signal detected in the step (b) is a signal dependent on the presence of the target nucleic acid.

According to an embodiment of the present disclosure, the step (b) is carried out at a detection temperature selected within the signal-changing temperature range.

According to an embodiment of the present disclosure, the incubating comprises a nucleic acid amplification reaction.

According to an embodiment of the present disclosure, the incubating in the step (a) comprises a plurality of cycles, and the detection of the signal in the step (b) is carried out at at least one of the plurality of cycles.

According to an embodiment of the present disclosure, the presence of the target nucleic acid in the step (c) is determined by a signal change, wherein the signal change is measured using the signal detected at least one of the plurality of cycles and a reference signal value.

According to an embodiment of the present disclosure, the reference signal value is obtained from a reaction in the absence of the target nucleic acid.

According to an embodiment of the present disclosure, the incubating in the step (a) comprises a plurality of cycles, and the detection of the signal in the step (b) is carried out at at least two of the plurality of cycles.

According to an embodiment of the present disclosure, the presence of the target nucleic acid in the step (c) is determined by a signal change, wherein the signal change is measured using signals detected at the at least two of the plurality of cycles.

According to another aspect of the present disclosure, there is provided a composition for detecting a target nucleic acid in a sample, comprising:
(a) a first probe comprising a hybridizing nucleotide sequence to a first region of the target nucleic acid;
   wherein the first probe has a reporter molecule and a first quencher molecule,
   wherein the reporter molecule and the first quencher molecule are located at positions such that (i) the reporter molecule and the first quencher molecule are in close proximity to each other, prior to hybridization of the first probe with the target nucleic acid, thereby causing the first quencher molecule to quench a signal from the reporter molecule, and (ii) the reporter molecule and the first quencher molecule are separated upon hybridization of the first probe with the target nucleic acid, thereby causing the first quencher molecule to unquench a signal from the reporter molecule; and
(b) a second probe comprising a hybridizing nucleotide sequence to a second region of the target nucleic acid;
   wherein the second probe has a second quencher molecule,
   wherein the Tm value of the first probe is higher than the Tm value of the second probe, and
   wherein the reporter molecule of the first probe is quenched by the second quencher molecule of the second probe when the first probe and the second probe are hybridized with the target nucleic acid.

According to an embodiment of the present disclosure, the reporter molecule and the second quencher molecule are located at positions such that the second quencher molecule of the second probe is in close proximity to the reporter molecule of the first probe upon hybridization of the first probe and the second probe with the target nucleic acid, thereby causing the second quencher molecule to quench a signal from the reporter molecule.

According to an embodiment of the present disclosure, the second quencher molecule is linked to the 3'-end portion or the 5'-end portion of the second probe.

According to an embodiment of the present disclosure, when the second probe is hybridized with the corresponding target nucleic acid in the 3' direction downstream of the first probe, the reporter molecule is located at the 3'-end portion of the first probe, and the second quencher molecule is located at the 5'-end portion of the second probe.

According to an embodiment of the present disclosure, when the second probe is hybridized with the corresponding target nucleic acid in the 5' direction upstream of the first probe, the reporter molecule is located at the 5'-end portion of the first probe, and the second quencher molecule is located at the 3'-end portion of the second probe.

According to an embodiment of the present disclosure, the first quencher molecule of the first probe and the second quencher molecule of the second probe are the same type of quencher molecule.

According to an embodiment of the present disclosure, the composition has a signal-changing temperature range (SChTR) in which the signal changes depending on the presence of the target nucleic acid, and two signal-constant temperature ranges (SCoTRs) in which the signal is constant even in the presence of the target nucleic acid,

According to an embodiment of the present disclosure, the signal-changing temperature range is higher than a first signal-constant temperature range of the two signal-constant temperature ranges, and lower than a second signal-constant temperature range of the two signal-constant temperature ranges.

According to an embodiment of the present disclosure, the first probe and the second probe are hybridized with the target nucleic acid at temperatures within the first signal-constant temperature range in the presence of the target nucleic acid.

According to an embodiment of the present disclosure, the first probe and the second probe are not hybridized with the target nucleic acid at temperatures within the second signal-constant temperature range in the presence of the target nucleic acid.

According to an embodiment of the present disclosure, the first probe is hybridized with the target nucleic acid but the second probe is not hybridized with the target nucleic acid at temperatures within the signal-changing temperature range in the presence of the target nucleic acid.

According to an embodiment of the present disclosure, the signal-changing temperature range depends on the Tm values of the first probe and the second probe.

### Advantageous Effects

The features and advantages of the present disclosure will be summarized as follows:
(a) The present disclosure pertains to a method for detecting multiple target nucleic acids by a single type of label alone in a single reaction vessel by using multiple detection temperatures, which is characterized by providing a signal change dependent on the presence of a corresponding target nucleic acid at a corresponding detection temperature of each of the target nucleic acids.
   Specifically, at least one of the multiple target nucleic acids is detected using two probes having different Tm values that can hybridize adjacently on the target nucleic acid.
(b) A second feature of the present disclosure is that the composition for detecting target nucleic acids, comprising two probes having different Tm values, has a signal-changing temperature range in which the signal changes depending on the presence of the target nucleic acid, and two signal-constant temperature ranges in which the signal is constant even in the presence of the target nucleic acid in reaction with the target nucleic acid (e.g., an amplification reaction). Specifically, the composition for detecting target nucleic acids, comprising two probes having different Tm values, is an InterSC composition having a characteristic that the signal-changing temperature range is higher than one of two signal-constant temperature ranges, and lower than the other of the two signal-constant temperature ranges.
(c) The composition for detecting target nucleic acids, comprising two probes having different Tm values according to the present disclosure, not only enables detection of a single target nucleic acid but also allows for detection of multiple target nucleic acids using a single type of label in real time. Specifically, when using a plurality of compositions for detecting a plurality of target nucleic acids, a plurality of target nucleic acids can be detected in real time using a single type of label by adjusting the signal-changing temperature range of each of the compositions for detecting a plurality of target nucleic acids (for example, by adjusting signal-changing temperature ranges not to overlap with each other). Moreover, the method of the present disclosure has the advantage of dramatically reducing its analysis time compared to conventional melting analysis after target amplification to detect multiple target nucleic acids using a single type of label.

### Description of Drawings

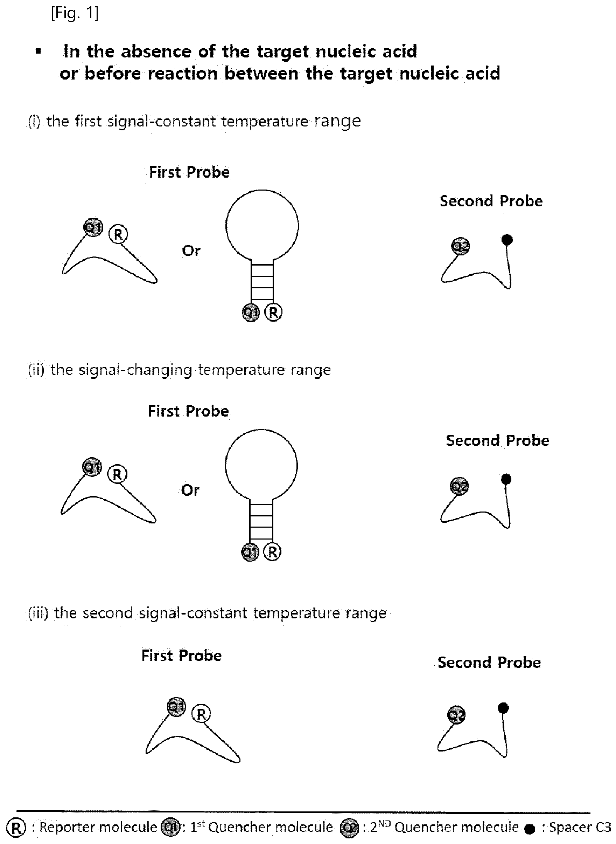

FIG. 1 shows the conformations of the first probe and the second probe in (i) a first signal-constant temperature range, (ii) a signal-changing temperature range, and (iii) a second signal-constant temperature range, in the absence of the target nucleic acid or before reaction between the target nucleic acid and the composition for detecting the target nucleic acid comprising two probes having different Tm values according to the present disclosure.

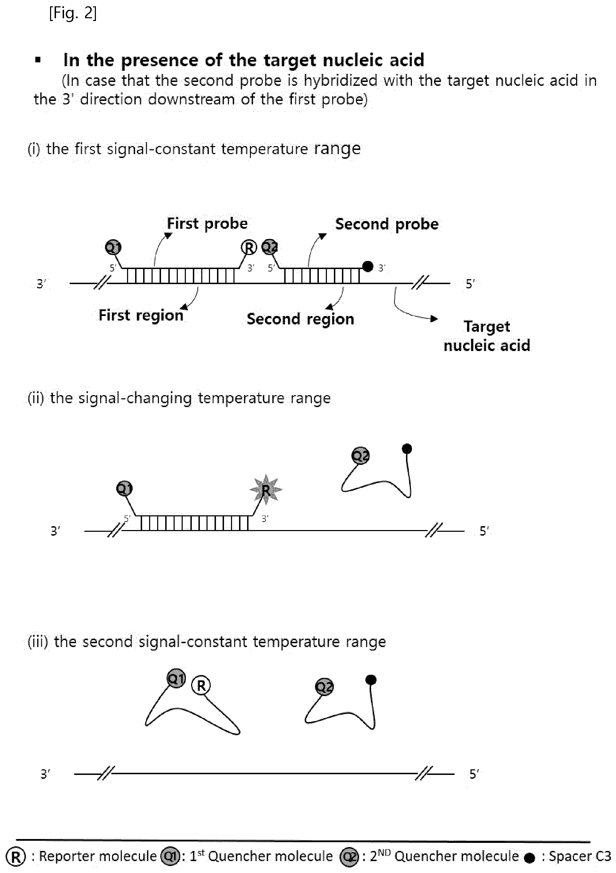

FIG. 2 shows the conformations of the first probe and the second probe in the presence of the target nucleic acid in (i) a first signal-constant temperature range, (ii) a signal-changing temperature range, and (iii) a second signal-constant temperature range, in a specific embodiment where the second probe hybridizes with the target nucleic acid in the 3' direction downstream of the first probe.

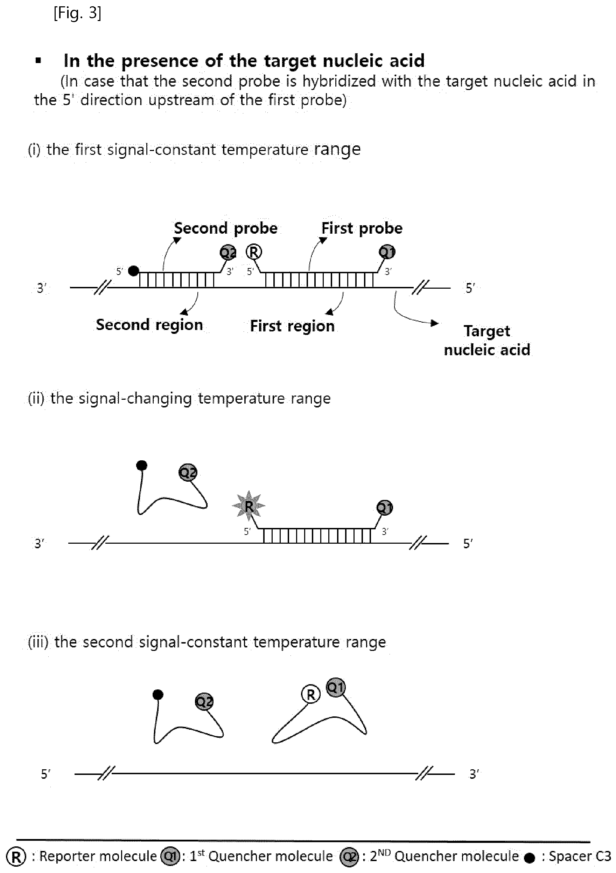

FIG. 3 shows the conformations of the first probe and the second probe in the presence of the target nucleic acid in (i) a first signal-constant temperature range, (ii) a signal-changing temperature range, and (iii) a second signal-constant temperature range, in a specific embodiment where the second probe hybridizes with the target nucleic acid in the 5' direction upstream of the first probe.

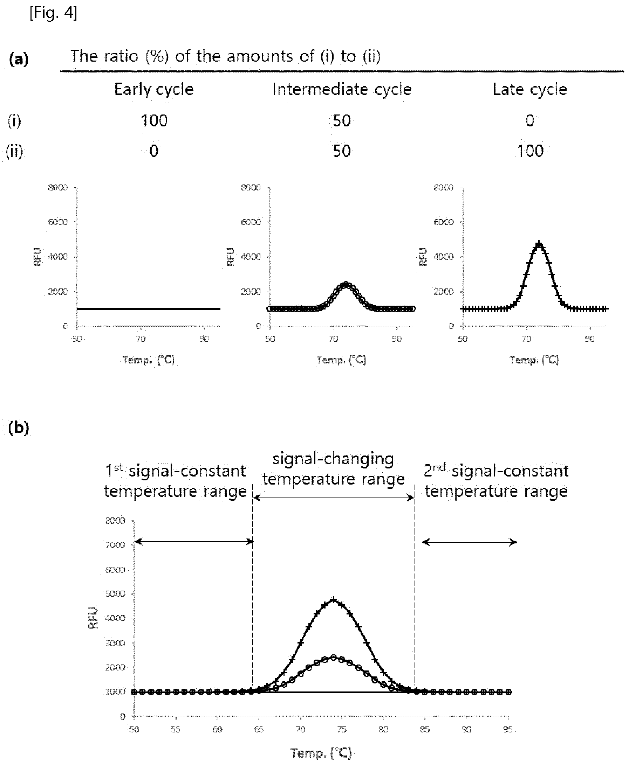

FIG. 4 illustrates the melt curve for the target nucleic acid amplification reaction using a composition for detecting a target nucleic acid, which comprises two probes having different Tm values, according to the present disclosure. FIG. 4(a) presents (i) the total amount ratio (or abundance ratio, %) of the first probe and/or the second probe before reacting with the target nucleic acid and (ii) the total amount ratio (or abundance ratio, %) of the first probe and/or the second probe that has reacted with the target nucleic acid, in the early, intermediate, and late cycles of the target nucleic acid amplification reaction using a composition for detecting a target nucleic acid, which comprises two probes having different Tm values. The lower graph represents the melt curves in the early, intermediate, and late cycles. Specifically, the numbers in row (i) indicate the total amount ratio (or abundance ratio, %) of the first probe and the second probe present in the forms (i) to (iii) of FIG. 1, while the numbers in row (ii) indicate the total amount ratio (or abundance ratio, %) of the first probe and the second probe present in the forms (i) to (iii) of FIG. 2 or in the forms (i) to (iii) of FIG. 3. FIG. 4(b) presents merged melt curves at each cycle in FIG. 4(a).

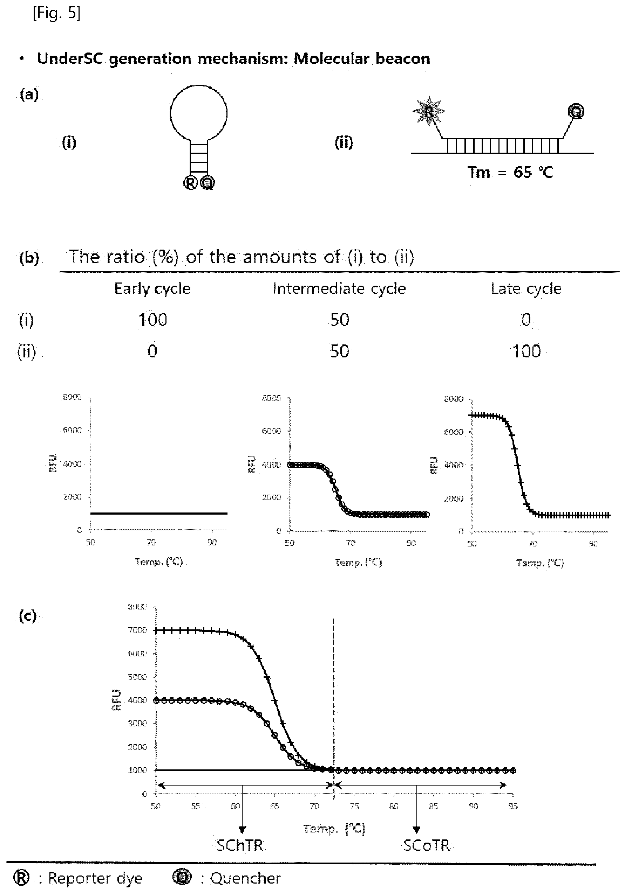

FIG. 5 illustrates the melt curves in the early, intermediate, and late cycles of target nucleic acid amplification using a molecular beacon method, which is applicable as an UnderSC signal generation mechanism. (a) represents the conformations of the molecular beacon depending on the presence or absence of the target nucleic acid, wherein (i) shows the conformation of the molecular beacon either in the absence of the target nucleic acid or before reacting with it, and (ii) shows the conformation of the molecular beacon after reacting with the target nucleic acid. (b) represents the melt curves in the early, intermediate, and late cycles of target nucleic acid amplification. (c) presents merged melt curves of (b) for each of the early, intermediate, and late cycles.

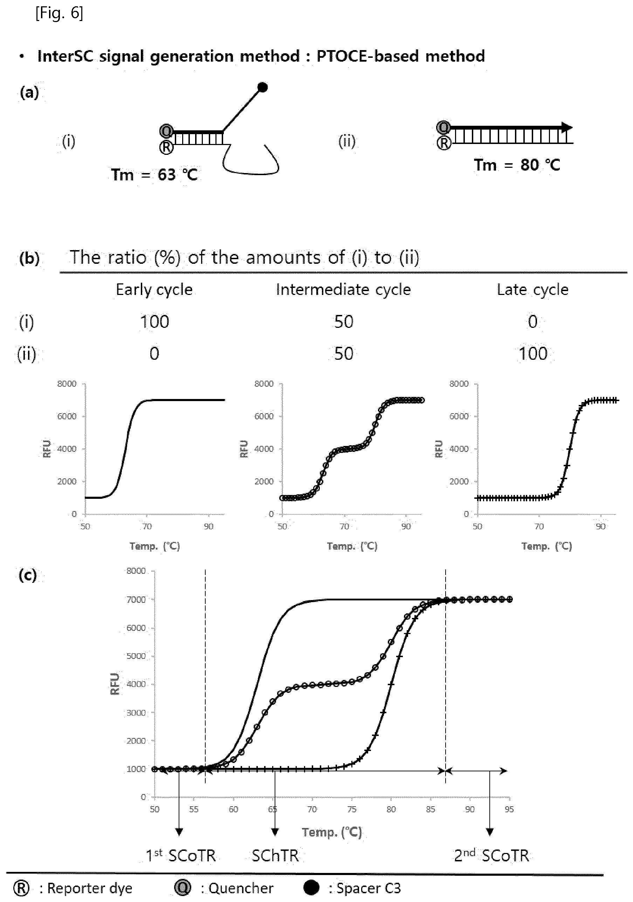

FIG. 6 illustrates the melt curves in the early, intermediate, and late cycles of target nucleic acid amplification using a PTOCE-based method, which is applicable as an InterSC signal generation mechanism. FIG. 6(a) represents the conformations of oligonucleotides in the PTOCE-based method depending on the presence or absence of the target nucleic acid, wherein (i) shows the conformation of the oligonucleotides (PTO and CTO) either in the absence of the target nucleic acid or before reacting with it, and (ii) shows the conformation of the oligonucleotides (extended duplex) after reacting with the target nucleic acid. FIG. 6(b) represents the melt curves in the early, intermediate, and late cycles of target nucleic acid amplification. (c) presents merged melt curves of (b) for each of the early, intermediate, and late cycles.

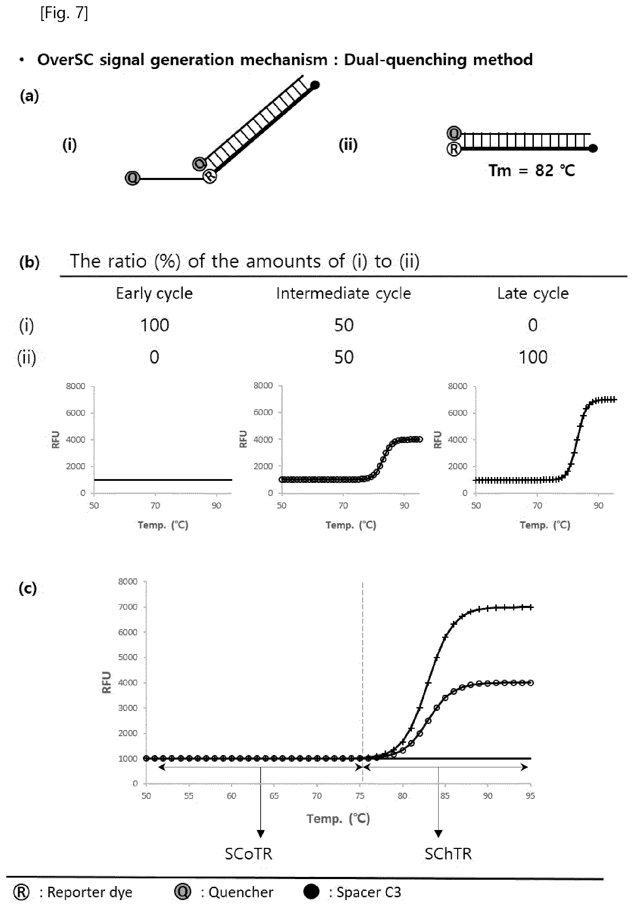

FIG. 7 illustrates the melt curves in the early, intermediate, and late cycles of target nucleic acid amplification using a dual-quenching method, which is applicable as an OverSC signal generation mechanism. FIG. 7(a) represents the conformations of oligonucleotides in the dual-quenching method depending on the presence or absence of the target nucleic acid, wherein (i) shows the conformation of the oligonucleotides (PTO and CQO) either in the absence of the target nucleic acid or before reacting with it, and (ii) shows the conformation of the oligonucleotides (activated tag duplex fragment) after reacting with the target nucleic acid. FIG. 7(b) represents the melt curves in the early, intermediate, and late cycles of target nucleic acid amplification. FIG. 7(c) presents merged melt curves of (b) for each of the early, intermediate, and late cycles.

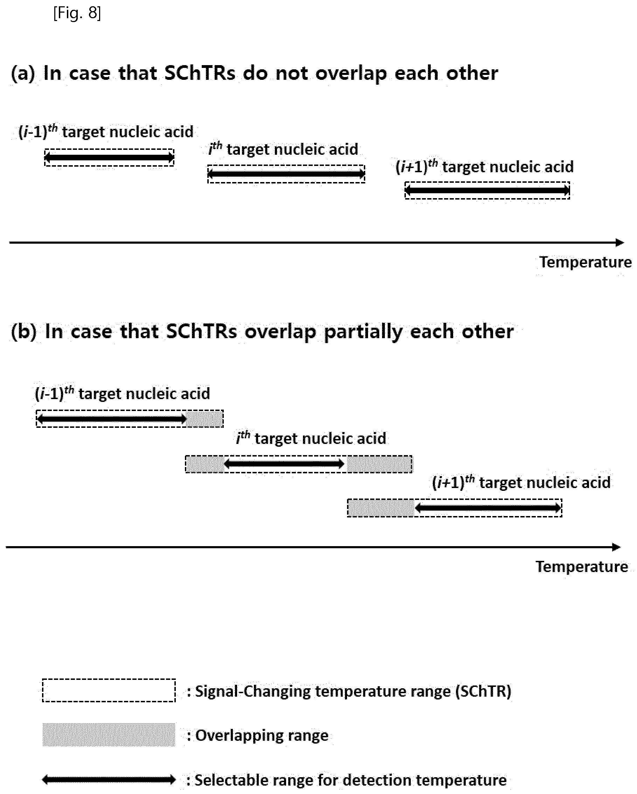

FIG. 8 illustrates the temperature ranges within which detection temperatures can be selected, respectively, when SChTRs do not overlap or partially overlap.

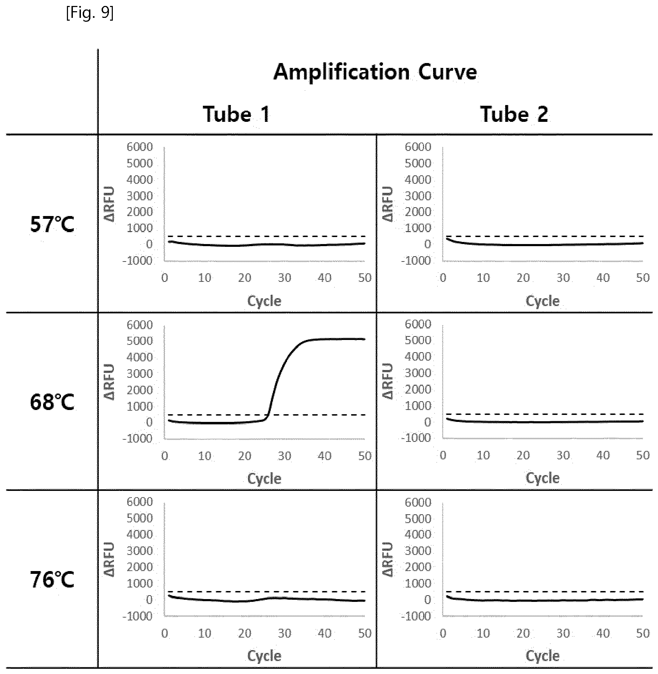

FIG. 9 shows the amplification curve results at different temperatures in real time PCR according to Example 1.

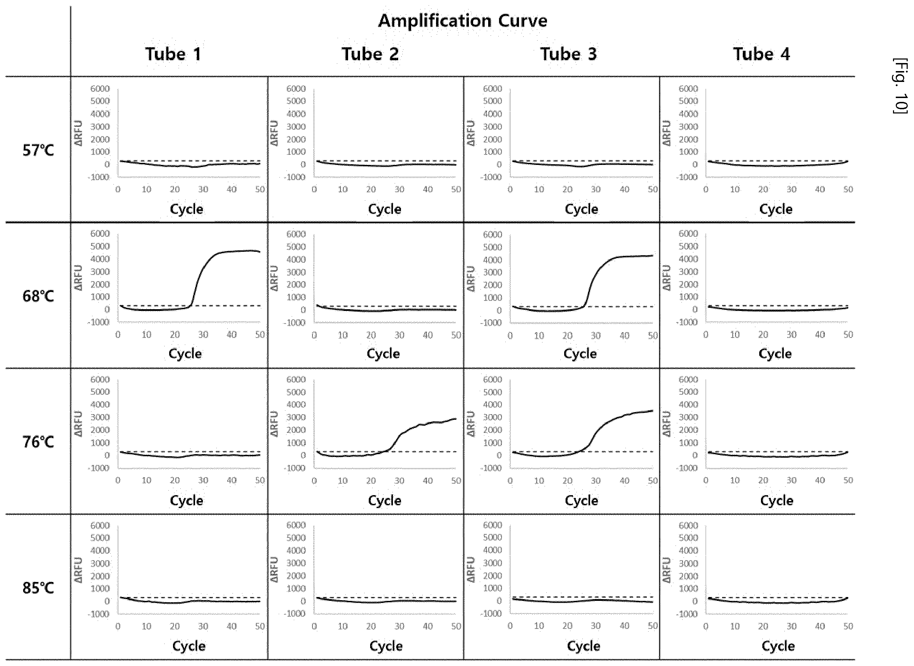

FIG. 10 shows the amplification curve results at different temperatures in multiplex real-time PCR according to Example 2.

### Best Mode

The present inventors have endeavored to develop a novel method for detecting target nucleic acids in real time with improved convenience and high efficiency. In particular, efforts were made to develop a method that can detect multiple target nucleic acid sequences using a single type of label. As a result, the present inventors confirmed that when two probes having different Tm values capable of hybridizing adjacent to the target nucleic acid are used, the target nucleic acid can be detected. Furthermore, it was confirmed that multiple target nucleic acids can be detected using a single type of label.

### I. Method for Detecting n Target Nucleic Acids in a Sample

In one aspect, the present disclosure provides a method for detecting *n* target nucleic acids in a sample, comprising:
(a) detecting signals at *n* detection temperatures, while incubating with *n* compositions for detecting the target nucleic acids, a sample suspected of containing at least one of the *n* target nucleic acids in a reaction vessel;
   wherein *n* is an integer of 2 or more,
   wherein the incubating comprises a plurality of cycles and the detection of signals is carried out at at least one of the plurality of cycles,
   wherein each of the *n* compositions for detecting the target nucleic acids provides a signal change at a corresponding detection temperature among the *n* detection temperatures in the presence of a corresponding target nucleic acid, the signal change indicating the presence of a corresponding target nucleic acid,
   wherein a composition for detecting an *i^{th}* target nucleic acid among the *n* compositions for detecting the target nucleic acids provides a signal change at an *i^{th}* detection temperature among the *n* detection temperatures and provides a constant signal at the other detection temperatures in the presence of the *i^{th}* target nucleic acid, the signal change indicating the presence of the *i^{th}* target nucleic acid,
   wherein *i* represents an integer from 1 to *n,* and the *i^{th}* detection temperature is lower than the (*i+*1)*^{th}* detection temperature,
   wherein in the temperature range covering all of the *n* detection temperatures, the composition for detecting the *i^{th}* target nucleic acid has a signal-changing temperature range in which the signal changes depending on the presence of the *i^{th}* target nucleic acid, and one or two signal-constant temperature ranges in which the signal is constant even in the presence of the *i^{th}* target nucleic acid,
   wherein the composition for detecting the *i^{th}* target nucleic acid is any one of:
      (i) an Under-Signal-Change (UnderSC) composition having a characteristic that the signal-changing temperature range is lower than the signal-constant temperature range,
      (ii) an Inter-Signal-Change (InterSC) composition having a characteristic that the signal-changing temperature range is higher than one of two signal-constant temperature ranges, and lower than the other of the two signal-constant temperature ranges, and
      (iii) an Over-Signal-Change (OverSC) composition having a characteristic that the signal-changing temperature range is higher than the signal-constant temperature range,
   wherein at least one of the *n* compositions for detecting target nucleic acids comprises a first probe and a second probe having different Tm values,
   wherein the first probe comprises a hybridizing nucleotide sequence to a first region of a corresponding target nucleic acid,
   wherein the first probe has a reporter molecule and a first quencher molecule,
   wherein the reporter molecule and the first quencher molecule are located at positions such that (i) the reporter molecule and the first quencher molecule are in close proximity to each other, prior to hybridization of the first probe with the corresponding target nucleic acid, thereby causing the first quencher molecule to quench a signal from the reporter molecule, and (ii) the reporter molecule and the first quencher molecule are separated upon hybridization of the first probe with the corresponding target nucleic acid, thereby causing the first quencher molecule to unquench a signal from the reporter molecule,
   wherein the second probe comprises a hybridizing nucleotide sequence to a second region of the corresponding target nucleic acid,
   wherein the second probe has a second quencher molecule,
   wherein the Tm value of the first probe is higher than the Tm value of the second probe, and
   wherein the reporter molecule of the first probe is quenched by the second quencher molecule of the second probe when the first probe and the second probe are hybridized with the corresponding target nucleic acid; and
(b) determining the presence of the *n* target nucleic acids from the signals detected in step (a), wherein the presence of the *i^{th}* target nucleic acid is determined by the signal change detected at the *i^{th}* detection temperature.

Terms such as "first," "second," "A," "B," "(a)," "(b)," "(i)," and "(ii)" may be used to describe the components of the present disclosure. These terms are merely used to distinguish one component from another and do not limit the nature, order, sequence, or other characteristics of the components.

The present invention is described in detail below as follows:

### Step (a): Incubating and Detection of Signals

First, a sample suspected of containing at least one of the *n* target nucleic acids is mixed with *n* compositions for detecting target nucleic acids in a single reaction vessel and incubated.

The term "target nucleic acid", "target nucleic acid sequence", or "target sequence" as used herein refers to a nucleic acid sequence to be detected or quantified. The target nucleic acid includes a single-strand as well as a double-strand. The target nucleic acid includes, not only a sequence initially present in a nucleic acid sample, but also a sequence newly generated in a reaction.

The target nucleic acid includes any DNA (gDNA and cDNA) RNA molecules, and their hybrids (chimeric nucleic acids). The target nucleic acid may be in a double-stranded or single-stranded form.

Target nucleic acids include any naturally occurring prokaryotic, eukaryotic (for example, protozoans and parasites, fungi, yeast, higher plants, lower animals, and higher animals, including mammals and humans) or viral (for example, Herpes virus, HIV, influenza virus, Epstein-Barr virus, hepatitis virus, polio virus, etc.) or viroid nucleic acid. In addition, the nucleic acid molecule may be any nucleic acid molecule which is produced or can be produced by recombination, or any nucleic acid molecule which is or can be chemically synthesized. As such, the nucleic acid sequence may or may not be found in nature. The target nucleic acid sequence may be a known or unknown sequence.

In one embodiment, the *n* target nucleic acids may include a nucleotide variation. For example, one of the *n* target nucleic acids may include one type of nucleotide variation, and another of the *n* target nucleic acids may include a different type of nucleotide variation.

As used herein, the term "nucleotide variation" refers to any single or multiple nucleotide substitutions, deletions or insertions in a DNA sequence at a particular location among contiguous DNA segments. Such contiguous DNA segments include a gene or any other portion of a chromosome. These nucleotide variations may be mutant or polymorphic allele variations. For example, the nucleotide variation detected in the present disclosure includes single nucleotide polymorphism (SNP), mutation, deletion, insertion, substitution and translocation. Examples of nucleotide variation include numerous variations in a human genome (e.g., variations in the MTHFR (methylenetetrahydrofolate reductase) gene), variations involved in drug resistance of pathogens and tumorigenesis-causing variations. As used herein, the term "nucleotide variation" includes any variation at a particular location in a nucleic acid sequence. That is, the term "nucleotide variation" includes a wild type and any mutation thereof, at a particular location in a nucleic acid sequence.

The *n* target nucleic acids herein may be genes from *n* different organisms, *n* different genes from the same organism, or combinations thereof.

As used herein, the term "sample" refers to any cell, tissue, or fluid from a biological source, or any other medium that can advantageously be evaluated according to the present invention, and includes virus, bacteria, tissues, cells, blood, serum, plasma, lymph, milk, urine, feces, intraocular fluid, saliva, semen, brain extract, spinal fluid, appendix, spleen and tonsil tissue extracts, amniotic fluid, ascites, and non-biological samples (e.g., food and water). In addition, the sample includes naturally occurring nucleic acid molecules isolated from a biological source, and synthesized nucleic acid molecules. Further, the sample may be a lysate, an extract or an isolated target nucleic acid itself of a specific specimen.

As used herein, the incubation reaction refers to any reaction that induces a signal change depending on the presence of a corresponding target nucleic acid at a corresponding detection temperature, as each of the target nucleic acids reacts with a corresponding composition for detecting a target nucleic acid.

Herein, the incubating comprises a plurality of cycles.

In one embodiment, the incubating in step (a) may include an amplification reaction, and may include, for example, a signal amplification reaction and/or a nucleic acid amplification reaction.

In one embodiment, the amplification reaction comprises a plurality of cycles.

In one embodiment, the incubating in step (a) is carried out under conditions that allow target amplification and a signal change by a composition for detecting a target nucleic acid. Such conditions include a temperature, salt concentration and pH of a solution.

In one embodiment, the incubating in step (a) is carried out at a signal amplification process with no nucleic acid amplification.

In one embodiment, the signal may be amplified simultaneously with target amplification. Alternatively, the signal may be amplified without target amplification.

In one embodiment, the signal change takes place during a process including signal amplification and target amplification.

In one embodiment, the amplification of the target nucleic acid may be carried out by a polymerase chain reaction (PCR). The PCR is widely used in the art to amplify target nucleic acids and includes cycles of denaturation of target nucleic acids, annealing (hybridization) between target nucleic acids and primers, and extension of primers (Mullis et al., U.S. Pat. Nos. 4,683,195, 4,683,202, and 4,800,159; Saiki et al., (1985) Science 230, 1350-1354).

In one embodiment, the amplification of target nucleic acids may be carried out by ligase chain reaction (LCR) (U.S. Pat. Nos. 4,683,195 and 4,683,202; PCR Protocols: A Guide to Methods and Applications (Innis et al., eds, 1990)), strand displacement amplification (SDA) (Walker, et al., Nucleic Acids Res. 20(7):1691-6 (1992); Walker PCR Methods Appl 3(1):1-6 (1993)), transcription-mediated amplification (Phyffer, et al., J. Clin. Microbiol. 34:834-841 (1996); Vuorinen, et al., J. Clin. Microbiol 33:1856-1859 (1995)), helicase dependent amplification (HAD) (M. Vincent, Y. Xu and H. Kong, EMBO Rep., 2004, 5, 795-800), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatch et al., Genet. Anal. 15(2):35-40 (1999)), Q-Beta Replicase (Lizardi et al., BiolTechnology6:1197 (1988)), Loop-mediated isothermal amplification (LAMP) (Y. Mori, H. Kanda and T. Notomi, J. Infect. Chemother, 2013, 19, 404-411), or Recombinase Polymerase Amplification (RPA) (J. Li, J. Macdonald and F. von Stetten, Analyst, 2018, 144, 31-67).

Various DNA polymerase may be used in the amplification reaction and include the "Klenow" fragment of E. coli DNA polymerase I, thermostable DNA polymerase, bacteriophage T7 DNA polymerase, Vent^{®} polymerase, and Terminator^{™} polymerase. In particular, the polymerase is a thermostable DNA polymerase obtainable from various bacteria, which include *Thermus aquaticus* (Taq), *Thermus thermophilus* (Tth), *Thermus filiformis, Thermus flavus, Thermococcus litoralis,* and *Pyrococcus furiosus* (Pfu). Most of the above polymerases can be isolated as is from bacteria, or can be commercially purchased.

The above-described amplification method can amplify target nucleic acid and/or signal through a repetition of a series of reactions with or without change in temperature. The unit of amplification including the repetition of such series of reactions, is referred to as a "cycle". The cycle may be expressed as the number of repetitions or a duration, depending on the amplification method used.

In one embodiment, the series of reactions may be carried out sequentially. For example, for a PCR, after denaturation of target nucleic acids (that is, templates), annealing of primers and subsequently, extension of the primers may be carried out sequentially. In this case, the cycle may be expressed as the number of repetitions.

In one embodiment, the series of reactions may be carried out simultaneously. For example, in the LAMP, which is an isothermal amplification assay, an annealing reaction of the primers on some templates among a plurality of templates and an extension reaction of already annealed primers on other templates may occur at the same time. In this case, the cycle may be expressed as a duration. In particular, 1 cycle may be 5 seconds, 10 seconds, 1 minute, 2 minutes, 3 minutes, 4 minutes, 5 minutes, 10 minutes, 20 minutes, 30 minutes, 1 hour, or 2 hours.

In one embodiment, the incubating may be carried out for a plurality of cycles that allows for the measurement of a signal change dependent on the presence of a target nucleic acid. For example, the plurality of cycles may include 2 to 100 cycles, 2 to 90 cycles, 2 to 80 cycles, 2 to 70 cycles, 2 to 60 cycles, 2 to 50 cycles, 2 to 40 cycles, 2 to 30 cycles, 2 to 20 cycles, 2 to 10 cycles, 5 to 100 cycles, 5 to 90 cycles, 5 to 80 cycles, 5 to 70 cycles, 5 to 60 cycles, 5 to 50 cycles, 5 to 40 cycles, 5 to 30 cycles, 5 to 20 cycles, 5 to 10 cycles, 10 to 100 cycles, 10 to 90 cycles, 10 to 80 cycles, 10 to 70 cycles, 10 to 60 cycles, 10 to 50 cycles, 10 to 40 cycles, 10 to 30 cycles, 10 to 20 cycles, 20 to 100 cycles, 20 to 90 cycles, 20 to 80 cycles, 20 to 70 cycles, 20 to 60 cycles, 20 to 50 cycles, 20 to 40 cycles, or 20 to 30 cycles, and particularly, may include 10 cycles, 15 cycles, 20 cycles, 25 cycles, 30 cycles, 35 cycles, 40 cycles, 45 cycles, or 50 cycles.

In one embodiment, the detection of signals may be carried out in each cycle, in a selected few cycles, or at the end-point of an incubation reaction including a plurality of cycles.

In one embodiment, the amplification reaction of a target nucleic acid may be a multiple target nucleic acid amplification reaction.

The term "multiple target nucleic acid amplification reaction" used herein refers to a reaction that amplifies two or more nucleic acids as targets in a single reaction vessel. The multiple target nucleic acid amplification reaction refers to a reaction that amplifies two or more nucleic acids together. For example, the multiple target nucleic acid amplification reaction may amplify, in a single reaction, 2 or more, 3 or more, 4 or more, 5 or more, 6 or more, 7 or more, 8 or more, 9 or more, 10 or more, 20 or more, 30 or more, 40 or more, or 50 or more of target nucleic acids together.

In one embodiment, the method according to the present disclosure may detect 2 to 50, 2 to 40, 2 to 30, 2 to 20, 2 to 15, 2 to 12, 2 to 10, 2 to 9, 2 to 8, 2 to 7, 2 to 6, 2 to 5, 3 to 50, 3 to 40, 3 to 30, 3 to 20, 3 to 15, 3 to 12, 3 to 10, 3 to 9, 3 to 8, 3 to 7, 3 to 6, 3 to 5, 4 to 50, 4 to 40, 4 to 30, 4 to 20, 4 to 15, 4 to 12, 4 to 10, 4 to 9, 4 to 8, 4 to 7, 4 to 6 or 4 to 5 target nucleic acids by using a single type of label in a single reaction vessel.

In one embodiment, the method according to the present disclosure, when using multiple types of labels, may detect a greater number of target nucleic acids than the number of target nucleic acids detectable using a single type of label according to the method of the present disclosure, and for example, may be able to detect more target nucleic acids by a fold increase of the number of label types used.

The method according to the present disclosure is used to determine whether at least one of *n* target nucleic acids is present in a sample. For example, when *n is* 2, the present disclosure may be used to determine whether at least one of a first target nucleic acid and a second target nucleic acid is present in the sample. In another example, when *n* is 3, the present disclosure may be used to determine whether at least one of a first target nucleic acid, a second target nucleic acid, and a third target nucleic acid is present in the sample.

In one embodiment, *n* is an integer of 2 or more. For example, *n* may be 2, 3, 4, 5, 6, 7, 8, 9, 10, 11, 12, 13, 14, 15, 20, 25, 30, 35, 40, 45, or 50, but is not limited thereto.

In the present disclosure, a combination of compositions for detecting *n* target nucleic acids, *i.e.,* the first to the *n*^{th} target nucleic acids, is used to detect each of *n* target nucleic acids, *i.e.,* the first to the *n*^{th} target nucleic acids, wherein *n* is an integer of 2 or more.

As used herein, the term "combination of compositions for detecting the first to the *n*^{th} target nucleic acids" refers to a compilation or mixture of compositions specific for each of the first to *n*^{th} target nucleic acids. Herein, a composition for detecting one target nucleic acid is specific for a corresponding target nucleic acid. The expression "a composition for detecting a target nucleic acid is specific for a corresponding target nucleic acid" means that the composition for detecting the target nucleic acid is involved in detecting the corresponding target nucleic acid, but is not involved in detecting other target nucleic acids. In other words, the expression means that the composition for detecting the target nucleic acid interacts with the corresponding target nucleic acid but does not interact with other target nucleic acids.

Herein, the composition for detecting the *n*^{th} target nucleic acid is specific for the *n*^{th} target nucleic acid. For example, the composition for detecting the first target nucleic acid is specific for the first target nucleic acid, the composition for detecting the second target nucleic acid is specific for the second target nucleic acid, and the composition for detecting the third target nucleic acid is specific to the third target nucleic acid.

The combination of compositions for detecting the first to the *n*^{th} target nucleic acids as used herein is employed in a single reaction. In other words, the compositions for detecting the first to the *n*^{th} target nucleic acids coexist in a single reaction solution or reaction vessel.

As used herein, the term "composition for detecting a target nucleic acid" refers to a composition comprising components used to detect a target nucleic acid.

According to the method of the present disclosure, each of the compositions for detecting the first to *n*^{th} target nucleic acids comprises a label that provides a signal depending on the presence of a corresponding target nucleic acid among the first to *n*^{th} target nucleic acids, and the signals provided from each of the compositions for detecting the first to *n*^{th} target nucleic acids are not distinguished from each other by a single detection channel.

The composition for detecting a target nucleic acid may comprises various oligonucleotides involved in amplifying and detecting the target nucleic acid.

The label herein may be linked to an oligonucleotide or may exist in free form. Alternatively, the label may be incorporated into the oligonucleotide during the incubating.

Although the label and oligonucleotide are described as key elements in the compositions for detecting the first to *n*^{th} target nucleic acids, it should be understood that various other components may be also included in the compositions.

Examples of components included in the composition for detecting the target nucleic acid include, but are not limited to, an oligonucleotide set used to amplify or detect the target nucleic acid, a label, a nucleic acid polymerase, a buffer, a polymerase cofactor, and a deoxyribonucleotide-5-triphosphate. Optionally, the composition for detecting the target nucleic acid may also include various polynucleotide molecules, reverse transcriptase, various buffers and reagents, and antibodies that inhibit nucleic acid polymerase activity. The composition for detecting the target nucleic acid may also include an oligonucleotide set or reagents necessary for performing a positive control reaction. Optimal amounts of reagents to be used in a given reaction can be readily determined by one of ordinary skill in the art having the benefit of the present disclosure. The above-described components of the composition for detecting the target nucleic acid may be present or stored in one or more containers before the reaction.

International Patent Application Publication WO 2022-265463 discloses that various signal generation mechanisms known in the art for detecting a target nucleic acid have a signal-changing temperature range (SChTR) in which the signal changes depending on the presence of the target nucleic acid, and one or two signal-constant temperature ranges (SCoTRs) in which the signal is constant even in the presence of the target nucleic acid. In addition, the literature discloses that various conventional signal generation mechanisms can be categorized into any one of the following based on the number and order of this signal-changing temperature range and these signal-constant temperature ranges: (i) an Under-Signal-Change (UnderSC) signal generation mechanism having a characteristic that the signal-changing temperature range is lower than the signal-constant temperature range, (ii) an Inter-Signal-Change (InterSC) signal generation mechanism having a characteristic that the signal-changing temperature range is higher than one of two signal-constant temperature ranges, and lower than the other of the two signal-constant temperature ranges, and (iii) an Over-Signal-Change (OverSC) signal generation mechanism having a characteristic that the signal-changing temperature range is higher than the signal-constant temperature range.

The *n* compositions for detecting target nucleic acids used herein also apply any one of the three signal generation mechanisms. That is, each of the *n* compositions for detecting target nucleic acids used in the present disclosure is any one of (i) an Under-Signal-Change (UnderSC) composition, (ii) an Inter-Signal-Change (InterSC) composition, and (iii) an Over-Signal-Change (OverSC) composition.

In one embodiment, when *n* is 2, the composition for detecting the first target nucleic acid is an UnderSC or InterSC composition, and the composition for detecting the second target nucleic acid is an InterSC or OverSC composition.

In one embodiment, when *n is* 3 or more, the composition for detecting the first target nucleic acid is an UnderSC or InterSC composition, the composition for detecting the *n*^{th} target nucleic acid is an InterSC or OverSC composition, and each composition for detecting target nucleic acids other than the first nucleic acid and *n*^{th} target nucleic acid is an InterSC composition.

Additionally, at least one of the *n* compositions for detecting target nucleic acids comprises a first probe and a second probe having different Tm values.

The term "probe" as used herein refers to a single-stranded nucleic acid molecule comprising one or more portions substantially complementary to a target nucleic acid sequence.

The "composition for detecting a target nucleic acid comprising a first probe and a second probe having different Tm values," which is used as at least one of the compositions for detecting *n* target nucleic acids in the present disclosure, is described in more detail below with reference to the drawings, but is not limited thereto.

FIG. 1 shows the conformations of the first probe and the second probe in (i) a first signal-constant temperature range, (ii) a signal-changing temperature range, and (iii) a second signal-constant temperature range, in the absence of the target nucleic acid or before reaction between the target nucleic acid and the composition for detecting the target nucleic acid comprising two probes having different Tm values according to the present disclosure.

FIG. 2 and FIG. 3 show the conformations of the first probe and the second probe in the presence of the target nucleic acid in (i) the first signal-constant temperature range, (ii) the signal-changing temperature range, and (iii) the second signal-constant temperature range. Specifically, FIG. 2 and FIG. 3 illustrate the conformations of the first probe and the second probe in the amplified reaction product of the target nucleic acid (e.g., the product at the late cycle).

### (i) First Probe

The first probe comprises a hybridizing nucleotide sequence to a first region of the target nucleic acid. That is, the first probe is capable of hybridizing to the first region of the target nucleic acid.

The term "hybridize", "hybridizing" or "hybridization" as used herein refers to the formation of double strands by noncovalent association between two complementary single-stranded polynucleotides under certain hybridization conditions or stringent conditions.

In particular, the expression herein that one oligonucleotide "comprises a hybridizing nucleotide sequence" to another oligonucleotide means that all or a portion of one oligonucleotide has a complementary nucleotide sequence necessary for hybridization with all or a portion of another oligonucleotide.

The term "complementary" is used herein to mean that primers or probes are sufficiently complementary to hybridize selectively to a target nucleic acid under the designated annealing conditions or stringent conditions, encompassing the terms "substantially complementary" and "perfectly complementary", particularly perfectly complementary.

In contrast, the term "non-complementary" is used herein to mean that primers or probes are sufficiently non-complementary not to hybridize selectively to a target nucleic acid under the designated annealing conditions or stringent conditions, encompassing the terms "substantially non-complementary" and "perfectly non-complementary", particularly perfectly non-complementary.

Further, when referring to hybridization of a portion of one oligonucleotide to another oligonucleotide, the portion of one oligonucleotide can be regarded as an individual oligonucleotide.

The hybridization may occur between two nucleic acid strands perfectly matched or substantially matched with some mismatches (e.g., 1-4 mismatches). The complementarity for hybridization may depend on hybridization conditions, particularly temperature.

The hybridization between two oligonucleotides (e.g., hybridization of the first probe and the target nucleic acid) may be carried out under suitable hybridization conditions routinely determined through optimization procedures. Conditions such as temperature, concentration of components, hybridization and washing times, buffer components, and their pH and ionic strength may be varied depending on various factors, including the length and GC content of the oligonucleotide (e.g., the first probe and the second probe) and the target nucleotide sequence. For instance, when a relatively short oligonucleotide is used, it is preferable that low-stringency conditions are adopted. The detailed conditions for hybridization can be found in Joseph Sambrook, et al., Molecular Cloning, A Laboratory Manual, Cold Spring Harbor Laboratory Press, Cold Spring Harbor, N.Y. (2001); and M. L. M. Anderson, Nucleic Acid Hybridization, Springer-Verlag New York Inc. N.Y. (1999).

There is no intended distinction between the terms "annealing" and "hybridizing", and these terms will be used interchangeably.

The first probe has a reporter molecule and a first quencher molecule.

In one embodiment, before the first probe hybridizes with the target nucleic acid, the reporter molecule and the first quencher molecule linked to the first probe are positioned in close proximity to each other. As a result, the first quencher molecule linked to the first probe quenches the signal from the reporter molecule linked to the first probe.

The expression herein "the reporter molecule and the quencher molecule are in close proximity to each other" means that (i) an oligonucleotide having both a reporter molecule and a quencher molecule forms a specific conformational structure, for example, a random coil or hairpin structure, so that the reporter molecule and the quencher molecule are three-dimensionally adjacent to each other or (ii) an oligonucleotide having the reporter molecule and an oligonucleotide having the quencher molecule form a double strand so that the reporter molecule and the quencher molecule are in close proximity to each other.

In one embodiment, the first probe forms a random coil or hairpin structure, prior to hybridization of the first probe with the target nucleic acid, thereby allowing the first quencher molecule linked to the first probe to intramolecularly quench a signal from the reporter molecule linked to the first probe.

In one embodiment, when the first probe hybridizes with the target nucleic acid, the reporter molecule and the first quencher molecule of the first probe are separated from each other. Specifically, upon hybridization of the first probe with the target nucleic acid, the first quencher molecule of the first probe is separated from the reporter molecule of the first probe, thereby causing the first quencher molecule to unquench a signal from the reporter molecule.

The expression herein "the reporter molecule and the quencher molecule are separated from each other" means that (i) an oligonucleotide having both a reporter molecule and a quencher molecule forms a double strand with another oligonucleotide to undergo its conformational change such as disruption of a hairpin structure, so that the reporter molecule and the quencher molecule are three-dimensionally separated or (ii) an oligonucleotide having the reporter molecule and an oligonucleotide having the quencher molecule in a double stranded state dissociate from each other, so that the reporter molecule and the quencher molecule are separated.

In one embodiment, the reporter molecule and the first quencher molecule are located at positions such that (i) the reporter molecule and the first quencher molecule are in close proximity to each other, prior to hybridization of the first probe with the target nucleic acid, thereby causing the first quencher molecule to quench a signal from the reporter molecule, and (ii) the reporter molecule and the first quencher molecule are separated upon hybridization of the first probe with the target nucleic acid, thereby causing the first quencher molecule to unquench a signal from the reporter molecule. Specifically, the first quencher molecule of the first probe is located apart from the reporter molecule of the first probe by 12 to 60 nucleotides, 15 to 60 nucleotides, 18 to 60 nucleotides, 20 to 60 nucleotides, 12 to 50 nucleotides, 15 to 50 nucleotides, 18 to 50 nucleotides, 20 to 50 nucleotides, 12 to 40 nucleotides, 15 to 40 nucleotides, 18 to 40 nucleotides, 20 to 40 nucleotides, 12 to 30 nucleotides, 15 to 30 nucleotides, 18 to 30 nucleotides, or 20 to 30 nucleotides, and more specifically, by 15 to 60 nucleotides, and even more specifically, by 18 to 30 nucleotides.

In one embodiment, the reporter molecule is linked to the 3'-end portion or the 5'-end portion of the first probe.

The term used herein "3'-end portion" or "5'-end portion" in conjunction with the first probe and/or the second probe refers to a portion or region comprising any lengthy consecutive sequence from the 3'-end or 5'-end of the probe. For example, the 3'-end portion of the probe refers to a sequence comprising 1 to 10 nucleotides from its 3'-end, specifically 1 to 5 nucleotides, and more specifically 1 to 3 nucleotides. Similarly, the 5'-end portion of the probe refers to a sequence comprising 1 to 10 nucleotides from its 5'-end, specifically 1 to 5 nucleotides, and more specifically 1 to 3 nucleotides.

### (i) Second Probe

The second probe comprises a hybridizing nucleotide sequence to a second region of the target nucleic acid. That is, the second probe is capable of hybridizing to the second region of the target nucleic acid.

Additionally, the second probe has a second quencher molecule.

In the present disclosure, the first region and the second region of the target nucleic acid are adjacent to each other.

The term used herein "adjacent," when referring to the first region and the second region of the target nucleic acid, refers to a state in which the reporter molecule of the first probe hybridized with the first region of the target nucleic acid and the second quencher molecule of the second probe hybridized with the second region of the target nucleic acid are sufficiently close to interact with each other. That is, as long as the second quencher molecule of the second probe hybridized with the second region of the target nucleic acid can quench a signal from the reporter molecule of the first probe hybridized with the first region of the target nucleic acid, the first region and the second region of the target nucleic acid may be positioned immediately adjacent to each other without any separation or in close proximity, being apart from each other by a few nucleotides. In one embodiment, the first region and the second region of the target nucleic acid may be positioned immediately adjacent to each other. For example, the first region and the second region of the target nucleic acid may be positioned in close proximity to form a nick. In another embodiment, the first region and the second region of the target nucleic acid may be positioned in close proximity to each other. For example, the first region of the target nucleic acid may be positioned apart from the second region of the target nucleic acid by 1 to 30 nucleotides, 1 to 20 nucleotides, 1 to 15 nucleotides, 1 to 10 nucleotides, 1 to 5 nucleotides, or 1 to 4 nucleotides. In yet another embodiment, the first region and the second region of the target nucleic acid may overlap with each other. For example, 1 to 10 nucleotides, 1 to 7 nucleotides, 1 to 5 nucleotides, 1 to 3 nucleotides, or 1 to 2 nucleotides may overlap.

In one embodiment, when the first probe and the second probe hybridize with the target nucleic acid, the reporter molecule of the first probe is quenched by the second quencher molecule of the second probe. Specifically, when the first probe is hybridized with the first region of the target nucleic acid, the second probe hybridizes with the second region of the target nucleic acid to allow the second quencher molecule of the second probe to be adjacent to the reporter molecule of the first probe hybridized with the first region of the target nucleic acid. As a result, the second quencher molecule intermolecularly quenches a signal from the reporter molecule.

In one embodiment, when the first probe and the second probe hybridize with the target nucleic acid, the second quencher molecule of the second probe is positioned adjacent to the reporter molecule of the first probe. As a result, the reporter molecule and the second quencher molecule are located at positions such that the second quencher molecule can quench a signal from the reporter molecule. Specifically, when the first probe and the second probe are hybridized with the target nucleic acid, the second quencher molecule is located immediately adjacent to or 1 to 4 nucleotides apart from the reporter molecule. More specifically, the second quencher molecule is 1, 2, 3, or 4 nucleotides apart from the reporter molecule.

In one embodiment, the second quencher molecule is linked to the 3'-end portion or the 5'-end portion of the second probe.

In one embodiment, when the second probe is hybridized with the target nucleic acid in the 3' direction downstream of the first probe, the reporter molecule is located at the 3'-end portion of the first probe, and the second quencher molecule is located at the 5'-end portion of the second probe.

The expression used herein "the second probe is hybridized with the target nucleic acid in the 3' direction downstream of the first probe," when describing the relative positions of the first probe and the second probe, means that the second probe hybridizes with the target nucleic acid such that the 5'-end of the second probe is positioned adjacent to the 3'-end of the first probe hybridized with the target nucleic acid. Describing this in terms of the first region and the second region of the target nucleic acid, the first region of the target nucleic acid is located downstream in the 3'-direction of the second region of the target nucleic acid (see FIG. 2).

In one embodiment, when the second probe is hybridized with the target nucleic acid in the 5' direction upstream of the first probe, the reporter molecule is located at the 5'-end portion of the first probe, and the second quencher molecule is located at the 3'-end portion of the second probe.

The expression used herein "the second probe is hybridized with the target nucleic acid in the 5' direction upstream of the first probe," when describing the relative positions of the first probe and the second probe, means that the second probe hybridizes with the target nucleic acid such that the 3'-end of the second probe is positioned adjacent to the 5'-end of the first probe hybridized with the target nucleic acid. Describing this in terms of the first region and the second region of the target nucleic acid, the first region of the target nucleic acid is located upstream in the 5'-direction of the second region of the target nucleic acid (see FIG. 3).

In one embodiment, the first probe and/or the second probe are "blocked" at its 3'-end to prevent its extension. The blocking may be achieved according to a conventional method. For example, the blocking may be performed by adding a chemical moiety such as biotin, label, a phosphate group, an alkyl group, a non-nucleotide linker, a phosphorothioate or alkane-diol residue to the 3'-hydroxyl group of the last nucleotide. Alternatively, the blocking may be performed by removing the 3'-hydroxyl group of the last nucleotide or by using a nucleotide without the 3'-hydroxyl group, such as dideoxynucleotide.

The reporter molecule and the quencher molecule used in the present disclosure are interactive labels. As a representative example of the interactive label system, the FRET (fluorescence resonance energy transfer) label system includes a fluorescent reporter molecule (donor molecule) and a quencher molecule (acceptor molecule). In FRET, the energy donor is fluorescent, but the energy acceptor may be fluorescent or non-fluorescent. In another form of interactive label systems, the energy donor is non-fluorescent, e.g., a chromophore, and the energy acceptor is fluorescent. In yet another form of interactive label systems, the energy donor is luminescent, e.g., bioluminescent, chemiluminescent, or electrochemiluminescent, and the acceptor is fluorescent. The interactive label system includes a label pair based on "contact-mediated quenching" (Salvatore et al., Nucleic Acids Research, 2002 (30) no.21 e122 and Johansson et al., J. AM. CHEM. SOC 2002 (124) pp 6950-6956). The interactive label system includes any label system in which signal change is induced by interaction between at least two molecules (e.g., dyes).

The reporter molecule and the quencher molecule useful as interactive labels may include any molecule known in the art. Examples of those are: Cy2^{™} (506), YO-PRO^{™}-1 (509), YOYO^{™}-1 (509), Calcein (517), FITC (518), FluorX^{™} (519), Alexa^{™} (520), Rhodamine 110 (520), Oregon Green^{™} 500 (522), Oregon Green^{™} 488 (524), RiboGreen^{™} (525), Rhodamine Green^{™} (527), Rhodamine 123 (529), Magnesium Green^{™}(531), Calcium Green^{™} (533), TO-PRO^{™}-1 (533), TOTO1 (533), JOE (548), BODIPY530/550 (550), Dil (565), BODIPY TMR (568), BODIPY558/568 (568), BODIPY564/570 (570), Cy3^{™} (570), Alexa^{™} 546 (570), TRITC (572), Magnesium Orange^{™} (575), Phycoerythrin R&B (575), Rhodamine Phalloidin (575), Calcium Orange^{™} (576), Pyronin Y (580), Rhodamine B (580), TAMRA (582), Rhodamine Red^{™} (590), Cy3.5^{™} (596), ROX (608), Calcium Crimson^{™} (615), Alexa^{™} 594 (615), Texas Red (615), Nile Red (628), YO-PRO^{™}-3 (631), YOYO^{™}-3 (631), Rphycocyanin (642), C-Phycocyanin (648), TO-PRO^{™}-3 (660), TOTO3 (660), DiD DiIC(5) (665), Cy5^{™} (670), Thiadicarbocyanine (671), Cy5.5 (694), HEX (556), TET (536), Biosearch Blue (447), CAL Fluor Gold 540 (544), CAL Fluor Orange 560 (559), CAL Fluor Red 590 (591), CAL Fluor Red 610 (610), CAL Fluor Red 635 (637), FAM (520), Fluorescein (520), Fluorescein-C3 (520), Pulsar 650 (566), Quasar 570 (667), Quasar 670 (705) and Quasar 705 (610). The numeric in parenthesis is a maximum emission wavelength in nanometer. In a specific embodiment, the reporter molecule and the quencher molecule include JOE, FAM, TAMRA, ROX and fluorescein-based label.

Suitable reporter-quencher pairs are disclosed in various publications as follows: Pesce et al., editors, Fluorescence Spectroscopy (Marcel Dekker, New York, 1971); White et al., Fluorescence Analysis: A Practical Approach (Marcel Dekker, New York, 1970); Berlman, Handbook of Fluorescence Spectra of Aromatic Molecules, 2nd Edition (Academic Press, New York, 1971); Griffiths, Color AND Constitution of Organic Molecules (Academic Press, New York, 1976); Bishop, editor, Indicators (Pergamon Press, Oxford, 1972); Haugland, Handbook of Fluorescent Probes and Research Chemicals (Molecular Probes, Eugene, 1992); Pringsheim, Fluorescence and Phosphorescence (Interscience Publishers, New York, 1949); Haugland, R. P., Handbook of Fluorescent Probes and Research Chemicals, 6th Edition (Molecular Probes, Eugene, Oreg., 1996); U.S. Pat. Nos. 3,996,345 and 4,351,760.

In one embodiment, a non-fluorescent black quencher molecule capable of quenching fluorescence of a wide wavelength or a specific wavelength may be used. Examples of non-fluorescent black quencher molecules include BHQ and DABCYL.

In the FRET label applied to the present disclosure, the reporter comprises a donor of FRET and the quencher comprises the remaining partner (acceptor) of FRET. For example, a fluorescein dye is used as a reporter and a rhodamine dye is used as a quencher.

In one embodiment, the second quencher molecule linked to the second probe is the same type of quencher molecule as the first quencher molecule linked to the first probe. For example, when the first quencher molecule linked to the first probe is a non-fluorescent black quencher molecule (e.g., BHQ), the second quencher molecule linked to the second probe may be the same type of non-fluorescent black quencher molecule (e.g., BHQ). Similarly, when the first quencher molecule linked to the first probe is a fluorescent acceptor molecule (e.g., rhodamine dye), the second quencher molecule linked to the second probe may also be the same type of fluorescent acceptor molecule (e.g., rhodamine dye).

Through the incubation reaction in step (a) described above, the composition for detecting the target nucleic acid according to the present disclosure, which comprises the first probe and the second probe, reacts with the target nucleic acid to provide a signal dependent on the presence of the target nucleic acid. Specifically, the composition for detecting the target nucleic acid comprising the first probe and the second probe according to the present disclosure provides a change in signal as the target nucleic acid is amplified.

The first probe and the second probe according to the present disclosure are characterized by having different Tm values. Specifically, the Tm value of the hybrid between the first probe and the target nucleic acid is higher than the Tm value of the hybrid between the second probe and the target nucleic acid. For example, the Tm value of the first probe is at least 2°C, 3°C, 4°C, 5°C, 7°C, 8°C, 9°C, 10°C, 11°C, 12°C, 13°C, 14°C, 15°C, or 20°C higher than the Tm value of the second probe.

By using two probes with different Tm values, the composition for detecting the target nucleic acid according to the present disclosure has a temperature range in which the signal changes depending on the presence of the target nucleic acid during a reaction with the target nucleic acid (e.g., an amplification reaction), referred to as the signal-changing temperature range, and two temperature ranges in which the signal is constant even in the presence of the target nucleic acid, referred to as the signal-constant temperature ranges.

Figs. 1 to 3 illustrate the predominant forms of the first probe and the second probe depending on the presence or absence of the target nucleic acid and temperature.

First, FIG. 1 shows the conformations of the first probe and the second probe in (i) a first signal-constant temperature range, (ii) a signal-changing temperature range, and (iii) a second signal-constant temperature range, in the absence of the target nucleic acid or before reaction between the target nucleic acid and the composition for detecting the target nucleic acid comprising two probes having different Tm values according to the present disclosure.

In one embodiment, prior to the reaction of the composition for detecting the target nucleic acid, which comprises two probes with different Tm values according to the present disclosure with the target nucleic acid or when the target nucleic acid is absent, the first probe forms a random coil or hairpin structure across the first signal-constant temperature range, the signal-changing temperature range, and the second signal-constant temperature range, thereby allowing the reporter molecule of the first probe to be positioned in close proximity to the quencher molecule of the first probe. Specifically, in the second signal-constant temperature range, the hairpin structure may also be disrupted, resulting in the first probe existing in a random coil form. As a result, the first quencher molecule quenches a signal from the reporter molecule across the entire temperature range. Meanwhile, when the target nucleic acid is absent, the second probe exists as a single strand across the entire temperature range (see FIG. 1 (i) to FIG. 1 (iii)).

FIG. 2 shows the conformations of the first probe and the second probe in the presence of the target nucleic acid in (i) a first signal-constant temperature range, (ii) a signal-changing temperature range, and (iii) a second signal-constant temperature range, in a specific embodiment where the second probe hybridizes with the target nucleic acid in the 3' direction downstream of the first probe.

FIG. 3 shows the conformations of the first probe and the second probe in the presence of the target nucleic acid in (i) a first signal-constant temperature range, (ii) a signal-changing temperature range, and (iii) a second signal-constant temperature range, in a specific embodiment where the second probe hybridizes with the target nucleic acid in the 5' direction upstream of the first probe.

In one embodiment, when the target nucleic acid is present, both the first probe and the second probe hybridize with the target nucleic acid within the first signal-constant temperature range. In this temperature range, the first probe hybridizes with the first region of the target nucleic acid to cause the reporter molecule and the first quencher molecule of the first probe to be separated, thereby allowing the first quencher molecule to unquench a signal from the reporter molecule. However, the second probe hybridizes with the second region of the target nucleic acid to allow the second quencher molecule of the second probe to be in close proximity to the reporter molecule of the first probe, thereby causing the second quencher molecule to quench a signal from the reporter molecule (see FIG. 2(i) or FIG. 3(i)). That is, as shown in FIG. 1(i) and FIG. 2(i) or FIG. 3(i), the reporter molecule is quenched by either the first quencher molecule or the second quencher molecule within the first signal-constant temperature range, regardless of the presence of the target nucleic acid. As a result, the signal remains constant without any change within the first signal-constant temperature range even in the presence of the target nucleic acid.

In one embodiment, when the target nucleic acid is present, the first probe hybridizes with the first region of the target nucleic acid within the signal-changing temperature range,, but the second probe is not hybridized with the second region of the target nucleic acid. In other words, the second probe is dissociated from the target nucleic acid and exists as a single strand. In this temperature range, both the first quencher molecule of the first probe and the second quencher molecule of the second probe are positioned apart from the reporter molecule of the first probe, thereby unquenching a signal from the reporter molecule (see FIG. 2(ii) or FIG. 3(ii)). That is, as shown in FIG. 1(ii) and FIG. 2(ii) or FIG. 3(ii), within the signal-changing temperature range, the first quencher molecule quenches a signal from the reporter molecule in the absence of the target nucleic acid, whereas both the first quencher molecule and the second quencher molecule unquench a signal from the reporter molecule in the presence of the target nucleic acid. As a result, within the signal-changing temperature range, the signal changes depending on the presence of the target nucleic acid.

In one embodiment, when the target nucleic acid is present, neither the first probe nor the second probe is hybridized with the target nucleic acid within the second signal-constant temperature range. In other words, both the first probe and the second probe are dissociated from the target nucleic acid and exist as single strands. In this temperature range, the first quencher molecule of the first probe is positioned in close proximity to the reporter molecule of the first probe, thereby quenching a signal from the reporter molecule (see FIG. 2(iii) or FIG. 3(iii)). That is, as shown in FIG. 1(iii) and FIG. 2(iii) or FIG. 3(iii), within the second signal-constant temperature range, the reporter molecule is quenched by the first quencher molecule regardless of the presence of the target nucleic acid. As a result, within the second signal-constant temperature range, the signal remains constant without any change even in the presence of the target nucleic acid.

Meanwhile, in the case of an intermediate reaction product (e.g., a product in an early or intermediate cycle) in which the amplification of the target nucleic acid is in progress, the first probe and the second probe that have not yet participated in the reaction may coexist. At this time, depending on the temperature, the two probes exist in the conformations shown in FIG. 1(i) to FIG. 1(iii).

In one embodiment, during the reaction (e.g., a target nucleic acid amplification reaction) between the composition for detecting the target nucleic acid according to the present disclosure and the target nucleic acid, the amounts of the first probe and the second probe remain constant at the concentrations initially included in the composition for detecting the target nucleic acid. The first probe and the second probe included in the composition for detecting the target nucleic acid may exist in the forms shown in FIG. 1 to FIG. 3, depending on the presence of the target nucleic acid and/or temperature changes, as described above. Moreover, the ratio between the amount of the first probe and the second probe in the forms shown in FIG. 1 and the amount of the first probe or second probe in the forms shown in FIG. 2 or FIG. 3 varies depending on the degree of reaction with the target nucleic acid (e.g., the degree of amplification of the target nucleic acid).

FIG. 4(a) presents (i) the total amount ratio (or abundance ratio, %) of the first probe and/or the second probe before reacting with the target nucleic acid and (ii) the total amount ratio (or abundance ratio, %) of the first probe and/or the second probe that has reacted with the target nucleic acid, in the early, intermediate, and late cycles of the target nucleic acid amplification reaction using a composition for detecting a target nucleic acid, which comprises two probes having different Tm values, according to the present disclosure. The figure also illustrates the melt curves in the early, intermediate, and late cycles. Specifically, the numbers in row (i) of FIG. 4(a) indicate the total amount ratio (or abundance ratio, %) of the first probe and the second probe present in the forms (i) to (iii) of FIG. 1, while the numbers in row (ii) of FIG. 4(a) indicate the total amount ratio (or abundance ratio, %) of the first probe and the second probe present in the forms (i) to (iii) of FIG. 2 or in the forms (i) to (iii) of FIG. 3.

The term "amount ratio" or "abundance ratio," as used herein when referring to the first probe and/or the second probe that has reacted with the target nucleic acid, refers to the amount ratio (or abundance ratio, %) of hybrids that can be formed by hybridization of the two probes at a specific temperature (or temperature range). For example, the amount ratio (or abundance ratio, %) of the first probe that has reacted with the target nucleic acid refers to the total amount ratio (or abundance ratio, %) of the first probe present in the forms (i) to (iii) of FIG. 2.

The amount ratio in rows (i) and (ii) of FIG. 4(a) changes as the cycle number increases, that is, as the target nucleic acid is amplified. Specifically, when the melt curves in the early, intermediate, and late cycles are merged, a graph as shown in FIG. 4(b) is obtained. This graph demonstrates that the composition for detecting a target nucleic acid according to the present disclosure has two temperature ranges in which the signal remains constant even in the presence of the target nucleic acid, as well as a temperature range in which the signal changes as the target nucleic acid is amplified.

In a specific embodiment, the amount of the first probe and the second probe that have reacted with the target nucleic acid increases as the target nucleic acid is amplified. In contrast, as the target nucleic acid is amplified, the amount of the first probe and the second probe that have not reacted with the target nucleic acid decreases. That is, the amount ratio changes, and as the target nucleic acid is amplified, the signal indicating the presence of the target nucleic acid also changes. Due to this change in the amount ratio, the signal indicating the presence of the target nucleic acid within the signal-changing temperature range also changes. Accordingly, the composition for detecting a target nucleic acid according to the present disclosure can provide a signal dependent on the presence of the target nucleic acid. Meanwhile, even though the total amount ratio between the first probe and/or the second probe that have reacted with the target nucleic acid and the first probe and/or the second probe that have not reacted with the target nucleic acid changes as the target nucleic acid is amplified, the signal remains constant within the two signal-constant temperature ranges.

As described above, the composition for detecting a target nucleic acid according to the present disclosure has a signal-changing temperature range in which the signal changes depending on the presence of the target nucleic acid, and two signal-constant temperature ranges in which the signal remains constant even in the presence of the target nucleic acid. Specifically, the composition comprising the first probe and the second probe having different Tm values is an InterSC composition having a characteristic that the signal-changing temperature range is higher than the first signal-constant temperature range among the two signal-constant temperature ranges, and lower than the second signal-constant temperature range among the two signal-constant temperature ranges.

In one embodiment, the signal-changing temperature range is determined depending on the Tm values of the first probe and the second probe. Accordingly, the signal-changing temperature range and the two signal-constant temperature ranges can be controlled by adjusting the Tm values of the first probe and the second probe.

As used herein, the term "signal-constant temperature range (SCoTR)" refers to a temperature range in which a composition for detecting a target nucleic acid provides a constant signal despite the presence of the target nucleic acid. The signal-constant temperature range is a temperature range in which the composition provides a constant signal over reaction time, which may also be referred to herein as a temperature range in which the composition does not provide a significant signal, or a temperature range in which the composition does not provide a signal indicative of the presence of a target nucleic acid.

As used herein, the term "signal-changing temperature range (SChTR)" refers to a temperature range in which a composition provides a signal that changes dependent on the presence of a target nucleic acid. The signal-changing temperature range is a temperature range in which the composition provides a signal that changes over reaction time, which may also be referred to herein as a temperature range in which the composition provides a significant signal, or a temperature range in which the composition provides a signal indicative of the presence of the target nucleic acid.

The term "constant signal" as used herein refers to no substantial change in signal during a reaction between a target nucleic acid and a composition (e.g., during a target nucleic acid amplification reaction). That is, the term refers to all or any signal pattern other than significant signal changes brought about by the amplification of the target nucleic acid present. In particular, the constant signal means no signal change. For example, if the signal during an amplification reaction does not exceed the background signal intensity or the intensity of a signal in the absence of the target nucleic acid, it may be expressed as "the signal is constant". In the present disclosure, the constant signal may be used interchangeably with the signal that does not change, or the signal that does not show change.

In the present disclosure, a "signal change" means a significant signal change that is, a significant change in signal where the signal change indicates the presence of the target nucleic acid. For example, a significant signal change, that is, a signal change that indicates the presence of the target nucleic acid may refer to the generation or extinguishment of a signal that has a distinct intensity compared to the background signal intensity or the intensity of a signal in the absence of the target nucleic acid, or may refer to a substantial increase, or decrease of the intensity of a signal indicating the presence of the target nucleic acid, as the target nucleic acid and/or signal is amplified during an incubation reaction in step (a).

In one embodiment, the signal change includes "signal generation or extinguishment" and "signal increase or decrease" from a label.

In one embodiment, the signal change may be generated depending on the presence of the target nucleic acid. For example, the signal change may be generated as a signal indicating the presence of the target nucleic acid is generated or extinguished.

In one embodiment, the signal change may be generated upon amplification of the target nucleic acid. That is, the signal change may be generated as the amount of the target nucleic acid increases upon amplification of the target nucleic acid. For example, upon amplification of the target nucleic acid, a signal indicating the presence of the target nucleic acid may increase or decrease, thereby inducing the signal change.

In one embodiment, the signal change may be generated upon the amplification of a signal dependent on the target nucleic acid. That is, upon amplification of the signal dependent on the target nucleic acid, the intensity of the signal changes, thereby changing the signal. For example, as the signal dependent on the presence of the target nucleic acid is amplified, the signal indicating the presence of the target nucleic acid may increase, or decrease, thereby inducing the signal change.

In the present disclosure, "signal change" and/or "constant signal" is based on signals detected at the same temperature during a nucleic acid amplification reaction using the same composition. For example, "signal change" and/or "constant signal" are designated based on difference between signal values detected at the same temperature using *n* compositions, and more specifically, "signal change" and/or "constant signal" are designated based on (i) a difference between signal values detected at the same temperature in a plurality of cycles, or (ii) a difference between "a reference signal value" described below and a signal value detected at the same temperature as the temperature for which the reference signal value is set. That is, "signal change" and/or "constant signal" is not intended to be designated based on a difference between signal values detected at different temperatures.

In one embodiment, the expression "one temperature range is lower than the other temperature range" used with regard to the signal-changing temperature range and signal-constant temperature range of the composition for detecting a target nucleic acid means that the highest temperature of one temperature range is lower than the lowest temperature of the other temperature range. Further, the expression "one temperature range is higher than the other temperature range" means that the lowest temperature of one temperature range is higher than the highest temperature of the other temperature range. For example, that a signal-constant temperature range is higher than a signal-changing temperature range means that the lowest temperature within the signal-constant temperature range is higher than the highest temperature within the signal-changing temperature range.

In one embodiment, the composition for detecting the target nucleic acid may further comprise primers capable of amplifying a target nucleic acid sequence comprising the first region and the second region of the target nucleic acid.

In one embodiment, the composition for detecting the target nucleic acid may further comprise a polymerase for amplifying the target nucleic acid.

In one embodiment, the polymerase is a polymerase lacking nuclease activity (e.g., 5'→3' exonuclease activity).

In another embodiment, the polymerase is a polymerase having nuclease activity (e.g., 5'→3' exonuclease activity). In this case, the first probe and/or the second probe are resistant to the nuclease activity.

The composition for detecting a target nucleic acid according to the present disclosure is characterized by having a signal-changing temperature range and two signal-constant temperature ranges. Due to this characteristic, by adjusting the signal-changing temperature range, not only can a single target nucleic acid be detected, but multiple target nucleic acids can also be detected using a single type of label. However, when a polymerase having nuclease activity is used, the first probe and/or the second probe may be cleaved by the nuclease activity. Such cleavage of the probe induces the release of the reporter molecule, the first quencher molecule, and/or the second quencher molecule linked to the probe, causing the reporter molecule to be unquenched from the first quencher molecule and/or the second quencher molecule across the entire temperature range. As a result, the composition for detecting a target nucleic acid according to the present disclosure exhibits signal changes dependent on the presence of the target nucleic acid across the entire temperature range without signal-constant temperature ranges. Therefore, when the composition for detecting a target nucleic acid comprises a polymerase having nuclease activity, such as 5'-3' exonuclease activity, it is preferable that the first probe and/or the second probe be resistant to 5'→3' nuclease activity.

In one embodiment, resistance to 5'-3' exonuclease activity is conferred by nucleotides with a backbone resistant to 5'-3' exonuclease activity and includes various phosphorothioate linkages, phosphonate linkages, phosphoramidate linkages, and 2'-carbohydrate modifications. More preferably, it includes phosphorothioate linkages, alkyl phosphotriester linkages, aryl phosphotriester linkages, alkyl phosphonate linkages, aryl phosphonate linkages, hydrogen phosphonate linkages, alkyl phosphoramidate linkages, aryl phosphoramidate linkages, phosphoroselenoate linkages, 2'-O-aminopropyl modifications, 2'-O-alkyl modifications, 2'-O-allyl modifications, 2'-O-butyl modifications, α-anomeric oligodeoxynucleotides, and 1-(4'-thio-β-D-ribofuranosyl) modifications.

As described above, the method according to the present disclosure utilizes *n* different compositions for detecting *n* target nucleic acids, each corresponding to a different target nucleic acid. In particular, the method involves using at least one of the *n* compositions for detecting target nucleic acids as an InterSC composition, which comprises two probes having different Tm values according to the present disclosure. For example, when *n is* 2, the first target nucleic acid and the second target nucleic acid are detected using a composition for detecting a first target nucleic acid and a composition for detecting a second target nucleic acid, wherein at least one of these two compositions comprises two probes having different Tm values. When *n* is 3, the first target nucleic acid, the second target nucleic acid, and the third target nucleic acid are detected using a composition for detecting a first target nucleic acid, a composition for detecting a second target nucleic acid, and a composition for detecting a third target nucleic acid, wherein at least one of these three compositions comprises two probes having different Tm values.

Specifically, when *n* is 2, the signal generation mechanisms of the composition for detecting the first target nucleic acid and the composition for detecting the second target nucleic acid may be combined as shown in Table 1 below. When *n* is 3, the composition for detecting the first target nucleic acid to the composition for detecting the third target nucleic acid may be combined as shown in Table 2. In Tables 1 and 2, "UnderSC," "InterSC," and "OverSC" refer to UnderSC, InterSC, and OverSC compositions, respectively, to which various UnderSC, InterSC, and OverSC signal generation mechanisms known in the art are applied. In particular, "InterSC" refers to an InterSC composition in which a signal generation mechanism different from the method utilizing two probes having different Tm values according to the present disclosure is applied.

**[Table 1]**

| when *n* is 2 | | |
|---|---|---|
| N=2 | The first target | The second target |
| 1 | UnderSC | a composition according to the present disclosure |
| 2 | a composition according to the present disclosure | a composition according to the present disclosure |
| 3 | a composition according to the present disclosure | InterSC |
| 4 | InterSC | a composition according to the present disclosure |
| 5 | a composition according to the present disclosure | OverSC |

**[Table 2]**

| when *n* is 3 | | | |
|---|---|---|---|
| N=3 | The first target | The second target | The third target |
| 1 | a composition according to the present disclosure | InterSC | InterSC |
| 2 | a composition according to the present disclosure | InterSC | OverSC |
| 3 | UnderSC | a composition according to the present disclosure | InterSC |
| 4 | UnderSC | a composition according to the present disclosure | OverSC |
| 5 | InterSC | a composition according to the present disclosure | InterSC |
| 6 | InterSC | a composition according to the present disclosure | OverSC |
| 7 | UnderSC | InterSC | a composition according to the present disclosure |
| 8 | InterSC | InterSC | a composition according to the present disclosure |
| 9 | a composition according to the present disclosure | a composition according to the present disclosure | InterSC |
| 10 | a composition according to the present disclosure | a composition according to the present disclosure | OverSC |
| 11 | a composition according to the present disclosure | InterSC | a composition according to the present disclosure |
| 12 | UnderSC | a composition according to the present disclosure | a composition according to the present disclosure |
| 13 | InterSC | a composition according to the present disclosure | a composition according to the present disclosure |
| 14 | a composition according to the present disclosure | a composition according to the present disclosure | a composition according to the present disclosure |

Details of the UnderSC, InterSC, and OverSC compositions are found in International Patent Application Publication WO 2022-265463, which is incorporated herein by reference in its entirety. As examples of representative signal generation mechanisms known in the art that can be adopted by UnderSC compositions, InterSC compositions, and InterSC compositions, FIG. 5 illustrates the molecular beacon method (Tyagi et al., Nature Biotechnology v.14, March 1996), FIG. 6 illustrates the PTOCE-based method (WO 2012/096523), and FIG. 7 illustrates the dual-quenching method (WO 2016/101959), showing the signal-changing temperature range and the signal-constant temperature range(s) for each method. Specifically, in each of Figs. 5 to 7: (a) represents the conformations of the oligonucleotides included in the composition for detecting a target nucleic acid, depending on the presence or absence of the target nucleic acid, where (i) shows the conformation of the oligonucleotide either in the absence of the target nucleic acid or before reacting with the target nucleic acid, and (ii) shows the conformation of the oligonucleotide after reacting with the target nucleic acid. (b) represents the melt curves in the early, intermediate, and late cycles of target nucleic acid amplification. (c) presents a single graph combining the melt curves of each of the early, intermediate, and late cycles. The PTOCE-based method generally involves the formation of an extended strand dependent on the presence of the target nucleic acid. The PTOCE-based methods commonly involve the formation of the extended strand depending on the presence of a target nucleic acid sequence. The term "PTOCE-based method" is used herein to encompass various methods for providing signals comprising the formation of an extended strand through cleavage and extension of PTO.

A specific embodiment of signal generation by the PTOCE-based methods comprises the steps of:
(a) hybridizing the target nucleic acid with the upstream oligonucleotide and the PTO; (b) contacting the resultant of the step (a) to an enzyme having 5' nuclease activity under conditions for cleavage of the PTO; wherein the upstream oligonucleotide or its extended strand induces cleavage of the PTO by the enzyme having 5' nuclease activity such that the cleavage releases a fragment comprising the 5'-tagging portion or a part of the 5'-tagging portion of the PTO; (c) hybridizing the fragment released from the PTO with the CTO; wherein the fragment released from the PTO is hybridized with the capturing portion of the CTO; (d) performing an extension reaction using the resultant of the step (c) and a template-dependent nucleic acid polymerase; wherein the fragment hybridized with the capturing portion of the CTO is extended to form an extended strand; and (e) detecting the formation of the extended strand by detecting signal provided dependent on the presence of the extended strand. In the step (a), a primer set for amplification of the target nucleic acid may be used instead of the upstream oligonucleotide. In this case, the method further comprises repeating all or some of the steps (a)-(e) with denaturation between repeating cycles.

A specific embodiment of signal generation by the dual quenching assay comprises the following steps:
(a) hybridizing a target nucleic acid sequence with a PTO; the PTO comprising (i) a targeting portion comprising a nucleotide sequence substantially complementary to the target nucleic acid sequence, (ii) a Melting Temperature Deciding Region (MTDR), comprising a nucleotide sequence non-complementary to the target nucleic acid sequence, and (iii) at least one set of interactive labels comprising at least one fluorophore and at least one quencher; b) hybridizing the PTO with a CQO; wherein the CQO comprises (i) a capturing portion comprising a nucleotide sequence reverse complementary to the MTDR of the PTO and (ii) at least one quenching molecule, wherein the MTDR is configured to hybridize with the capturing portion of the CQO to form a Tag Duplex; (c) contacting the Tag Duplex with an enzyme having nuclease activity; wherein the enzyme having nuclease activity induces cleavage of the Tag Duplex when the Tag Duplex is hybridized with the target nucleic acid sequence, thereby releasing an activated Tag Duplex fragment comprising a PTO fragment comprising the MTDR hybridized to the capturing portion of the CQO and the at least one fluorophore; (d) melting and/or hybridizing said activated Tag Duplex fragment to obtain a signal from the at least one fluorophore; and e) detecting the activated Tag Duplex fragment by measuring the signal from the at least one fluorophore, wherein the signal is indicative of the presence of the target nucleic acid sequence.

In one embodiment, each of the *n* compositions for detecting the target nucleic acids provides a signal change at a corresponding detection temperature among the *n* detection temperatures, the signal change indicating the presence of a corresponding target nucleic acid.

For example, the composition for detecting an *i*^{th} target nucleic acid among the *n* target nucleic acids provides a signal change at an *i*^{th} detection temperature among the *n* detection temperatures and provides a constant signal at the other detection temperatures in the presence of the *i*^{th} target nucleic acid.

In one embodiment, *i* represents an integer from 1 to *n,* and the *i*^{th} detection temperature is lower than the (*i*+1)^{th} detection temperature. In one embodiment, when *i* is *n,* there is no *i*+1 detection temperature (*i.e., n*+1 detection temperature). For example, when *n is* 3, *i* represents an integer from 1 to 3, and there are a first detection temperature, a second detection temperature, and a third detection temperature, wherein the first detection temperature is lower than the second detection temperature, and the second detection temperature is lower than the third detection temperature.

According to the present disclosure, within the temperature range covering all of the *n* detection temperatures, the composition for detecting the *i*^{th} target nucleic acid has a signal-changing temperature range in which the signal changes depending on the presence of the *i*^{th} target nucleic acid, and one or two signal-constant temperature ranges in which the signal is constant even in the presence of the *i*^{th} target nucleic acid.

In one embodiment, the composition for detecting the *i*^{th} target nucleic acid is any one of: (i) an Under-Signal-Change (UnderSC) composition having a characteristic that the signal-changing temperature range is lower than the signal-constant temperature range, (ii) an Inter-Signal-Change (InterSC) composition having a characteristic that the signal-changing temperature range is higher than one of two signal-constant temperature ranges, and lower than the other of the two signal-constant temperature ranges, and (iii) an Over-Signal-Change (OverSC) composition having a characteristic that the signal-changing temperature range is higher than the signal-constant temperature range.

In one embodiment, the composition for detecting the *i*^{th} target nucleic acid, in the presence of the *i*^{th} target nucleic acid, provides a signal change upon amplification of the target nucleic acid at the *i*^{th} detection temperature (*i.e.,* change in an *i*^{th} signal) while providing no signal change at the other detection temperatures, even as the target nucleic acid is amplified (*i.e.,* the signal is constant). That is, the composition for detecting the *i*^{th} target nucleic acid have a signal-changing temperature range in which the signal changes as the *i*^{th} target nucleic acid is amplified, and signal-constant temperature range(s) in which the signal is constant even as the *i*^{th} target nucleic acid is amplified.

The term "*i*^{th} signal" as used herein refers to a signal provided at an *i*^{th} detection temperature by the composition for detecting an *i*^{th} target nucleic acid, which is interchangeably used with "signal at an *i*^{th} detection temperature". In one embodiment, when detecting *n* target nucleic acids, the *i*^{th} signal may mean a signal provided by the *n* compositions for detecting target nucleic acids including the composition for detecting the *i*^{th} target nucleic acid, at the *i*^{th} detection temperature.

In one embodiment, the composition for detecting the *i*^{th} target nucleic acid, in the absence of the *i*^{th} target nucleic acid, provides no signal change, *i.e.,* provides a constant signal at the *i*^{th} detection temperature during an incubation reaction (e.g., a target nucleic acid amplification reaction).

In one embodiment, the signal-changing temperature range is a temperature range in which a difference between signal values (e.g., signal intensities) in the presence of the target nucleic acid and in the absence of the target nucleic acid is generated.

In one embodiment, the signal-changing temperature range is a temperature range in which the signal value changes depending on the level of amplification of the target nucleic acid (e.g., the amount of the amplified target nucleic acid).

In one embodiment, the signal-constant temperature range is a temperature range in which the signal value does not change regardless of the presence of the target nucleic acid. In other words, the signal-constant temperature range is a temperature range in which there is no difference between the signal value in the presence of the target nucleic acid and the signal value in the absence of the target nucleic acid.

In one embodiment, the *i*^{th} detection temperature may be selected from within the signal-changing temperature range of the composition for detecting the *i*^{th} target nucleic acid. In the present disclosure, the composition for detecting the *i*^{th} target nucleic acid is referred to as having the *i*^{th} detection temperature. In addition, the *i*^{th} target nucleic acid corresponding to the composition for detecting the *i*^{th} target nucleic acid may be referred to as a target nucleic acid having the *i*^{th} detection temperature.

According to an embodiment, one detection temperature, which is determined by the composition for detecting a corresponding target nucleic acid, is assigned to one target nucleic acid.

In certain embodiments, when *n* is 2, the composition for detecting the first target nucleic acid provides a signal change at the first detection temperature and provides a constant signal at the other detection temperature, that is, the second detection temperature in the presence of the first target nucleic acid; and the composition for detecting the second target nucleic acid provides a signal change at the second detection temperature and provides a constant signal at the first detection temperature in the presence of the second target nucleic acid.

In certain embodiments, when *n* is 3, the composition for detecting the first target nucleic acid provides a signal change at the first detection temperature and provides a constant signal at the second detection temperature and the third detection temperature in the presence of the first target nucleic acid; the composition for detecting the second target nucleic acid provides a signal change at the second detection temperature and provides a constant signal at the first detection temperature and the third detection temperature in the presence of the second target nucleic acid; and the composition for detecting the third target nucleic acid provides a signal change at the third detection temperature and provides a constant signal at the first detection temperature and the second detection temperature in the presence of the third target nucleic acid.

In one embodiment, the *i*^{th} detection temperature is selected from within the signal-changing temperature range of the composition for detecting the *i*^{th} target nucleic acid, and the *i*^{th} detection temperature is not included in the signal-changing temperature ranges of the compositions for detecting the other target nucleic acids.

In one embodiment, the signal-changing temperature range of any one of the compositions for detecting target nucleic acids may overlap with the signal-changing temperature range of a composition for detecting a target nucleic acid having an adjacent detection temperature, while not overlapping with the signal-changing temperature range of a composition for detecting a target nucleic acid having a detection temperature not adjacent thereto. In this case, the detection temperature of the composition for detecting a target nucleic acid that has the signal-changing temperature range overlapping with the signal-changing temperature range of the composition for detecting another target nucleic acid is selected within the signal-changing temperature range that does not overlap with the signal-changing temperature range of the composition for detecting another target nucleic acid. By selecting the detection temperature as such, only the signal indicative of the presence of a single particular target nucleic acid can be provided at a single detection temperature (see FIG. 8).

In one embodiment, the signal-changing temperature range of any one of compositions for detecting target nucleic acids may overlap with the signal-changing temperature range of the composition for detecting a target nucleic acid having an adjacent detection temperature, but either of the two signal-changing temperature ranges is not completely included in the other signal-changing temperature range.

The term "adjacent detection temperature" is used herein to refer to consecutive detection temperatures among *n* detection temperatures, and for example, the adjacent detection temperature of the *i*^{th} detection temperature is the (*i*-1)^{th} detection temperature or the (*i+*1)^{th} detection temperature.

In one embodiment, the signal-changing temperature range of the composition for detecting the *i*^{th} target nucleic acid may partially overlap with the signal-changing temperature range of the composition for detecting a target nucleic acid having an adjacent detection temperature, while not overlapping with the signal-changing temperature range of the composition for detecting a target nucleic acid having a detection temperature not adjacent thereto.

In one embodiment, the composition for detecting the *i*^{th} target nucleic acid comprises a label that provides a signal dependent on the presence of the *i*^{th} target nucleic acid.

In one embodiment, the label herein may be linked to an oligonucleotide or may exist in free form. Alternatively, the label may be incorporated into the oligonucleotide during the incubating (e.g., a nucleic acid amplification). In other words, the composition for detecting the target nucleic acid may initially include a labeled oligonucleotide or may provide a labeled oligonucleotide as the label is incorporated into a newly generated oligonucleotide (e.g., an extended strand) during an incubation reaction.

In one embodiment, the composition for detecting the *i*^{th} target nucleic acid includes an incorporating label that is incorporated into an oligonucleotide during the incubating and provides a signal depending on the presence of the *i*^{th} target nucleic acid.

In one embodiment, the composition for detecting the *i*^{th} target nucleic acid provides a labeled oligonucleotide that serves to provide a signal depending on the presence of the *i*^{th} target nucleic acid.

In one embodiment, the composition for detecting the *i*^{th} target nucleic acid initially includes a labeled oligonucleotide that serves to provide a signal depending on the presence of the *i*^{th} target nucleic acid. The first probe and the second probe having different Tm values as described herein corresponds to an example of the labeled oligonucleotides.

Alternatively, the composition for detecting the *i*^{th} target nucleic acid may include an oligonucleotide and a label that provides a signal depending on the presence of the *i*^{th} target nucleic acid, and the label is incorporated into the oligonucleotide during an incubation reaction (e.g., a nucleic acid amplification reaction), thereby providing a labeled oligonucleotide that serves to provide a signal depending on the presence of the *i*^{th} target nucleic acid.

As used herein, the term "labeled oligonucleotide" refers to an oligonucleotide involved in the generation of a signal being detected.

In one embodiment, the labeled oligonucleotide may comprise an oligonucleotide that specifically hybridizes to a target nucleic acid (e.g., a probe or a primer); when the probe or primer hybridized with the target nucleic acid is cleaved to release a fragment, the labeled oligonucleotide may comprise a capture oligonucleotide that specifically hybridizes to the fragment; when the fragment hybridized to the capture oligonucleotide is extended to form an extended strand, the labeled oligonucleotide may comprise an oligonucleotide that specifically hybridizes to the extended strand, an oligonucleotide that is produced by incorporating a label during the fragment extension, an oligonucleotide that specifically hybridizes with the capture oligonucleotide, and a combination thereof.

In one embodiment, the labeled oligonucleotide includes an oligonucleotide involved in actual signal generation. For example, hybridization or non-hybridization between the labeled oligonucleotide and another oligonucleotide (e.g., an oligonucleotide comprising a nucleotide sequence complementary to the labeled oligonucleotide or the target nucleic acid) determines signal generation.

In one embodiment, the labeled oligonucleotide may be a 'probe' known in the art. According to an embodiment, the 3'-end of the probe is "blocked" to prohibit its extension. The blocking may be achieved in accordance with conventional methods. For instance, the blocking may be performed by adding to the 3'-hydroxyl group of the last nucleotide a chemical moiety such as biotin, labels, phosphate groups, alkyl groups, non-nucleotide linkers, phosphorothioate or alkane-diol residues. Alternatively, the blocking may be carried out by removing the 3'-hydroxyl group of the last nucleotide or using a nucleotide with no 3'-hydroxyl group such as dideoxynucleotide.

In one embodiment, the labeled oligonucleotide may be composed of at least one oligonucleotide. According to an embodiment, when the labeled oligonucleotide is composed of a plurality of oligonucleotides, the labeled oligonucleotide may be labeled in various fashions. For example, all or portion of the plurality of oligonucleotides may have at least one label.

In one embodiment, the label may be a single label or interactive labels.

For example, the single label includes a fluorescent label, a luminescent label, a chemiluminescent label, an electrochemical label, and a metal label. In one embodiment, the single label provides different signals (for example, different signal intensities) depending on its presence on a double strand or a single strand. In one embodiment, the single label is a fluorescent label. Preferred types and binding sites of single fluorescent labels used in the present disclosure are disclosed in U.S. Pat. Nos. 7,537,886 and 7,348,141, the teachings of which are incorporated herein by reference in their entirety. For example, the single fluorescent label includes, without limitation, JOE, FAM, TAMRA, ROX, and fluorescein-based label. The single label may be linked to an oligonucleotide by various methods. For example, the label may be linked to a probe via a spacer containing carbon atoms (e.g., a 3-carbon spacer, a 6-carbon spacer, or a 12-carbon spacer).

In one embodiment, the interactive labels may include at least one reporter molecule and at least one quencher molecule. In particular, the interactive labels may include one reporter molecule and one quencher molecule. Or, the interactive labels may include one reporter molecule and two quencher molecules.

The detailed description and examples of interactive labels can be found in the section describing interactive labels applicable to the composition for detecting a target nucleic acid, which comprises the first probe and the second probe having different Tm values.

In one embodiment, when the label is a single label, the single label may be linked to a single oligonucleotide.

In one embodiment, when the label is interactive labels, the interactive labels may include at least one reporter molecule and at least one quencher molecule, wherein the interactive labels may be all linked to one oligonucleotide or may be linked to each of a plurality of oligonucleotides.

In one embodiment, an incorporating label may be used in the process of incorporating a label during a primer extension to generate a signal (e.g., Plexor technology, Sherrill C B et al., Journal of the American Chemical Society, 126:4550-45569 (2004)). In addition, the incorporating label may be used in a signal generation by a duplex formed in a manner dependent on cleavage of a mediation oligonucleotide hybridized with a target nucleic acid.

In one embodiment, the incorporating label may be generally linked to a nucleotide. In addition, a nucleotide having a non-natural base may be used.

As used herein, the term "non-natural base" refers to derivatives of natural bases such as adenine (A), guanine (G), thymine (T), cytosine (C) and uracil (U), which are capable of forming hydrogen-bonding base pairs. The term "non-natural base" as used herein includes bases having base pairing patterns different from natural bases as mother compounds, as described, for example, in U.S. Pat. Nos. 5,432,272, 5,965,364, 6,001,983, and 6,037,120. The base pairing between non-natural bases includes two or three hydrogen bonds as with natural bases. The base pairing between non-natural bases is also formed in a specific manner. Specific examples of non-natural bases include the following bases in base pair combinations: iso-C/iso-G, iso-dC/iso-dG, Z/P, V/J, K/X, H/J, Pa/Ds, Pa/Q, Pn/Ds, Pn/Dss, Px/Ds, NaM/5SICS, 5FM/5SICS, and M/N (see U.S. Pat. Nos. 5,432,272; 5,965,364; 6,001,983; 6,037,120; 6,140,496; 6,627,456; 6,617,106; and 7,422,850; and Filip Wojciechowski et al., Chem. Soc. Rev., 2011, 40, 5669-5679).

Conventional methods for detecting multiple target nucleic acids have the disadvantage of requiring the use of different types of fluorescent labels for different target nucleic acids or, even when using a single type of fluorescent label, requiring additional analyses such as melting curve analysis. In contrast, the method according to the present disclosure enables real-time detection of multiple target nucleic acids without the need for additional analyses such as melting curve analysis, by using a composition for detecting target nucleic acids that provides a duplex, while utilizing a single type of label (e.g., a single fluorescent label).

In one embodiment, each of the *n* compositions for detecting target nucleic acids provides one or more duplexes.

As used herein, the term "duplex" refers to a double-stranded nucleic acid molecule formed by hybridizing two single-stranded nucleic acid molecules having a sequence partially or totally complementary to each other under hybridization conditions. The two single-stranded nucleic acid molecules forming the duplex may exist in associated form (i.e., a double-stranded molecule) or dissociated form (i.e., two single-stranded molecules) depending on the temperature (in particular, the detection temperature). In this respect, the term "duplex" when referring the expression "a composition for detecting a target nucleic acid provides a duplex" is used to encompass both a duplex in associated form and a duplex in dissociated form.

In one embodiment, the duplex may be also referred to as "hybrid".

The term "association" or "dissociation" has the same meaning as the term "hybridization" or "denaturation", respectively.

As mentioned above, the expression "a composition for detecting a target nucleic acid provides a duplex" as used herein may mean that the composition provides a duplex in associated form and/or a duplex in dissociated form. Likewise, the expression "a composition for detecting a target nucleic acid generates a duplex during incubating" as used herein may mean that the composition generates a duplex in associated form and/or a duplex in dissociated form during an incubation reaction.

In one embodiment, at least one of the duplexes provided by the composition for detecting a target nucleic acid is a duplex providing a signal. In particular, the duplex is a duplex providing a signal change. In other words, the composition for detecting an *i*^{th} target nucleic acid provides a duplex providing a signal, and particularly, the composition for detecting the *i*^{th} target nucleic acid provides a duplex providing a signal change depending on the presence of the *i*^{th} target nucleic acid.

The term "duplex providing a signal" as used herein refers to a duplex capable of providing a signal that can be distinguished depending on whether the duplex is in associated form or dissociated form. For example, this means that the duplex in associated form generates (or extinguishes) a signal, and the duplex in dissociated form extinguishes (or generates) a signal.

In one embodiment, the duplex providing the signal may include at least one label.

As used herein, the term "duplex providing a signal change" refers to a duplex providing a signal change indicative of the presence of a target nucleic acid as the amount of the duplex providing the signal change changes depending on the presence of the target nucleic acid. In particular, a hybrid formed by the first probe and/or the second probe hybridized with the target nucleic acid in the composition for detecting the target nucleic acid according to the present disclosure corresponds to an example of a duplex providing a signal change described herein.

In one embodiment, the duplex providing the signal change includes a label. In particular, at least one label is linked to at least one of the two single-stranded nucleic acid molecules constituting the duplex. For example, the duplex providing the signal change includes a single label, and in this case, the single label is linked to any one of the two single-stranded nucleic acid molecules constituting the duplex. As another example, the duplex providing the signal change includes interactive labels, and in this case, the interactive labels are all linked to one of the two single-stranded nucleic acid molecules constituting the duplex providing the signal change, or one of the interactive labels is linked to one of the two single-stranded nucleic acid molecules and the other of the interactive label is linked to the other of the two single-stranded nucleic acid molecules.

In one embodiment, the composition for detecting the *i*^{th} target nucleic acid provides a duplex providing a signal change.

In one embodiment, the composition for detecting the *i*^{th} target nucleic acid provides a signal from the label when the duplex providing the signal change is in associated form. In other words, the composition for detecting the *i*^{th} target nucleic acid provides a signal depending on association of the two single-stranded nucleic acid molecules constituting the duplex.

In one embodiment, the composition for detecting the *i*^{th} target nucleic acid provides a signal from the label when the duplex providing the signal change is in dissociated form. In other words, the composition for detecting the *i*^{th} target nucleic acid provides a signal depending on dissociation of the two single-stranded nucleic acid molecules constituting the duplex.

In one embodiment, the association or dissociation of the duplex may depend on temperature.

In one embodiment, the duplex providing the signal change may be a duplex that has initially (originally) been included in the composition for detecting a target nucleic acid.

In one embodiment, when the duplex providing the signal change has been included in the composition for detecting the target nucleic acid, the duplex may be generated by hybridization between a labeled oligonucleotide and an oligonucleotide hybridizable with the labeled oligonucleotide. A double-stranded nucleic acid hybridization probe (U.S. Pat. No. 7,799,522), also known as Yin-Yang probe, is an exemplary duplex providing a signal change, which has initially been included in the composition for detecting the target nucleic acid.

In one embodiment, when the duplex providing the signal change has initially been included in the composition for detecting the target nucleic acid, the amount of the duplex providing the signal change varies, in particular, decreases, in a manner dependent on the presence of the target nucleic acid, thereby providing the signal change. For example, a double-stranded nucleic acid hybridization probe (i.e., the duplex providing the signal change) initially included in the composition for detecting the target nucleic acid forms a new duplex as one of the two single strands constituting the Yin-Yang probe hybridizes with the amplified target nucleic acid as the target nucleic acid is amplified. As a result, the amount of the double-stranded nucleic acid hybridization probe decreases, thereby providing a signal change depending on the presence of the target nucleic acid.

In one embodiment, the duplex providing the signal change may be a duplex newly provided by the composition for detecting a target nucleic acid during an incubation reaction.

In one embodiment, the duplex providing the signal change, which is generated during the incubation reaction, may be provided by hybridization between a labeled oligonucleotide and the target nucleic acid.

Signals by formation of a duplex between the labeled oligonucleotide and the target nucleic acid may be generated by various methods, including Scorpion method (Whitcombe et al., Nature Biotechnology 17:804-807 (1999)), Sunrise (or Amplifluor) method (Nazarenko et al., Nucleic Acids Research, 25(12):2516-2521 (1997), and U.S. Pat. No. 6,117,635), LUX method (U.S. Pat. No. 7,537,886), Plexor method (Sherrill CB, et al., Journal of the American Chemical Society, 126:4550-4556 (2004)), Molecular beacon method (Tyagi et al., Nature Biotechnology v.14 MARCH 1996), Hybeacon method (French DJ et al., Mol. Cell Probes, 15(6):363-374 (2001)), adjacent hybridization probe method (Bernard P.S. et al., Anal. Biochem., 273:221 (1999)), and LNA method (U.S. Pat. No. 6,977,295).

In one embodiment, the duplex providing the signal change generated during the incubating reaction may be a duplex formed by a cleavage reaction dependent on the presence of the target nucleic acid. In the aforementioned PTOCE-based method, an extended duplex comprising a label, which is generated dependent on the presence of the target nucleic acid, corresponds to an example of a duplex formed by a cleavage reaction dependent on the presence of the target nucleic acid (see Fig. 6(a)(ii)). For this reaction, a 5'-nuclease and a 3'-nuclease, particularly a nucleic acid polymerase with 5'-nuclease activity, a nucleic acid polymerase with 3'-nuclease activity, or FEN nuclease may be used.

In one embodiment, the signal change is generated by a duplex generated in a manner dependent on the cleavage of a mediation oligonucleotide specifically hybridized with the target nucleic acid.

As used herein, the term "mediation oligonucleotide" refers to an oligonucleotide mediating the generation of a duplex, not including a target nucleic acid.

In one embodiment, the cleavage of the mediation oligonucleotide alone does not generate a signal, but after hybridization and cleavage of the mediation oligonucleotide, a fragment (a cleavage product) produced by the cleavage is involved in a series of reactions for signal generation.

In one embodiment, the hybridization or cleavage of the mediation oligonucleotide alone does not generate a signal.

In one embodiment, the mediation oligonucleotide includes an oligonucleotide that hybridizes with a target nucleic acid and is cleaved to release a fragment, thereby mediating the generation of a duplex.

In one embodiment, the fragment mediates the generation of a duplex by extension of the fragment on a capture oligonucleotide.

According to an embodiment, the mediation oligonucleotide comprises (i) a targeting portion comprising a hybridizing nucleotide sequence complementary to the target nucleic acid, and (ii) a tagging portion comprising a non-complementary nucleotide sequence to the target nucleic acid.

In one embodiment, the composition for detecting a target nucleic acid may include a tagging oligonucleotide that hybridizes with the target nucleic acid, and the cleavage reaction dependent on the presence of the target nucleic acid may involve cleavage of the tagging oligonucleotide. The tagging oligonucleotide corresponds to an example of the mediation oligonucleotide described above.

According to an embodiment, cleavage of the mediation oligonucleotide releases a fragment, and the fragment is specifically hybridized with a capture oligonucleotide and extended on the capture oligonucleotide. When the capture oligonucleotide comprises a label, the capture oligonucleotide corresponds to an example of the labeled oligonucleotide described herein.

According to an embodiment, the mediation oligonucleotide hybridized with a target nucleic acid is cleaved and releases a fragment, the fragment is specifically hybridized to a capture oligonucleotide, and the fragment is extended to generate an extended strand, which induces the formation of an extended duplex between the extended strand and the capture oligonucleotide, thereby providing a signal indicative of the presence of the target nucleic acid.

According to an embodiment, a third oligonucleotide comprising a hybridizing nucleotide sequence complementary to the extended strand may be additionally used. When the third oligonucleotide is used, the hybridization of the extended strand and the third oligonucleotide forms another type of duplex, thereby providing a signal indicating the presence of the target nucleic acid (e.g., PCE-SH). In this case, another type of duplex is a duplex providing the signal change.

Signals by a duplex generated in a manner dependent on cleavage of the mediation oligonucleotide may be generated by various methods, including PTOCE (PTO cleavage and extension) method (WO 2012/096523), PCE-SH (PTO Cleavage and Extension-Dependent Signaling Oligonucleotide Hybridization) method (WO 2013/115442), and PCE-NH (PTO Cleavage and Extension-Dependent Non-Hybridization) method (WO 2014/104818).

Regarding the terms disclosed in the above references, the corresponding examples of the oligonucleotides are as follows: the mediation oligonucleotide corresponds to a PTO (Probing and Tagging Oligonucleotide), the capture oligonucleotide corresponds to a CTO (Capturing and Templating Oligonucleotide), and the third oligonucleotide corresponds to an SO (Signaling Oligonucleotide) or an HO (Hybridization Oligonucleotide). The SO, HO, CTO, extended strand, or a combination thereof may play the role of a labeled oligonucleotide.

In one embodiment, the duplex providing the signal change may be a single-typed duplex or plural-typed duplexes. Specifically, when the duplex providing the signal change is a single-typed duplex, the number of the duplexes may be 1, and when the duplex providing the signal change is plural-typed duplexes, the number of the duplexes may be 2, 3, 4, 5, 6, 7, 8, 9, 10, 15, or 20, specifically, 2, 3, or 4, and more specifically, 2 or 3.

In one embodiment, the single-typed duplex, or any of the plural-typed duplexes includes a label.

In one embodiment, when the duplex providing the signal change is a single-typed duplex, the amount of the single-typed duplex changes depending on the presence of a target nucleic acid, thereby changing the signal.

In one embodiment, when the duplex is plural-typed duplexes, the amount ratios between the plural-typed duplexes changes depending on the presence of a target nucleic acid, thereby changing the signal. In the composition for detecting a target nucleic acid comprising two probes having different Tm values according to the present disclosure, the hybrid of the first probe with the target nucleic acid and the hybrid of the second probe with the target nucleic acid correspond to an example of plural-typed duplexes.

In one embodiment, Tm values of the plural-typed duplexes are different from each other. For example, the Tm values of the duplexes are different from each other by at least 2°C, at least 3°C, at least 4°C, at least 5°C, at least 7°C, at least 8°C, at least 9°C, at least 10°C, at least 11°C, at least 12°C, at least 13°C, at least 14°C, at least 15°C, or at least 20°C.

In one embodiment, the amount of the duplex refers to the sum of the amount of the duplex in a dissociated state, in which the two nucleic acid strands constituting the duplex are separated (i.e., a dissociated form of the duplex), and the amount of the duplex in an associated state, in which the two nucleic acid strands are hybridized (i.e., an associated form of the duplex).

In one embodiment, at least two of the plural-typed duplexes include the same single-stranded nucleic acid molecule. In this case, the same single-stranded nucleic acid molecule is included in a first duplex initially included in the composition for detecting the target nucleic acid, and during an incubation reaction, a new second duplex including the same single-stranded nucleic acid molecule may be generated. In this case, the same single-stranded nucleic acid molecule included in the first duplex can be considered to be consumed as it participates in the generation of the second duplex during the incubation reaction, and thus, the amount of the first duplex including the same single-stranded nucleic acid molecule decreases, whereas the amount of the second duplex including the same single-stranded nucleic acid molecule increases.

In one embodiment, the signal-changing temperature range of the composition for detecting the *i*^{th} target nucleic acid may be determined on the basis of the length and/or sequence of the duplex providing a signal change.

In one embodiment, when the composition for detecting the *i*^{th} target nucleic acid provides a single-typed duplex, the composition for detecting the target nucleic acid may have one signal-changing temperature range and one signal-constant temperature range. The signal-changing temperature range and signal-constant temperature range may be determined on the basis of the length and/or sequence of the single-typed duplex.

In one embodiment, when the composition for detecting the *i*^{th} target nucleic acid provides plural-typed duplexes, in particular, two different types of duplexes, the composition for detecting the target nucleic acid may have one signal-changing temperature range and two signal-constant temperature ranges. The signal-changing temperature range and the signal-constant temperature ranges may be determined on the basis of the lengths and/or sequences of the two different types of duplexes.

In one embodiment, any one of the *n* compositions for detecting the target nucleic acids may include an amplification oligonucleotide that serves to amplify a corresponding target nucleic acid. In one embodiment, the amplification oligonucleotide may be the same as the labeled oligonucleotide.

As used herein, the term "amplification oligonucleotide" refers to any oligonucleotides that serve to amplify target nucleic acids.

In one embodiment, the amplification oligonucleotide may be a 'primer' known in the art. As used herein, the term 'primer' refers to an oligonucleotide that is capable of acting as a point of initiation of synthesis when placed under conditions in which synthesis of primer extension product which is complementary to a target nucleic acid strand (template) is induced, *i.e.,* in the presence of nucleotides and an agent for polymerization, such as DNA polymerase, and at a suitable temperature and pH. The primer must be long enough to prime the synthesis of extension products in the presence of the agent for polymerization. An appropriate length of the primer is determined by multiple factors, including temperature, the field of application, and the source of primer.

The primer may include a forward primer (also referred to as an upstream primer or an upstream oligonucleotide), a reverse primer (also referred to as a downstream primer or a downstream oligonucleotide), or both. The amplification oligonucleotide may be an oligonucleotide having a structure known in the art, or may be synthesized by a method known in the art.

That the amplification oligonucleotide and the labeled oligonucleotide are the same means that a single oligonucleotide simultaneously acts as an amplification oligonucleotide that amplifies a target nucleic acid, and as a labeled oligonucleotide that generates a signal in the presence of the target nucleic acid. As one example, the labeled oligonucleotide may be hybridized with the target nucleic acid and extended, thereby generating a signal.

Note that the composition for detecting a target nucleic acid used in the present disclosure does not necessarily provide a signal at any temperature in the presence of the target nucleic acid.

In one embodiment, even when the signal generation mechanisms of the compositions for detecting target nucleic acids are the same, the compositions for detecting target nucleic acids including oligonucleotides of different sequences may be considered to be different from each other. The different compositions for detecting nucleic acids have different detection temperatures from each other.

In one embodiment, the detection temperatures according to the present disclosure may be pre-determined in light of the signal-changing temperature range of each of the *n* compositions for detecting target nucleic acids.

In one embodiment, the signal-changing temperature range of any one of the *n* compositions for detecting target nucleic acids may be determined on the basis of the length and/or sequence of the duplex. In other words, by adjusting the Tm value of the duplex, the signal-changing temperature range may be predetermined.

In one embodiment, when the signal change is generated by a labeled oligonucleotide (e.g., a molecular beacon) that specifically hybridizes to the target nucleic acid, the detection of signals may be successfully achieved at a predetermined detection temperature by adjusting the Tm value of the labeled oligonucleotide.

In one embodiment, when a scorpion primer is used as the labeled oligonucleotide, the detection of the signal is successfully achieved at a predetermined temperature by adjusting the Tm value of the region hybridizing with the extended strand.

In one embodiment, when the signal is provided by a duplex generated in the presence of the target nucleic acid, the detection of the signal is successfully achieved at a predetermined temperature by adjusting the Tm value of the duplex. For example, when the signal is generated by a PTOCE-based method, the detection of the signal is successfully achieved at a predetermined temperature by adjusting the Tm value of the extended duplex formed by the extension of the PTO fragment on the CTO.

The PTOCE-based method has the advantage of easily controlling the Tm value of the duplex or the Tm value of a third hybrid whose hybridization is affected by the duplex.

As described above, the detection temperature is determined in view of the signal-changing temperature range that varies depending on the duplex provided by the composition for detecting a target nucleic acid.

In one embodiment, the detection temperature of any one of the n compositions for the detection of *n* target nucleic acids may be predetermined within a signal-changing temperature range that does not overlap with the signal-changing temperature ranges of the other compositions (see FIG. 8)

In one embodiment, the detection temperatures assigned to the compositions for detecting the target nucleic acids are different from each other by at least 2°C, at least 3°C, at least 4°C, at least 5°C, at least 7°C, at least 8°C, at least 9°C, at least 10°C, at least 11°C, at least 12°C, at least 15°C, at least 20°C, or more.

In one embodiment, the n detection temperatures may be selected from within a temperature range of 45°C to 97°C, 45°C to 96°C, 45°C to 95°C, 45°C to 94°C, 45°C to 93°C, 45°C to 92°C, 45°C to 91°C, 45°C to 90°C, 46°C to 97°C, 46°C to 96°C, 46°C to 95°C, 46°C to 94°C, 46°C to 93°C, 46°C to 92°C, 46°C to 91°C, 46°C to 90°C, 47°C to 97°C, 47°C to 96°C, 47°C to 95°C, 47°C to 94°C, 47°C to 93°C, 47°C to 92°C, 47°C to 91°C, 47°C to 90°C, 48°C to 97°C, 48°C to 96°C, 48°C to 95°C, 48°C to 94°C, 48°C to 93°C, 48°C to 92°C, 48°C to 91°C, 48°C to 90°C, 49°C to 97°C, 49°C to 96°C, 49°C to 95°C, 49°C to 94°C, 49°C to 93°C, 49°C to 92°C, 49°C to 91°C, 49°C to 90°C, 50°C to 97°C, 50°C to 96°C, 50°C to 95°C, 50°C to 94°C, 50°C to 93°C, 50°C to 92°C, 50°C to 91°C, or 50°C to 90°C.

For example, the highest detection temperature (*i.e.,* the *n*^{th} detection temperature) among the *n* detection temperatures may be selected from within a temperature range of 70°C to 97°C, 70°C to 95°C, 70°C to 93°C, 70°C to 90°C, 73°C to 97°C, 73°C to 95°C, 73°C to 93°C, 73°C to 90°C, 75°C to 97°C, 75°C to 95°C, 75°C to 93°C, 75°C to 90°C, 78°C to 97°C, 78°C to 95°C, 78°C to 93°C, 78°C to 90°C, 80°C to 97°C, 80°C to 95°C, 80°C to 93°C, 80°C to 90°C, 83°C to 97°C, 83°C to 95°C, 83°C to 93°C, 83°C to 90°C, 85°C to 97°C, 85°C to 95°C, 85°C to 93°C, or 85°C to 90°C.

For example, the lowest detection temperature (*i.e.,* a first detection temperature) among the *n* detection temperatures may be selected from within a temperature range of 45°C to 70°C, 45°C to 68°C, 45°C to 65°C, 45°C to 63°C, 45°C to 60°C, 45°C to 58°C, 45°C to 55°C, 48°C to 70°C, 48°C to 68°C, 48°C to 65°C, 48°C to 63°C, 48°C to 60°C, 48°C to 58°C, 48°C to 55°C, 50°C to 70°C, 50°C to 68°C, 50°C to 65°C, 50°C to 63°C, 50°C to 60°C, 50°C to 58°C, or 50°C to 55°C.

For example, intermediate detection temperatures (for example, from a second detection temperature to the (*n-*1)^{th} detection temperature) among the *n* detection temperatures may be selected from within a temperature range of 55°C to 85°C, 55°C to 83°C, 55°C to 80°C, 55°C to 78°C, 55°C to 7.5°C, 55°C to 73°C, 55°C to 70°C, 55°C to 68°C, 55°C to 65°C, 55°C to 63°C, 55°C to 60°C, 58°C to 85°C, 58°C to 83°C, 58°C to 80°C, 58°C to 78°C, 58°C to 75°C, 58°C to 73°C, 58°C to 70°C, 58°C to 68°C, 58°C to 65°C, 58°C to 63°C, 58°C to 60°C, 60°C to 85°C, 60°C to 83°C, 60°C to 80°C, 60°C to 78°C, 60°C to 75°C, 60°C to 73°C, 60°C to 70°C, 60°C to 68°C, 60°C to 65°C, 60°C to 63°C, 63°C to 85°C, 63°C to 83°C, 63°C to 80°C, 63°C to 78°C, 63°C to 75°C, 63°C to 73°C, 63°C to 70°C, 63°C to 68°C, 63°C to 65°C, 65°C to 85°C, 65°C to 83°C, 65°C to 80°C, 65°C to 78°C, 65°C to 75°C, 65°C to 73°C, 65°C to 70°C, 65°C to 68°C, 68°C to 85°C, 68°C to 83°C, 68°C to 80°C, 68°C to 78°C, 68°C to 75°C, 68°C to 73°C, 68°C to 70°C, 70°C to 85°C, 70°C to 83°C, 70°C to 80°C, 70°C to 78°C, 70°C to 75°C, or 70°C to 73°C.

According to an embodiment, the *n* target nucleic acids are each assigned to *n* detection temperatures, *n* compositions for detecting the *n* target nucleic acids appropriate for the *n* detection temperatures are prepared, and then step (a) may be performed.

In one embodiment, when *n is* 3, the first detection temperature may be selected from within a temperature range of 50°C to 60°C, the second detection temperature may be selected from within a temperature range of 65°C to 75°C, and the third detection temperature may be selected from within a temperature range of 80°C to 95°C.

In step (a), signals are detected at the n detection temperatures during the incubating.

In one embodiment, the detection of signals may be carried out in each cycle, in selected cycles, or at the end-point of reaction.

In one embodiment, the detection of signals may be carried out in at least one cycle. For example, the signals may be detected at *n* detection temperatures in one cycle selected or at *n* detection temperatures in each of two cycles selected. For example, when *n* is 3 and signals are detected in cycle 1 and cycle 30, the signals (*i.e.*, a first signal, a second signal, and a third signal) are detected at a first detection temperature, a second detection temperature, and a third detection temperature in cycle 1, and the signals are detected at a first detection temperature, a second detection temperature, and a third detection temperature in cycle 30.

In one embodiment, the detection of the signals may be carried out in at least two cycles.

In one embodiment, the signal change may be measured using the signals detected in the at least two cycles. For example, the amplification of nucleic acids may be carried out over 30 cycles, 40 cycles, 45 cycles, or 50 cycles of PCR, and in each cycle, signals may be measured at *n* detection temperatures. Then, the values of signals detected at each detection temperature in a plurality of cycles may be depicted as an amplification curve (a collection of data points of cycles and RFUs in cycles) at each detection temperature. As a specific example, when *n is* 3, an amplification curve at the first detection temperature, an amplification curve at the second detection temperature, and an amplification curve at the third detection temperature may be obtained, and then change in signal may be obtained from each of the amplification curves.

The term "amplification curve" as used herein refers to a curve resulting from a signal-generation reaction, in particular an amplification reaction of a target analyte (in particular, target nucleic acid). The amplification curve includes a curve resulting from an amplification reaction in the presence of the target nucleic acid in the sample, or a curve or line resulting from an amplification reaction in the absence of the target nucleic acid in the sample.

In one embodiment, a signal change and/or a constant signal may be measured from an indicator of amplification of a target nucleic acid.

As used herein, the term "indicator of amplification" refers to any indicator which is closely related to the occurrence of amplification of a target nucleic acid and obtainable from the signal provided in step (a). The indicator may refer to a value which is generated dependently on the amplification of a target nucleic acid. The indicator may be an indicator having larger values as the target nucleic acid is amplified (*i.e.*, as the amount of the target nucleic acid increases), or may be an indicator having smaller values as the target nucleic acid is amplified. The indicator may be any indicator as long as it indicates amplification of the target nucleic acid.

In one embodiment, the indicator may include one obtained from an amplification curve. The indicator may include a signal value (e.g., RFU) in a particular cycle, a signal value in each cycle, a difference in signal values between particular cycles, or a difference between a reference signal value and a signal value in a particular cycle in an amplification curve. In one embodiment, examples of the indicator include, without limitation, Ct (cycle threshold) value, ΔRFU (e.g., difference in RFUs in two cycles, difference between a reference RFU and a RFU in a particular cycle, etc.) or RFU ratio (e.g., ratio of RFUs in two cycles or ratio of RFUs between a reference RFU and a RFU in a particular cycle, etc.).

According to an embodiment, the indicator of amplification is the Ct value or Cq value. The concept of the Ct value and Cq value are well known in the art.

According to an embodiment, the indicator of amplification is the ΔRFU or RFU ratio between RFU values obtained in an amplification reaction. For example, the indicator is the difference (subtraction) or ratio between RFUs in two cycles, or the difference (subtraction) or ratio between a RFU in a particular cycle and a reference RFU.

The method according to the present disclosure exploits the fact that the composition for detecting a target nucleic acid provides a signal change depending on the presence of the target nucleic acid, only at a corresponding detection temperature. According to an embodiment, the method according to the present disclosure can measure a signal change using signal values detected at detection temperatures in at least two cycles. In another embodiment, the method according to the present disclosure can measure a signal change by using a signal value detected at a detection temperature in one cycle (*i.e.,* a signal value detected in step (a)), and a reference signal value.

In one embodiment, when signals are detected in a plurality of cycles in step (a), the first cycle and the end cycle in which signals are detected may be selected to be separated from each other by at least 1 cycle to at least 20 cycles. In particular, the first cycle and the end cycle in which the signals are detected may be selected to be separated from each other by 1 cycle, 2 cycles, 3 cycles, 4 cycles, 5 cycles, 6 cycles, 7 cycles, 8 cycles, 9 cycles, 10 cycles, 11 cycles, 12 cycles, 13 cycles, 14 cycles, 15 cycles, 16 cycles, 17 cycles, 18 cycles, 19 cycles, 20 cycles, or more, and more specifically, by 5 cycles, 10 cycles, 15 cycles, 20 cycles, 30 cycles, or more.

In one embodiment, the detection of signals may be performed in at least one of intermediate cycles including an exponential phase region, or in at least one of late cycles including a plateau region. For example, when signals may be detected in two cycles, one cycle being any of early cycles including a baseline region and the other cycle being any of intermediate or late cycles, or one cycle being any of intermediate cycles and the other cycle being any of intermediate or late cycles.

In one embodiment, the early cycles include cycles from cycle 1 to any cycle close to the value obtained by dividing the end cycle by 3. For example, when the end cycle is 45, 45 divided by 3 equals to 15, and thus, the early cycles may be determined to be from cycle 1 to cycle 20, cycle 1 to cycle 15, cycle 1 to cycle 10, or cycle 1 to cycle 5. The intermediate cycles may be cycles close to a value obtained by dividing the end cycle by 2. For example, when the end cycle is cycle 45, 45 divided by 2 equals to 22.5, and thus, the intermediate cycles may be from cycle 16 to cycle 30, cycle 18 to cycle 30, cycle 20 to cycle 30, cycle 16 to cycle 27, cycle 18 to cycle 27, cycle 20 to cycle 27, cycle 16 to cycle 25, cycle 18 to cycle 25, or cycle 20 to cycle 25. The late cycles may be the end cycle of, or cycles close to the end cycle of an amplification reaction. For example, when the end cycle is cycle 45, the late cycles may be from cycle 31 to cycle 45, cycle 35 to cycle 45, cycle 38 to cycle 45, cycle 40 to cycle 45, or cycle 43 to cycle 45. The early cycles, intermediate cycles and late cycles may vary depending on the end cycle of the amplification reaction.

In one embodiment, the signal change may be measured using a signal value detected in at least one cycle and a "reference signal value". The reference signal value may refer to a value that can be used to confirm a signal change dependent on the presence of the target nucleic acid through a separate reaction.

In one embodiment, the reference signal value may be obtained from a reaction in the absence of a corresponding target nucleic acid at a corresponding detection temperature. For example, the reference signal value may be a "signal value at a detection temperature" in the absence of the target nucleic acid.

In one embodiment, there are *n* reference signal values for *n* detection temperatures.

In one embodiment, the "signal value at a detection temperature (e.g., an *i*^{th} detection temperature)" detected in the absence of the target nucleic acid (e.g., an *i*^{th} target nucleic acid) may be obtained through a separate negative control reaction.

In one embodiment, the reference signal value may be obtained by performing a negative control reaction at the same time as or separately from the method according to the present disclosure.

In one embodiment, the reference signal value may be obtained through a negative control reaction. According to certain embodiments, the reference signal value at the *i*^{th} detection temperature may be obtained by mixing a sample (e.g., distilled water) free of the *i*^{th} target nucleic acid with *n* compositions for detecting *n* target nucleic acids and detecting signals at the *i*^{th} detection temperature while amplifying nucleic acids. Here, the detection of signals may be performed in any cycle. Specifically, a signal value detected in any of the early cycles or late cycles of the negative control reaction may be used as the reference signal value. More specifically, a signal value detected in the same cycle as the cycle in which the signal is detected in step (a) may be used as the reference signal value.

In one embodiment, the reference signal value may be obtained through a positive control reaction. According to certain embodiments, the reference signal value may be obtained by mixing a sample containing an *i*^{th} target nucleic acid with the composition for detecting an *i*^{th} target nucleic acid and detecting a signal at an *i*^{th} detection temperature while amplifying the target nucleic acids.

When the reference signal value is obtained through the positive control reaction, the cycle in which a signal value is detected may be in a baseline region of the reaction. The baseline region refers to a region in which a signal (e.g., a fluorescent signal) remains substantially constant over the early cycles of an amplification reaction (e.g., PCR). In this region, since the level of amplification products is not enough to be detectable, most of the fluorescent signals in this region are attributed to the fluorescent signal inherent to the reaction sample, and to the background signal including the fluorescent signals of the measurement system itself. In other words, a signal value detected in a cycle in the baseline region of the positive control reaction will be substantially identical to the reference signal value obtained from a reaction in the absence of the target nucleic acid (e.g., the negative control reaction).

In one embodiment, a signal change may be measured through a difference between the reference signal value and the signal value detected in step (a).

In one embodiment, the reference signal value may be a threshold value predetermined from a negative control reaction, by taking into consideration the background signal and sensitivity of the detector, or characteristics of the labels used. Using the threshold value, significance of a signal change may be determined. The threshold value may be determined by any threshold setting method known in the art. For example, the threshold value may be determined in view of the background signal, sensitivity, label characteristics, signal variation of a detector, or margin of errors, and the like.

In one embodiment, when the signal value detected in step (a) is equal to or greater than the threshold value as the reference signal value, it may be determined that the signal has changed.

In one embodiment, detection of signals at each of the *n* detection temperatures may be performed using a single type of detector.

In one embodiment, the single type of detector is one detector. In one embodiment, the signals from the labels in each of the labeled oligonucleotides included in the *n* compositions for detecting target nucleic acids are not differentiated from each other by a single type of detector with respect to each target nucleic acid.

As used herein, a single or one type of fluorescent label refers to a fluorescent label that has identical or substantially identical signal characteristics (e.g., optical characteristics, emission wavelength, and electric signals). For example, FAM and CAL Fluor 610 provide different types of signals.

As used herein, the single or one type of fluorescent label means that signals from the fluorescent label are not differentiated from each other, using a detection channel. Such a single or one type of fluorescent label is not based on the chemical structure of the fluorescent label, and even when two fluorescent labels having different chemical structures are not differentiated using a detection channel, they are considered as one type.

According to the present disclosure, signals generated from the *n* compositions for detecting target nucleic acids that include one type of fluorescent label in common are not differentiated by one detection channel.

The term "detection channel" as used herein refers to a means for detecting a signal from a single type of a fluorescent label. Thermocyclers usable in the art, e.g., ABI 7500 (Applied Biosystems), QuantStudio (Applied Biosystems), CFX96 (Bio-Rad Laboratories), Cobas z 480 (Roche), LightCycler (Roche), etc. include a several channels (e.g., optical diodes) for detecting signals from a few different types of fluorescent labels, and these channels correspond to the detection channel as used herein.

The detection channel as used herein includes a means for detecting signals. For example, the detection channel may be an optical diode capable of detecting a fluorescent signal at a particular wavelength.

In one embodiment, the signals detected at the *n* detection temperatures are not differentiated from each other by the single type of detector.

### Step (b): Determining the Presence of Target Nucleic Acid

After detection of signals, the presence of *n* target nucleic acids is determined from the signals detected in step (a).

In one embodiment, the presence of the *i*^{th} target nucleic acid is determined by the signal change detected at the *i*^{th} detection temperature. For example, a signal change is measured from the signals detected at the *i*^{th} detection temperature, to determine the presence of the *i*^{th} target nucleic acid.

In one embodiment, when a change in signal at the *i*^{th} detection temperature is detected, it may be determined that the *i*^{th} target nucleic acid is present.

In one embodiment, when the signal is constant at the *i*^{th} detection temperature, it may be determined that the *i*^{th} target nucleic acid is absent.

In one embodiment, the signal change can be measured using signals detected in at least two cycles, or a signal value detected in at least one cycle and "a reference signal value".

Determining the presence of the target nucleic acid from signals detected at each detection temperature may be carried out by the process described in step (a) for measuring a signal change, e.g., a method using an indicator of amplification, or any other method known in the art.

In certain embodiments, when *n* is 3 and detection of signals is carried out in cycle 10, cycle 20, and cycle 30, the presence of the first target nucleic acid may be determined from signals detected at a first detection temperature (a first signal in cycle 10, a first signal in cycle 20, and a first signal in cycle 30), the presence of the second target nucleic acid may be determined from signals detected at a second detection temperature (a second signal in cycle 10, a second signal in cycle 20, and a second signal in cycle 30), and the presence of the third target nucleic acid may be determined from signals detected at a third detection temperature (a third signal in cycle 10, a third signal in cycle 20, and a third signal in cycle 30).

In certain embodiments, when *n* is 4 and detection of signals is performed in cycle 30, the presence of the first target nucleic acid is determined from a signal detected at a first detection temperature (*i.e.*, a first signal in cycle 30) and a reference signal value, the presence of the second target nucleic acid is determined from a signal detected at a second detection temperature (*i.e.*, a second signal in cycle 30) and a reference signal value, and the presence of the third target nucleic acid is determined from a signal detected at a third detection temperature (*i.e.*, a third signal in cycle 30) and a reference signal value.

In one embodiment, the reference signal value may be obtained through a separate negative control reaction or positive control reaction.

In one embodiment, the method according to the present disclosure may be performed along with a negative control reaction. A signal value detected in the negative control reaction may be used as a reference signal value. For example, a signal detected at an *i*^{th} detection temperature in one cycle (e.g., the end cycle) of a reaction comprising the composition for detecting an *i*^{th} target nucleic acid, may be compared with a signal detected at the same detection temperature (*i.e.*, the *i*^{th} detection temperature) in the same cycle (e.g., the end cycle) of the negative control reaction to determine whether or not the signal has changed.

In certain embodiments, when *n* is 3 and a signal is detected in cycle 30, the presence of the first target nucleic acid may be determined from a signal detected at the first detection temperature (*i.e.*, a first signal in cycle 30) and a first reference signal value (e.g., a signal detected at the first detection temperature in cycle 30 of a negative control reaction), the presence of the second target nucleic acid may be determined from a signal detected at the second detection temperature (*i.e.*, a second signal in cycle 30) and a second reference signal value (e.g., a signal detected at the second detection temperature in cycle 30 of a negative control reaction), and the presence of the third target nucleic acid may be determined from a signal detected at the third detection temperature (*i.e.*, *a* third signal in cycle 30) and a third reference signal value (e.g., a signal detected at the third detection temperature in cycle 30 of a negative control reaction).

In one embodiment, the method according to the present disclosure may be performed along with a positive control reaction. A signal value detected in the positive control reaction may be used as a reference signal value. For example, a first signal detected at an *i*^{th} detection temperature in one cycle, e.g., cycle 30, may be compared with a signal detected at the *i*^{th} detection temperature in a cycle, e.g., cycle 1 prior to cycle 30 of a positive control reaction to determine whether the signal has changed.

In one embodiment, when signal detection is performed in one cycle in step (a) and a signal change is measured using a reference signal value obtained through a positive control reaction, the signal detection in the positive control reaction to obtain the reference signal value may be performed in a cycle that is at least 30 cycles, at least 20 cycles, at least 10 cycles, or at least 5 cycles prior to the cycle in which the signal detection is performed in step (a).

In certain embodiments, when *n* is 3 and signal detection is performed in cycle 30, the presence of the first target nucleic acid may be determined from a signal detected at the first detection temperature (*i.e.*, a first signal in cycle 30) and a first reference signal value (e.g., a signal detected at the first detection temperature in cycle 1 of a positive control reaction for the first target nucleic acid), the presence of the second target nucleic acid may be determined from a signal detected at the second detection temperature (*i.e.*, a second signal in cycle 30) and a second reference signal value (e.g., a signal detected at the second detection temperature in cycle 1 of a positive control reaction for the second target nucleic acid), and the presence of the third target nucleic acid may be determined from a signal detected at the third detection temperature (*i.e.*, a third signal in cycle 30) and a third reference signal value (e.g., a signal detected at the third detection temperature in cycle 1 of a positive control reaction for the third target nucleic acid).

### II. Method for Detecting Target Nucleic Acid in Sample

In another aspect, the present disclosure provides a method for detecting a target nucleic acid in a sample, comprising:
(a) incubating the sample with a composition for detecting the target nucleic acid comprising a first probe and a second probe having different Tm values;
   wherein the first probe comprises a hybridizing nucleotide sequence to a first region of the target nucleic acid,
   wherein the first probe has a reporter molecule and a first quencher molecule,
   wherein the reporter molecule and the first quencher molecule are located at positions such that (i) the reporter molecule and the first quencher molecule are in close proximity to each other, prior to hybridization of the first probe with the target nucleic acid, thereby causing the first quencher molecule to quench a signal from the reporter molecule, and (ii) the reporter molecule and the first quencher molecule are separated upon hybridization of the first probe with the target nucleic acid, thereby causing the first quencher molecule to unquench a signal from the reporter molecule,
   wherein the second probe comprising a hybridizing nucleotide sequence to a second region of the target nucleic acid,
   wherein the second probe has a second quencher molecule,
   wherein the Tm value of the first probe is higher than the Tm value of the second probe, and
   wherein the reporter molecule of the first probe is quenched by the second quencher molecule of the second probe when the first probe and the second probe are hybridized with the target nucleic acid; and
(b) detecting a signal; and
(c) determining the presence of the target nucleic acid from the signal detected in the step (b).

The second aspect of the present disclosure utilizes a composition for detecting a target nucleic acid comprising two probes having different Tm values, as described in the first aspect of the present disclosure. Therefore, the common descriptions between them are omitted in order to avoid undue redundancy leading to complexity in this specification.

The method according to the present disclosure is described below step by step.

### Step (a): Incubating

First, a sample suspected to contain a target nucleic acid is mixed with a composition for detecting a target nucleic acid, which comprises a first probe and a second probe having different Tm values according to the present disclosure, and then incubated.

In one embodiment, the incubation reaction refers to a reaction in which the target nucleic acid reacts with the composition for detecting a target nucleic acid, thereby providing a signal depending on the presence of the target nucleic acid at a selected temperature within the signal-changing temperature range of the composition for detecting a target nucleic acid.

In one embodiment, the incubating in step (a) includes an amplification reaction, specifically, an amplification reaction of the target nucleic acid.

In one embodiment, the incubating comprises a plurality of cycles.

A detailed description of the incubating is provided in the section describing the incubating in the first aspect of the present disclosure, as described above.

The first probe comprises a hybridizing nucleotide sequence to a first region of the target nucleic acid. That is, the first probe is capable of hybridizing to the first region of the target nucleic acid.

The first probe has a reporter molecule and a first quencher molecule.

In one embodiment, before the first probe hybridizes with the target nucleic acid, the reporter molecule and the first quencher molecule linked to the first probe are positioned in close proximity to each other. As a result, the first quencher molecule linked to the first probe quenches the signal from the reporter molecule linked to the first probe.

In one embodiment, the first probe forms a random coil or hairpin structure, prior to hybridization of the first probe with the target nucleic acid, thereby allowing the first quencher molecule linked to the first probe to intramolecularly quench a signal from the reporter molecule linked to the first probe.

In one embodiment, when the first probe hybridizes with the target nucleic acid, the reporter molecule and the first quencher molecule of the first probe are separated from each other. Specifically, upon hybridization of the first probe with the target nucleic acid, the first quencher molecule of the first probe is separated from the reporter molecule of the first probe, thereby causing the first quencher molecule to unquench a signal from the reporter molecule.

In one embodiment, the reporter molecule and the first quencher molecule are located at positions such that (i) the reporter molecule and the first quencher molecule are in close proximity to each other, prior to hybridization of the first probe with the target nucleic acid, thereby causing the first quencher molecule to quench a signal from the reporter molecule, and (ii) the reporter molecule and the first quencher molecule are separated upon hybridization of the first probe with the target nucleic acid, thereby causing the first quencher molecule to unquench a signal from the reporter molecule.

In one embodiment, the reporter molecule is linked to the 3'-end portion or the 5'-end portion of the first probe.

The second probe comprises a hybridizing nucleotide sequence to a second region of the target nucleic acid. That is, the second probe is capable of hybridizing to the second region of the target nucleic acid.

Additionally, the second probe has a second quencher molecule.

In the present disclosure, the first region and the second region of the target nucleic acid are adjacent to each other.

In one embodiment, when the first probe and the second probe hybridize with the target nucleic acid, the reporter molecule of the first probe is quenched by the second quencher molecule of the second probe. Specifically, when the first probe is hybridized with the first region of the target nucleic acid, the second probe hybridizes with the second region of the target nucleic acid to allow the second quencher molecule of the second probe to be adjacent to the reporter molecule of the first probe hybridized with the first region of the target nucleic acid. As a result, the second quencher molecule intermolecularly quenches a signal from the reporter molecule.

In one embodiment, when the first probe and the second probe hybridize with the target nucleic acid, the second quencher molecule of the second probe is positioned adjacent to the reporter molecule of the first probe. As a result, the reporter molecule and the second quencher molecule are located at positions such that the second quencher molecule can quench a signal from the reporter molecule.

In one embodiment, the second quencher molecule is linked to the 3'-end portion or the 5'-end portion of the second probe.

In one embodiment, when the second probe is hybridized with the target nucleic acid in the 3' direction downstream of the first probe, the reporter molecule is located at the 3'-end portion of the first probe, and the second quencher molecule is located at the 5'-end portion of the second probe.

In one embodiment, when the second probe is hybridized with the target nucleic acid in the 5' direction upstream of the first probe, the reporter molecule is located at the 5'-end portion of the first probe, and the second quencher molecule is located at the 3'-end portion of the second probe.

In one embodiment, the first probe and/or the second probe are "blocked" at its 3'-end to prevent its extension.

The reporter molecule and the quencher molecule used in the present disclosure are interactive labels.

In one embodiment, the second quencher molecule linked to the second probe is the same type of quencher molecule as the first quencher molecule linked to the first probe.

Through the incubation reaction in step (a), the composition for detecting the target nucleic acid according to the present disclosure, which comprises the first probe and the second probe, reacts with the target nucleic acid to provide a signal dependent on the presence of the target nucleic acid. Specifically, the composition for detecting the target nucleic acid comprising the first probe and the second probe according to the present disclosure provides a change in signal as the target nucleic acid is amplified.

The first probe and the second probe according to the present disclosure are characterized by having different Tm values.

By using two probes with different Tm values, the composition for detecting the target nucleic acid according to the present disclosure has a temperature range in which the signal changes depending on the presence of the target nucleic acid during a reaction with the target nucleic acid (e.g., an amplification reaction), referred to as the signal-changing temperature range, and two temperature ranges in which the signal is constant even in the presence of the target nucleic acid, referred to as the signal-constant temperature ranges (see FIGs. 1 to 3).

Specifically, the composition for detecting a target nucleic acid, which comprises the first probe and the second probe having different Tm values, is an InterSC composition having a characteristic that the signal-changing temperature range is higher than the first signal-constant temperature range among the two signal-constant temperature ranges and lower than the second signal-constant temperature range among the two signal-constant temperature ranges.

In one embodiment, during the reaction (e.g., a target nucleic acid amplification reaction) between the composition for detecting the target nucleic acid according to the present disclosure and the target nucleic acid, the amounts of the first probe and the second probe remain constant at the concentrations initially included in the composition for detecting the target nucleic acid. As described in the first aspect above, the first probe and the second probe included in the composition for detecting the target nucleic acid may exist in the forms shown in FIGS. 1 to 3, depending on the presence of the target nucleic acid and/or temperature changes. Moreover, the amounts of the first probe and the second probe existing in the forms shown in FIGS. 1 to 3 vary depending on the degree of reaction with the target nucleic acid (e.g., the degree of amplification of the target nucleic acid). Specifically, the amount of the first probe and the second probe that have reacted with the target nucleic acid increases as the target nucleic acid is amplified. In contrast, the amount of the first probe and the second probe that have not reacted with the target nucleic acid decreases as the target nucleic acid is amplified. That is, the amount ratio changes, and as the target nucleic acid is amplified, the signal indicating the presence of the target nucleic acid also changes. Due to this change in the amount ratio, the signal indicating the presence of the target nucleic acid within the signal-changing temperature range also changes. Accordingly, the composition for detecting a target nucleic acid according to the present disclosure can provide a signal dependent on the presence of the target nucleic acid. Meanwhile, even though the total amount ratio between the first probe and/or the second probe that have reacted with the target nucleic acid and the first probe and/or the second probe that have not reacted with the target nucleic acid changes as the target nucleic acid is amplified, the signal remains constant within the two signal-constant temperature ranges.

In one embodiment, the first probe and the second probe are hybridized with the target nucleic acid at temperatures within the first signal-constant temperature range in the presence of the target nucleic acid.

In one embodiment, the first probe is hybridized with the target nucleic acid but the second probe is not hybridized with the target nucleic acid at temperatures within the signal-changing temperature range in the presence of the target nucleic acid.

In one embodiment, the first probe and the second probe are not hybridized with the target nucleic acid at temperatures within the second signal-constant temperature range in the presence of the target nucleic acid.

In one embodiment, the signal-changing temperature range is determined depending on the Tm values of the first probe and the second probe. Accordingly, the signal-changing temperature range and the two signal-constant temperature ranges can be controlled by adjusting the Tm values of the first probe and the second probe.

### Step (b): Detection of Signal

In this step, a signal indicating the presence of the target nucleic acid is detected.

In one embodiment, the detection of the signal indicating the presence of the target nucleic acid may be achieved by measuring the signal that changes during the incubating reaction, that is, as the target nucleic acid is amplified.

In one embodiment, the signal detected in step (b) is a signal dependent on the presence of the target nucleic acid.

In one embodiment, step (b) is performed at a selected temperature (i.e., detection temperature) within the signal-changing temperature range.

In one embodiment, the detection of the signal in step (b) is performed during and/or after the incubation reaction. Specifically, the detection of signals may be carried out in each cycle, in a selected few cycles, or at the end-point of an incubation reaction including a plurality of cycles.

In one embodiment, the detection of the signal in step (b) is performed in at least one cycle among a plurality of cycles. For example, the detection may be performed in at least one cycle during an incubation reaction including a plurality of cycles, specifically in an intermediate cycle or a late cycle.

In one embodiment, the detection of the signal in step (b) may be performed in a single cycle during an incubation reaction including a plurality of cycles. In this case, it may be difficult to determine whether the signal changes dependently on the presence of the target nucleic acid during the incubation reaction based solely on the signal value detected in the single cycle. Therefore, a separate reference signal value may be used to determine the signal indicative of the presence of the target nucleic acid.

The reference signal value may refer to a value that allows confirmation of a signal change dependent on the presence of the target nucleic acid through a separate reaction. In one embodiment, the reference value may be obtained from a reaction in which the target nucleic acid is absent (e.g., a negative control reaction). For example, the reference signal value may be a "signal value at the detection temperature" detected in the absence of the target nucleic acid.

In one embodiment, the detection of the signal in step (b) is performed in at least two cycles. For example, it may be performed in at least two cycles during an incubation reaction including a plurality of cycles. The signal values at the detection temperature detected in at least two cycles may be measured to determine the signal change.

### Step (c): Determination of the Presence of the Target Nucleic Acid

Finally, the presence of the target nucleic acid is determined from the signal detected in step (b).

In one embodiment, the presence of the target nucleic acid in step (c) is determined by the signal change.

In one embodiment, step (c) involves measuring the signal change using signals detected in at least two cycles among the plurality of cycles. If the signal changes, it is determined that the target nucleic acid is present. On the other hand, if the signal remains constant, it is determined that the target nucleic acid is absent.

In one embodiment, the presence of the target nucleic acid in step (c) is determined by measuring the signal change using the signal detected in at least one cycle among the plurality of cycles and the aforementioned reference signal value. If the signal changes, it is determined that the target nucleic acid is present. On the other hand, if the signal remains constant, it is determined that the target nucleic acid is absent.

### III. Composition for Detecting Target Nucleic Acid in Sample

In another aspect, the present disclosure provides a composition for detecting a target nucleic acid in a sample, comprising:
(a) a first probe comprising a hybridizing nucleotide sequence to a first region of the target nucleic acid;
   wherein the first probe has a reporter molecule and a first quencher molecule,
   wherein the reporter molecule and the first quencher molecule are located at positions such that (i) the reporter molecule and the first quencher molecule are in close proximity to each other, prior to hybridization of the first probe with the target nucleic acid, thereby causing the first quencher molecule to quench a signal from the reporter molecule, and (ii) the reporter molecule and the first quencher molecule are separated upon hybridization of the first probe with the target nucleic acid, thereby causing the first quencher molecule to unquench a signal from the reporter molecule; and
(b) a second probe comprising a hybridizing nucleotide sequence to a second region of the target nucleic acid;
   wherein the second probe has a second quencher molecule,
   wherein the Tm value of the first probe is higher than the Tm value of the second probe, and
   wherein the reporter molecule of the first probe is quenched by the second quencher molecule of the second probe when the first probe and the second probe are hybridized with the target nucleic acid.

The third aspect of the present disclosure is prepared to perform the method of the second aspect of the present disclosure. Therefore, the common descriptions between them are omitted in order to avoid undue redundancy leading to the complexity of this specification.

### IV. Kit for Detecting Target Nucleic Acid

In another aspect, the present disclosure provides a kit comprising *n* compositions for detecting *n* target nucleic acids in a sample.
wherein *n is* an integer of 2 or more,
wherein each of the *n* compositions for detecting the target nucleic acids provides a signal change at a corresponding detection temperature among the *n* detection temperatures in the presence of a corresponding target nucleic acid, the signal change indicating the presence of a corresponding target nucleic acid,
wherein a composition for detecting an *i*^{th} target nucleic acid among the *n* compositions for detecting the target nucleic acids provides a signal change at an *i*^{th} detection temperature among the *n* detection temperatures and provides a constant signal at the other detection temperatures in the presence of the *i*^{th} target nucleic acid, the signal change indicating the presence of the *i*^{th} target nucleic acid,
wherein *i* represents an integer from 1 to *n,* and the *i*^{th} detection temperature is lower than the (*i*+1)*^{th}* detection temperature,
wherein in the temperature range covering all of the *n* detection temperatures, the composition for detecting the *i*^{th} target nucleic acid has a signal-changing temperature range in which the signal changes depending on the presence of the *i*^{th} target nucleic acid, and one or two signal-constant temperature ranges in which the signal is constant even in the presence of the *i*^{th} target nucleic acid,
wherein the composition for detecting the *i*^{th} target nucleic acid is any one of:
   (i) an Under-Signal-Change (UnderSC) composition having a characteristic that the signal-changing temperature range is lower than the signal-constant temperature range,
   (ii) an Inter-Signal-Change (InterSC) composition having a characteristic that the signal-changing temperature range is higher than one of two signal-constant temperature ranges, and lower than the other of the two signal-constant temperature ranges, and
   (iii) an Over-Signal-Change (OverSC) composition having a characteristic that the signal-changing temperature range is higher than the signal-constant temperature range,
wherein at least one of the *n* compositions for detecting target nucleic acids comprises a first probe and a second probe having different Tm values,
wherein the first probe comprises a hybridizing nucleotide sequence to a first region of a corresponding target nucleic acid,
wherein the first probe has a reporter molecule and a first quencher molecule,
wherein the reporter molecule and the first quencher molecule are located at positions such that (i) the reporter molecule and the first quencher molecule are in close proximity to each other, prior to hybridization of the first probe with the corresponding target nucleic acid, thereby causing the first quencher molecule to quench a signal from the reporter molecule, and (ii) the reporter molecule and the first quencher molecule are separated upon hybridization of the first probe with the corresponding target nucleic acid, thereby causing the first quencher molecule to unquench a signal from the reporter molecule,
wherein the second probe comprises a hybridizing nucleotide sequence to a second region of the corresponding target nucleic acid,
wherein the second probe has a second quencher molecule,
wherein the Tm value of the first probe is higher than the Tm value of the second probe, and
wherein the reporter molecule of the first probe is quenched by the second quencher molecule of the second probe when the first probe and the second probe are hybridized with the corresponding target nucleic acid.

The kit of the present disclosure is prepared to perform the method of the present disclosure. Therefore, the common descriptions between them are omitted in order to avoid undue redundancy leading to the complexity of this specification.

According to an embodiment, the kit may further comprise instructions describing the method of the present invention.

According to an embodiment, the instructions for describing or practicing the methods of the present invention may be recorded on a suitable storage medium. For example, the instructions may be printed on a substrate, such as paper and plastic. In other embodiments, the instructions may be present as an electronic storage data file present on a suitable computer readable storage medium such as CD-ROM and diskette. In yet other embodiments, the instructions may not actually be present in the kit, but means for obtaining the instructions from a remote source, e.g. via the internet, are provided. An example of this embodiment is a kit that includes a web address where the instructions can be viewed and/or from which the instructions can be downloaded.

Hereinafter, the present invention will be described in more detail through embodiments. The following embodiments are provided to describe the present invention in further details, and it will become apparent to one of ordinary skill in the art in the technical field to which this invention belongs that the scope of the present invention as suggested in the appended claims is not limited by the following embodiments.

### Mode for Invention

### Example

### Example 1: Detection of Target Nucleic Acid Using Two Probes Having Different Tm Values

It was examined whether the detection of a target nucleic acid is possible using the first probe and the second probe having different Tm values according to the present disclosure.

### 1-1. Preparation of Target Nucleic Acid and Oligonucleotides

The genome DNA of Ureaplasma parvum (UP) (Accession No: ATCC 27815) was used as the template for the target nucleic acid. The detection temperature of the UP target nucleic acid was set to 68°C, and the first region and the second region were selected on the target nucleic acid template in the 3' to 5' direction.

Then, as shown in Table 3, the sequences and lengths of the first probe and the second probe were designed such that the Tm value of the first probe was 70°C and the Tm value of the second probe was 64°C. Specifically, the first probe comprised a sequence complementary to the first region of the target nucleic acid and had a first quencher molecule (AZOQ2) at its 5'-end and a reporter molecule (Cal Fluor Red 610) at its 3'-end. The second probe comprised a sequence complementary to the second region of the target nucleic acid and had a second quencher molecule (AZOQ2) at its 5'-end and Spacer C3 at its 3'-end to prevent extension by DNA polymerase.

A primer pair for amplifying the region comprising the first region and the second region of the target nucleic acid was designed as shown in Table 3.

**[Table 3]**

| Oligonucleotide Sequences | | | |
|---|---|---|---|
| Target nucleic acid | SEQ NO. | Oligo type | Sequence (5' -3') |
| UP | 1 | The first and second | AGATTTATACGGTATTACAACTGGTGATAGCGTT AGATTAGGAGATACAAATCTTTGAGTTAAAGTT |
| | | regions of the target nucleic acid | GAAAAAGACTTAACTACTTATGGTGAAGAATC TGTTTTTGGTGGTGGAAAAACCCTACGTGAAG GTATGGGAATGAATTCTACTATGAAGTTAGATGA TAAATTAGGTAATGCTGAAGT |
| | 2 | UP-Forward Primer | AGATTTATACGGTATTACAACTG |
| | 3 | UP-Reverse Primer | ACTTCAGCATTACCTAATTTATC |
| | 4 | UP-First Probe | [AZOQ2]GGTGAAGAATCTGTTTTTGGTGGTGG AAA[Cal Fluor Red 610] |
| | 5 | UP-Second Probe | [AZOQ2]CCTACGTGAAGGTATGGGAAT[Spacer C3] |

| | | | |
|---|---|---|---|
| Bold: The first region of the target nucleic acid. Underlined: The second region of the target nucleic acid. | | | |

### 1-2. Real-time Polymerase Chain Reaction

Real-time polymerase chain reaction (PCR) was performed using the above oligonucleotides.

The target nucleic acid (Tube 1: 500 pg of UP genomic DNA; Tube 2: distilled water (negative control)) was mixed with: 5 pmole of UP-forward primer (SEQ No: 2), 20 pmole of UP-reverse primer (SEQ NO: 3), 5 pmole of UP-first probe (SEQ NO: 4), and 5 pmole of UP-second probe (SEQ NO: 5), and then combined with DNA polymerase (2U SD Polymerase HotStart (BIORON, Germany)), 1 mM dNTP, and 100 mM MgSO₄, to prepare a reaction mixture in a final volume of 20 µL. The concentration of the reverse primer was adjusted to be higher than that of the forward primer so that the target nucleic acid strand, to which the first probe and the second probe hybridize among the double-stranded target nucleic acid, could be amplified more efficiently.

The tubes containing the reaction mixture were placed in a real-time thermal cycler (CFX96 Real-time Cycler, Bio-Rad), and denatured at 92°C for 2 minutes, and then subjected to 50 cycles of 15 seconds at 92°C, 15 seconds at 57°C, 5 seconds at 68°C, 10 seconds at 72°C, and 5 seconds at 76°C. The detection of signals was performed at three temperatures in each cycle: 57°C, a temperature within the first signal-constant temperature range where both the first probe and the second probe are hybridized with the target nucleic acid; 68°C, a detection temperature within the signal-changing temperature range where the first probe is hybridized with the target nucleic acid while the second probe is not hybridized; and 76°C, a temperature within the second signal-constant temperature range where both the first probe and the second probe are not hybridized with the target nucleic acid.

As a result, as shown in FIG. 9, the amplification curves at 57°C, a temperature within the first signal-constant temperature range, and at 76°C, a temperature within the second signal-constant temperature range, exhibited constant signals without signal change despite the amplification of the target nucleic acid. In contrast, the amplification curve at the detection temperature of 68°C exhibited a signal change as the target nucleic acid was amplified. These results indicate that a composition for detecting a target nucleic acid comprising two probes having different Tm values according to the present disclosure enables real-time detection of a target nucleic acid. Furthermore, as described above, the composition for detecting a target nucleic acid comprising two probes having different Tm values according to the present disclosure was confirmed to be classified as an InterSC signal generation mechanism.

Meanwhile, in the case of the negative control reaction (Tube 2), it was confirmed that the signal is constant at 57°C, 68°C, and 76°C.

As described above, the method for detecting the target nucleic acid according to the present disclosure can determine the signal indicating the presence of the target nucleic acid (i.e., a change in signal) using the reference signal value obtained from the negative control reaction. The present inventors set a reference signal value of 300 based on the signal value of the negative control reaction (i.e., RFU: 0) and determined that the signal had changed if the signal value at 57°C, 68°C, and 76°C was equal to or greater than the reference signal value. As a result, as shown in Table 4, it was confirmed that Tube 1 exhibited a change in signal at the detection temperature of 68°C, with a Ct value of 24.43, but exhibited constant signals without any change at 57°C and 76°C.

**[Table 4]**

| Tube | Ct (Cycle threshold) | | |
|---|---|---|---|
| | 57°C | 68°C | 76°C |
| 1 | N/A | 24.43 | N/A |
| 2 | N/A | N/A | N/A |

| | | | |
|---|---|---|---|
| Tube 1: 500 pg of UP genome DNA; Tube 2: Negative control; N/A: Not Applicable | | | |

### Example 2: Detection of Multiple Target Nucleic Acids

According to the present disclosure, multiple target nucleic acids can be detected in a single reaction vessel using a single type of label by adjusting the signal-changing temperature ranges of compositions for detecting the multiple target nucleic acids,.

Accordingly, it was examined whether multiple target nucleic acids could be detected in a single reaction vessel using a single type of label by employing a composition for detecting a target nucleic acid comprising two probes according to the present disclosure.

### 2-1. Preparation of Target Nucleic Acids and Oligonucleotides

The genome DNA of Ureaplasma parvum (UP) (Accession No: ATCC 27815) and Chlamydia trachomatis (CT) (Accession No: ATCC VR-1500) were used as templates for the target nucleic acids. The detection temperature of the UP target nucleic acid (i.e., the first detection temperature) was set to 68°C, and the detection temperature of the CT target nucleic acid (i.e., the second detection temperature) was set to 76°C. The first region and the second region were selected on each target nucleic acid template in the 3' to 5' direction.

Then, as shown in Table 5, the sequences and lengths of the first probe and the second probe for detecting the first target nucleic acid, UP were designed such that their Tm values were 70°C and 64°C, respectively. Similarly, the sequences and lengths of the first probe and the second probe for detecting the second target nucleic acid, CT, were designed such that their Tm values were 76.5°C and 71.5°C, respectively. The first probe and the second probe for detecting the first target nucleic acid, UP, were the same as those used in Table 3 of Example 1-1.

Specifically, each first probe for the two target nucleic acids comprised a sequence complementary to a first region of the corresponding target nucleic acid and had a first quencher molecule (AZOQ2) at its 5'-end and a reporter molecule (Cal Fluor Red 610) at its 3'-end. Each second probe for the two target nucleic acids comprised a sequence complementary to a second region of the corresponding target nucleic acid and had a second quencher molecule (AZOQ2) at its 5'-end and Spacer C3 at its 3'-end to prevent extension by DNA polymerase.

A primer pair for amplifying the region comprising a first region and a second region of the first target nucleic acid, and a primer pair for amplifying the region comprising a first region and a second region of the second target nucleic acid, were designed as shown in Table 5.

**[Table 5]**

| Oligonucleotide Sequences | | | |
|---|---|---|---|
| Target nucleic acid | SEQ NO. | Oligo type | Sequence (5' -3') |
| UP | 1 | The first and second regions of the target nucleic acid | AGATTTATACGGTATTACAACTGGTGATAGCGTTAGATT AGGAGATACAAATCTTTGAGTTAAAGTTGAAAAAGACT TAACTACTTATGGTGAAGAATCTGTTTTTGGTGGTGG AAAAACCCTACGTGAAGGTATGGGAATGAATTCTACTA TGAAGTTAGATGATAAATTAGGTAATGCTGAAGT |
| | 2 | UP-Forward Primer | AGATTTATACGGTATTACAACTG |
| | 3 | UP-Reverse Primer | ACTTCAGCATTACCTAATTTATC |
| | 4 | UP-First Probe | [AZOQ2]GGTGAAGAATCTGTTTTGGTGGTGGAAA[C al Fluor Red 610] |
| | 5 | UP-Second Probe | [AZOQ2]CCTACGTGAAGGTATGGGAAT[Spacer C3] |
| CT | 6 | The first and second regions of the target nucleic acid | CTTTATTAGGAGGAACTGAAATAGGAAAATTCACAGTC ACACCCAAAAGCTCTGGGAGCATGTTCTTAGTCTCAGC AGATATTATTGCATCAAGAATGGAAGGCGGCGTTGTT CTAGCTTTGGTACGAGAAGGTGATTCTAAGCCCTGCGC GATTAGTTATGGATACTCATCAGGCGTTCCTAATTTATGT AGTCTAAGAACCAG |
| | 7 | CT-Forward Primer | CTTTATTAGGAGGAACTGAAATAGG |
| | 8 | CT-Reverse Primer | CTGGTTCTTAGACTACATAAATTAGG |
| | 9 | CT-First Probe | [AZOQ2]TGCATCAAGAATGGAAGGCGGCGTTGTTCT AGCT[Cal Fluor Red 610] |
| | 10 | CT-Second Probe | [AZOQ2]GGTACGAGAAGGTGATTCTAAGCCCTGCG[S pacer C3] |

| | | | |
|---|---|---|---|
| Bold: The first region of the target nucleic acid. Underlined: The second region of the target nucleic acid. | | | |

### 2-2. Multiplex Real-Time Polymerase Chain Reaction

A multiplex real-time polymerase chain reaction (PCR) was performed in a single reaction vessel using the above oligonucleotides.

The target nucleic acids (Tube 1: 500 pg of UP genomic DNA; Tube 2: 500 pg of CT genomic DNA; Tube 3: a mixture of 500 pg of UP genomic DNA and 500 pg of CT genomic DNA; and Tube 4: distilled water (negative control)) were mixed with 5 pmole of UP-forward primer (SEQ NO: 2), 20 pmole of UP-reverse primer (SEQ NO: 3), 5 pmole of UP-first probe (SEQ NO: 4), 5 pmole of UP-second probe (SEQ NO: 5), 5 pmole of CT-forward primer (SEQ NO: 7), 20 pmole of CT-reverse primer (SEQ NO: 8), 5 pmole of CT-first probe (SEQ NO: 9), and 5 pmole of CT-second probe (SEQ NO: 10). The mixture was then combined with DNA polymerase (2U SD Polymerase HotStart (BIORON, Germany)), 1 mM dNTP, and 100 mM MgSO₄ to prepare a reaction mixture in a final volume of 20 µL. The concentration of the reverse primers was adjusted to be higher than that of the forward primers to facilitate the amplification of the target nucleic acid strand to which the first probe and the second probe hybridize among the double-stranded target nucleic acid.

The tubes containing the reaction mixture were placed in a real-time thermal cycler (CFX96 Real-time Cycler, Bio-Rad) and denatured at 92°C for 2 minutes, and then subjected to 50 cycles of 15 seconds at 92°C, 15 seconds at 57°C, 5 seconds at 68°C, 10 seconds at 72°C, 5 seconds at 76°C, and 5 seconds at 85°C. Signal detection was performed at 57°C, 68°C (the first detection temperature), 76°C (the second detection temperature), and 85°C in each cycle.

57°C is a temperature within the first signal-constant temperature range of the composition for detecting the UP target nucleic acid and also within the first signal-constant temperature range of the composition for detecting the CT target nucleic acid. At 57°C, which is a temperature within the first signal-constant temperature range, the first probe and the second probe exist in the form shown in FIG. 1(i) in the absence of the target nucleic acid , whereby the reporter molecule is quenched by the first quencher molecule. In contrast, the first probe and the second probe exist in the form shown in FIG. 2(i) in the presence of the target nucleic acid, whereby the reporter molecule is quenched by the second quencher molecule. That is, at this temperature, both the composition for detecting the UP target nucleic acid and the composition for detecting the CT target nucleic acid provide a constant signal regardless of the presence or absence of the corresponding target nucleic acid.

85°C is a temperature within the second signal-constant temperature range of the composition for detecting the UP target nucleic acid and also within the second signal-constant temperature range of the composition for detecting the CT target nucleic acid. At 85°C, which is a temperature within the second signal-constant temperature range, the first probe and the second probe exist in the form shown in FIG. 1(iii) in the absence of the target nucleic acid, whereby the reporter molecule is quenched by the first quencher molecule. Similarly, the first probe and the second probe exist in the form shown in FIG. 2(iii) in the presence of the target nucleic acid, whereby the reporter molecule is also quenched by the first quencher molecule. That is, at this temperature, both the composition for detecting the UP target nucleic acid and the composition for detecting the CT target nucleic acid provide a constant signal regardless of the presence or absence of the corresponding target nucleic acid.

Meanwhile, the first detection temperature, 68°C, is a temperature within the signal-changing temperature range of the composition for detecting the UP target nucleic acid and also within the first signal-constant temperature range of the composition for detecting the CT target nucleic acid. As described above, at 68°C, which is a temperature within the first signal-constant temperature range of the composition for detecting the CT target nucleic acid, the composition provides a constant signal regardless of the presence or absence of the CT target nucleic acid. In contrast, for the UP target nucleic acid, the first probe and the second probe exist in the form shown in FIG. 1(ii) in the absence of the UP target nucleic acid, whereby the reporter molecule is quenched by the first quencher molecule. However, the first probe and the second probe exist in the form shown in FIG. 2(ii) in the presence of the UP target nucleic acid, whereby the reporter molecule is separated from both the first quencher molecule and the second quencher molecule and is unquenched. As a result, at this temperature, the composition for detecting the UP target nucleic acid provides a signal change dependent on the presence of the target nucleic acid.

The second detection temperature, 76°C, is a temperature within the second signal-constant temperature range of the composition for detecting the UP target nucleic acid and also within the signal-changing temperature range of the composition for detecting the CT target nucleic acid. As described above, at 76°C, which is a temperature within the second signal-constant temperature range of the composition for detecting the UP target nucleic acid, the composition for detecting the UP target nucleic acid provides a constant signal regardless of the presence or absence of the UP target nucleic acid. In contrast, for the CT target nucleic acid, the first probe and the second probe exist in the form shown in FIG. 1(ii) in the absence of the CT target nucleic acid, whereby the reporter molecule of the first probe is quenched by the first quencher molecule. However, the first probe and the second probe exist in the form shown in FIG. 2(ii) in the presence of the CT target nucleic acid, whereby the reporter molecule of the first probe is unquenched by both the first quencher molecule and the second quencher molecule. As a result, at this temperature, the composition for detecting the CT target nucleic acid provides a signal change dependent on the presence of the target nucleic acid.

FIG. 10 shows the amplification curves at each temperature as the result of multiplex PCR. As shown in FIG. 10, the amplification curves at 57°C and 85°C remained constant without any signal change in all tubes, Tube 1 to Tube 4, regardless of the presence or absence of the UP target nucleic acid and/or the CT target nucleic acid.

Meanwhile, at the first detection temperature of 68°C, the amplification curves in Tube 1 and Tube 3, which contain the UP target nucleic acid, showed a signal change as the UP target nucleic acid was amplified.

Additionally, at the second detection temperature of 76°C, the amplification curves in Tube 2 and Tube 3, which contain the CT target nucleic acid, showed a signal change as the CT target nucleic acid was amplified.

In the negative control reaction Tube 4, the amplification curves at all temperatures showed constant signals without any signal change.

These results indicate that multiple target nucleic acids can be detected in a single reaction vessel using a single type of label by adjusting the Tm values of the first probe and the second probe in the composition for detecting a target nucleic acid according to the present disclosure (i.e., by adjusting the signal-changing temperature range).

Subsequently, the signal indicating the presence of the target nucleic acid (i.e., a change in signal) was determined using the reference signal value obtained from the negative control reaction. The present inventors set a reference signal value of 300 based on the signal value of the negative control reaction (i.e., RFU: 0) and determined that the signal had changed if the signal value at 57°C, 68°C, 76°C, or 85°C was equal to or greater than the reference signal value. As a result, as shown in Table 6, it was confirmed that Tube 1 exhibited a signal change only at 68°C, the detection temperature of the UP target nucleic acid (i.e., the first detection temperature), with a Ct value of 24.24. Tube 2 exhibited a signal change only at 76°C, the detection temperature of the CT target nucleic acid (i.e., the second detection temperature), with a Ct value of 24.12. Tube 3 exhibited signal changes at both the first detection temperature of 68°C and the second detection temperature of 76°C, with Ct values of 24.69 and 23.69, respectively. In contrast, at 57°C and 85°C, the signal remained constant without any change.

These results indicate that, as described above, multiple distinct target nucleic acids can be independently detected at their respective detection temperatures.

**[Table 6]**

| Tube | Ct (Cycle threshold) | | | |
|---|---|---|---|---|
| | 57°C | 68°C | 76°C | 85°C |
| 1 | N/A | 24.24 | N/A | N/A |
| 2 | N/A | N/A | 24.12 | N/A |
| 3 | N/A | 24.69 | 23.69 | N/A |
| 4 | N/A | N/A | N/A | N/A |

| | | | | |
|---|---|---|---|---|
| Tube 1: 500 pg of UP genomic DNA; Tube 2: 500 pg of CT genomic DNA; Tube 3: 500 pg of UP genomic DNA and 500 pg of CT genomic DNA; Tube 4: Negative control; N/A: Not Applicable | | | | |

In summary, the composition for detecting a target nucleic acid according to the present disclosure can provide a signal dependent on the target nucleic acid only at a designated detection temperature by adjusting the Tm values of the first probe and the second probe relative to each other. This means that it can be controlled so that only a signal for the corresponding target nucleic acid can be provided at different detection temperatures by adjusting the Tm values of the first probe and the second probe for each of the multiple target nucleic acids. Example 2, which utilizes this principle, confirmed that multiple target nucleic acids could be detected in real time within a single reaction vessel using the same type of label and a single detector.

Having described a preferred embodiment of the present invention, it is to be understood that variants and modifications thereof falling within the spirit of the invention may become apparent to those skilled in this art, and the scope of this invention is to be determined by appended claims and their equivalents.

## Claims

1. A method for detecting *n* target nucleic acids in a sample, comprising:
(a) detecting signals at *n* detection temperatures, while incubating with *n* compositions for detecting the target nucleic acids, a sample suspected of containing at least one of the *n* target nucleic acids in a reaction vessel;
wherein *n is* an integer of 2 or more,
wherein the incubating comprises a plurality of cycles and the detection of signals is carried out at at least one of the plurality of cycles,
wherein each of the *n* compositions for detecting the target nucleic acids provides a signal change at a corresponding detection temperature among the *n* detection temperatures in the presence of a corresponding target nucleic acid, the signal change indicating the presence of a corresponding target nucleic acid,
wherein a composition for detecting an *i^{th}* target nucleic acid among the *n* compositions for detecting the target nucleic acids provides a signal change at an *i^{th}* detection temperature among the *n* detection temperatures and provides a constant signal at the other detection temperatures in the presence of the *i^{th}* target nucleic acid, the signal change indicating the presence of the *i*^{th} target nucleic acid,
wherein *i* represents an integer from 1 to *n*, and the *i*^{th} detection temperature is lower than the (*i+*1)*^{th}* detection temperature,
wherein in the temperature range covering all of the *n* detection temperatures, the composition for detecting the *i*^{th} target nucleic acid has a signal-changing temperature range in which the signal changes depending on the presence of the *i*^{th} target nucleic acid, and one or two signal-constant temperature ranges in which the signal is constant even in the presence of the *i*^{th} target nucleic acid,
wherein the composition for detecting the *i*^{th} target nucleic acid is any one of:
(i) an Under-Signal-Change (UnderSC) composition having a characteristic that the signal-changing temperature range is lower than the signal-constant temperature range,
(ii) an Inter-Signal-Change (InterSC) composition having a characteristic that the signal-changing temperature range is higher than one of two signal-constant temperature ranges, and lower than the other of the two signal-constant temperature ranges, and
(iii) an Over-Signal-Change (OverSC) composition having a characteristic that the signal-changing temperature range is higher than the signal-constant temperature range,
wherein at least one of the *n* compositions for detecting target nucleic acids comprises a first probe and a second probe having different Tm values,
wherein the first probe comprises a hybridizing nucleotide sequence to a first region of a corresponding target nucleic acid,
wherein the first probe has a reporter molecule and a first quencher molecule, wherein the reporter molecule and the first quencher molecule are located at positions such that (i) the reporter molecule and the first quencher molecule are in close proximity to each other, prior to hybridization of the first probe with the corresponding target nucleic acid, thereby causing the first quencher molecule to quench a signal from the reporter molecule, and (ii) the reporter molecule and the first quencher molecule are separated upon hybridization of the first probe with the corresponding target nucleic acid, thereby causing the first quencher molecule to unquench a signal from the reporter molecule,
wherein the second probe comprises a hybridizing nucleotide sequence to a second region of the corresponding target nucleic acid,
wherein the second probe has a second quencher molecule,
wherein the Tm value of the first probe is higher than the Tm value of the second probe, and
wherein the reporter molecule of the first probe is quenched by the second quencher molecule of the second probe when the first probe and the second probe are hybridized with the corresponding target nucleic acid; and
(b) determining the presence of the *n* target nucleic acids from the signals detected in step (a), wherein the presence of the *i^{th}* target nucleic acid is determined by the signal change detected at the *i*^{th} detection temperature.

2. The method of claim 1, wherein the first quencher molecule is located at 12 to 60 nucleotides apart from the reporter molecule.

3. The method of claim 1, wherein the reporter molecule is linked to the 3'-end portion or the 5'-end portion of the first probe.

4. The method of claim 1, wherein the reporter molecule and the second quencher molecule are located at positions such that the second quencher molecule of the second probe is in close proximity to the reporter molecule of the first probe upon hybridization of the first probe and the second probe with the corresponding target nucleic acid, thereby causing the second quencher molecule to quench a signal from the reporter molecule.

5. The method of claim 4, wherein when the first probe and the second probe are hybridized with the corresponding target nucleic acid, the second quencher molecule is located immediately adjacent to or 1 to 4 nucleotides apart from the reporter molecule.

6. The method of claim 1, wherein the second quencher molecule is linked to the 3'-end portion or the 5'-end portion of the second probe.

7. The method of claim 1, wherein when the second probe is hybridized with the corresponding target nucleic acid in the 3' direction downstream of the first probe, the reporter molecule is located at the 3'-end portion of the first probe, and the second quencher molecule is located at the 5'-end portion of the second probe.

8. The method of claim 1, wherein when the second probe is hybridized with the corresponding target nucleic acid in the 5' direction upstream of the first probe, the reporter molecule is located at the 5'-end portion of the first probe, and the second quencher molecule is located at the 3'-end portion of the second probe.

9. The method of claim 1, wherein the first quencher molecule of the first probe and the second quencher molecule of the second probe are the same type of quencher molecule.

10. The method of claim 1, wherein the first probe and/or the second probe are blocked at its 3'-end to prevent its extension.

11. The method of claim 1, wherein the composition comprising the first probe and the second probe having different Tm values is an InterSC composition having a characteristic that the signal-changing temperature range is higher than a first signal-constant temperature of two signal-constant temperature ranges, and lower than a second signal-constant of the two signal-constant temperature ranges.

12. The method of claim 11, wherein the first probe and the second probe are hybridized with the corresponding target nucleic acid at temperatures within the first signal-constant temperature range in the presence of the corresponding target nucleic acid.

13. The method of claim 11, wherein the first probe and the second probe are not hybridized with the corresponding target nucleic acid at temperatures within the second signal-constant temperature range in the presence of the corresponding target nucleic acid.

14. The method of claim 11, wherein the first probe is hybridized with the corresponding target nucleic acid but the second probe is not hybridized with the corresponding target nucleic acid at temperatures within the signal-changing temperature range in the presence of the corresponding target nucleic acid.

15. The method of claim 11, wherein the signal-changing temperature range depends on the Tm values of the first probe and the second probe.

16. The method of claim 1, wherein the *i^{th}* detection temperature is selected within the signal-changing temperature range of the composition for detecting the *i*^{th} target nucleic acid, wherein the *i*^{th} detection temperature is not comprised in the signal-changing temperature ranges of the compositions for detecting the other target nucleic acids.

17. The method of claim 1, wherein the signal-changing temperature range of the composition for detecting the *i*^{th} target nucleic acid overlaps partially with the signal-changing temperature range of a composition for detecting a target nucleic acid having an adjacent detection temperature, and does not overlap with the signal-changing temperature range of a composition for detecting a target nucleic acid having a detection temperature that is not adjacent thereto.

18. The method of claim 1, wherein, when *n is* 2, the composition for detecting the first target nucleic acid is an UnderSC or InterSC composition, and the composition for detecting the second target nucleic acid is an InterSC or OverSC composition.

19. The method of claim 1, wherein, when *n* is 3 or more, the composition for detecting the first target nucleic acid is an UnderSC or InterSC composition, the composition for detecting the *n*^{th} target nucleic acid is an InterSC or OverSC composition, and each of compositions for detecting target nucleic acids other than the first target nucleic acid and the *n*^{th} target nucleic acid is an InterSC composition.

20. The method of claim 1, wherein the composition for detecting the *i*^{th} target nucleic acid comprises a label that provides a signal dependent on the presence of the *i*^{th} target nucleic acid.

21. The method of claim 20, wherein the label is linked to an oligonucleotide or is incorporated into an oligonucleotide during the incubating.

22. The method of claim 1, wherein the composition for detecting the *i*^{th} target nucleic acid provides a duplex providing a signal change.

23. The method of claim 22, wherein the duplex providing the signal change has initially been included in the composition for detecting the *i*^{th} target nucleic acid.

24. The method of claim 23, wherein the duplex providing the signal change is generated by hybridization between a label-linked oligonucleotide and an oligonucleotide hybridizable with the label-linked oligonucleotide.

25. The method of claim 22, wherein the duplex providing the signal change is generated in the incubating.

26. The method of claim 25, wherein the duplex providing the signal change is generated by hybridization between a label-linked oligonucleotide and the corresponding target nucleic acid.

27. The method of claim 25, wherein the duplex providing the signal change is generated by a cleavage reaction dependent on the presence of the corresponding target nucleic acid.

28. The method of claim 22, wherein the duplex providing the signal change comprises a label.

29. The method of claim 1, wherein the composition for detecting the *i*^{th} target nucleic acid provides a duplex providing a signal change, and the signal-changing temperature range of the composition for detecting the *i*^{th} target nucleic acid varies depending on the length and/or sequence of the duplex.

30. The method of claim 1, wherein the detection of signals is carried out at at least two of the plurality of cycles.

31. The method of claim 30, wherein the signal change is measured using the signals detected at the at least two of the plurality of cycles.

32. The method of claim 1, wherein the signal change at the *i*^{th} detection temperature is measured using a signal detected at the at least one of the plurality of cycles and a reference signal value.

33. The method of claim 32, wherein the reference signal value is obtained from a reaction in the absence of the *i*^{th} target nucleic acid.

34. The method of claim 1, wherein the detection of a signal at each of the *n* detection temperatures is carried out using a single type of detector.

35. The method of claim 34, wherein the signals detected at the n detection temperatures are not differentiated from each other by the single type of detector.

36. The method of claim 1, wherein the incubating comprises a nucleic acid amplification reaction.

37. Method for detecting a target nucleic acid in a sample, comprising:
(a) incubating the sample with a composition for detecting the target nucleic acid comprising a first probe and a second probe having different Tm values;
wherein the first probe comprises a hybridizing nucleotide sequence to a first region of the target nucleic acid,
wherein the first probe has a reporter molecule and a first quencher molecule,
wherein the reporter molecule and the first quencher molecule are located at positions such that (i) the reporter molecule and the first quencher molecule are in close proximity to each other, prior to hybridization of the first probe with the target nucleic acid, thereby causing the first quencher molecule to quench a signal from the reporter molecule, and (ii) the reporter molecule and the first quencher molecule are separated upon hybridization of the first probe with the target nucleic acid, thereby causing the first quencher molecule to unquench a signal from the reporter molecule,
wherein the second probe comprising a hybridizing nucleotide sequence to a second region of the target nucleic acid,
wherein the second probe has a second quencher molecule,
wherein the Tm value of the first probe is higher than the Tm value of the second probe, and
wherein the reporter molecule of the first probe is quenched by the second quencher molecule of the second probe when the first probe and the second probe are hybridized with the target nucleic acid; and
(b) detecting a signal; and
(c) determining the presence of the target nucleic acid from the signal detected in the step (b).

38. The method of claim 37, wherein the reporter molecule and the second quencher molecule are located at positions such that the second quencher molecule of the second probe is in close proximity to the reporter molecule of the first probe upon hybridization of the first probe and the second probe with the target nucleic acid, thereby causing the second quencher molecule to quench a signal from the reporter molecule.

39. The method of claim 37, wherein the second quencher molecule is linked to the 3'-end portion or the 5'-end portion of the second probe.

40. The method of claim 37, wherein when the second probe is hybridized with the corresponding target nucleic acid in the 3' direction downstream of the first probe, the reporter molecule is located at the 3'-end portion of the first probe, and the second quencher molecule is located at the 5'-end portion of the second probe.

41. The method of claim 37, wherein when the second probe is hybridized with the corresponding target nucleic acid in the 5' direction upstream of the first probe, the reporter molecule is located at the 5'-end portion of the first probe, and the second quencher molecule is located at the 3'-end portion of the second probe.

42. The method of claim 37, wherein the first quencher molecule of the first probe and the second quencher molecule of the second probe are the same type of quencher molecule.

43. The method of claim 37, wherein the composition comprising the first probe and the second probe having different Tm values has a signal-changing temperature range (SChTR) in which the signal changes depending on the presence of the target nucleic acid, and two signal-constant temperature ranges (SCoTRs) in which the signal is constant even in the presence of the target nucleic acid,

44. The method of claim 43, wherein the signal-changing temperature range is higher than a first signal-constant temperature range of the two signal-constant temperature ranges, and lower than a second signal-constant temperature range of the two signal-constant temperature ranges.

45. The method of claim 44, wherein the first probe and the second probe are hybridized with the target nucleic acid at temperatures within the first signal-constant temperature range in the presence of the target nucleic acid.

46. The method of claim 44, wherein the first probe and the second probe are not hybridized with the target nucleic acid at temperatures within the second signal-constant temperature range in the presence of the target nucleic acid.

47. The method of claim 44, wherein the first probe is hybridized with the target nucleic acid but the second probe is not hybridized with the target nucleic acid at temperatures within the signal-changing temperature range in the presence of the target nucleic acid.

48. The method of claim 43, wherein the signal-changing temperature range depends on the Tm values of the first probe and the second probe.

49. The method of claim 37, wherein the signal detected in the step (b) is a signal dependent on the presence of the target nucleic acid.

50. The method of claim 43, wherein the step (b) is carried out at a detection temperature selected within the signal-changing temperature range.

51. The method of claim 37, wherein the incubating comprises a nucleic acid amplification reaction.

52. The method of claim 37, wherein the incubating in the step (a) comprises a plurality of cycles, and the detection of the signal in the step (b) is carried out at at least one of the plurality of cycles.

53. The method of claim 52, wherein the presence of the target nucleic acid in the step (c) is determined by a signal change, wherein the signal change is measured using the signal detected at least one of the plurality of cycles and a reference signal value.

54. The method of claim 53, wherein the reference signal value is obtained from a reaction in the absence of the target nucleic acid.

55. The method of claim 37, wherein the incubating in the step (a) comprises a plurality of cycles, and the detection of the signal in the step (b) is carried out at at least two of the plurality of cycles.

56. The method of claim 56, wherein the presence of the target nucleic acid in the step (c) is determined by a signal change, wherein the signal change is measured using signals detected at the at least two of the plurality of cycles.

57. A composition for detecting a target nucleic acid in a sample, comprising:
(a) a first probe comprising a hybridizing nucleotide sequence to a first region of the target nucleic acid;
wherein the first probe has a reporter molecule and a first quencher molecule,
wherein the reporter molecule and the first quencher molecule are located at positions such that (i) the reporter molecule and the first quencher molecule are in close proximity to each other, prior to hybridization of the first probe with the target nucleic acid, thereby causing the first quencher molecule to quench a signal from the reporter molecule, and (ii) the reporter molecule and the first quencher molecule are separated upon hybridization of the first probe with the target nucleic acid, thereby causing the first quencher molecule to unquench a signal from the reporter molecule; and
(b) a second probe comprising a hybridizing nucleotide sequence to a second region of the target nucleic acid;
wherein the second probe has a second quencher molecule,
wherein the Tm value of the first probe is higher than the Tm value of the second probe, and
wherein the reporter molecule of the first probe is quenched by the second quencher molecule of the second probe when the first probe and the second probe are hybridized with the target nucleic acid.

58. The composition of claim 57, wherein the reporter molecule and the second quencher molecule are located at positions such that the second quencher molecule of the second probe is in close proximity to the reporter molecule of the first probe upon hybridization of the first probe and the second probe with the target nucleic acid, thereby causing the second quencher molecule to quench a signal from the reporter molecule.

59. The composition of claim 57, wherein the second quencher molecule is linked to the 3'-end portion or the 5'-end portion of the second probe.

60. The composition of claim 57, wherein when the second probe is hybridized with the corresponding target nucleic acid in the 3' direction downstream of the first probe, the reporter molecule is located at the 3'-end portion of the first probe, and the second quencher molecule is located at the 5'-end portion of the second probe.

61. The composition of claim 57, wherein when the second probe is hybridized with the corresponding target nucleic acid in the 5' direction upstream of the first probe, the reporter molecule is located at the 5'-end portion of the first probe, and the second quencher molecule is located at the 3'-end portion of the second probe.

62. The composition of claim 57, wherein the first quencher molecule of the first probe and the second quencher molecule of the second probe are the same type of quencher molecule.

63. The composition of claim 57, which has a signal-changing temperature range (SChTR) in which the signal changes depending on the presence of the target nucleic acid, and two signal-constant temperature ranges (SCoTRs) in which the signal is constant even in the presence of the target nucleic acid,

64. The composition of claim 63, wherein the signal-changing temperature range is higher than a first signal-constant temperature range of the two signal-constant temperature ranges, and lower than a second signal-constant temperature range of the two signal-constant temperature ranges.

65. The composition of claim 64, wherein the first probe and the second probe are hybridized with the target nucleic acid at temperatures within the first signal-constant temperature range in the presence of the target nucleic acid.

66. The composition of claim 64, wherein the first probe and the second probe are not hybridized with the target nucleic acid at temperatures within the second signal-constant temperature range in the presence of the target nucleic acid.

67. The composition of claim 64, wherein the first probe is hybridized with the target nucleic acid but the second probe is not hybridized with the target nucleic acid at temperatures within the signal-changing temperature range in the presence of the target nucleic acid.

68. The composition of claim 63, wherein the signal-changing temperature range depends on the Tm values of the first probe and the second probe.
